# FASCICULE DE BREVET EUROPEEN

(11) **EP 1 204 675 B1**
(45) Date de publication et mention de la délivrance du brevet: **21.05.2003**
(21) Numéro de dépôt: 00958620.7
(22) Date de dépôt: 26.07.2000
(51) Int. Cl.: C07K 7/06, A61K 38/05, A61K 38/08, A61P 31/04

(54) **DERIVES DE STREPTOGRAMINES, LEUR PREPARATION ET LES COMPOSITIONS QUI LES CONTIENNENT**
STREPTOGRAMINDERIVATE, IHRE HERSTELLUNG UND SIE ENTHALTENDE ZUBEREITUNGEN
STREPTOGRAMIN DERIVATIVES, PRODUCTION THEREOF AND COMPOSITIONS CONTAINING THE SAME

(30) Priorité: 27.07.1999 FR 9909708
(43) Date de publication de la demande: 15.05.2002
(73) Titulaire: Aventis Pharma S.A., 92160 Antony (FR)
(72) Inventeur: BACQUE, Eric, F-91390 Morsang sur Orge (FR); BARRIERE, Jean-Claude, F-91400 Bures sur Yvette (FR); DOERFLINGER, Gilles, F-91940 Les Ulis (FR); DUTRUC-ROSSET, Gilles, F-75012 Paris (FR); PANTEL, Guy, F-94510 La Queue En Brie (FR)
(86) Numéro de dépôt international: FR0002146
(87) Numéro de publication internationale: WO01010895

(56) Documents cités:
- WO-A-96/33213
- FR-A- 2 722 210
- FR-A- 2 723 372
- FR-A- 2 775 288

## Description

La présente invention concerne des dérivés du groupe B des streptogramines de formule générale : 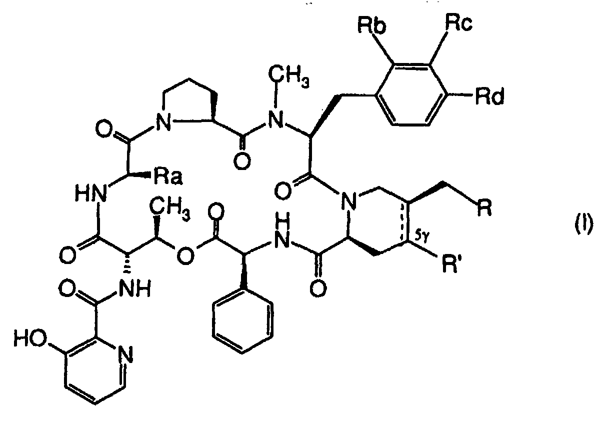 ainsi que leurs sels lorsqu'ils existent, qui présentent une activité antibactérienne particulièrement intéressante, seuls ou associés à un dérivé du groupe A des streptogramines.

Parmi les streptogramines connues, la pristinamycine (RP 7293), antibactérien d'origine naturelle produit par *Streptomyces pristinaespiralis* a été isolée pour la première fois en 1955. La pristinamycine commercialisée sous le nom de Pyostacine® est constituée principalement de pristinamycine IA associée à la pristinamycine IIA.

Un autre antibactérien de la classe des streptogramines : la virginiamycine, a été isolé à partir de *Streptomyces virginiae*, ATCC 13161 [Antibiotics and Chemotherapy, 5, 632 (1955)]. La virginiamycine (Staphylomycine®) est constituée principalement de facteur S associé au facteur M₁.

Des dérivés hémisynthétiques de streptogramines représentés par la structure : 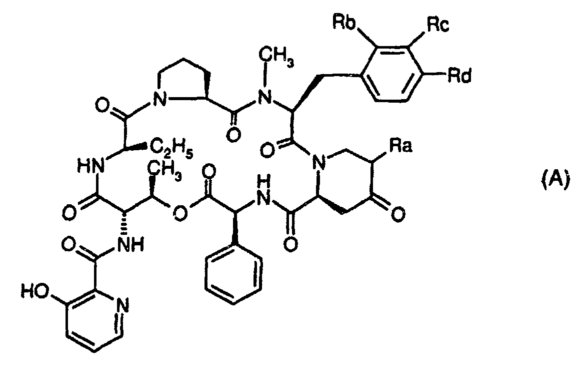 dans laquelle,
Ra est un radical de structure -CH₂R'a pour lequel R'a est un radical du type héterocyclylthio pouvant être substitué ou bien représente un radical de structure =CHR'a pour lequel R'a est un radical alcoylamino, alcoyloxy ou alcoylthio substitués, ou un radical du type héterocyclylamino, héterocyclyloxy, héterocyclylthio pouvant être substitués, Rb et Rc sont des atomes d'hydrogène et Rd est un atome d'hydrogène ou un radical diméthylamino, ou bien Ra est un atome d'hydrogène et Rb est hydrogène ou méthyle, Rc et Rd sont hydrogène ou divers substituants, ont été décrits dans les brevets ou demandes de brevet EP 133097, EP 248703, EP 770132 et EP 772630.
Associés à une composante hémisynthétique du groupe A des streptogramines, ils manifestent une synergie d'action et sont utilisables comme agents antibactériens soit par voie injectable seulement, soit uniquement par voie orale.

Dans le brevet européen EP 133098 ont été également décrits des dérivés de synergistine du groupe B portant des chaînes aminométhylènyle en position 5δ, qui sont des intermédiaires de synthèse.

Il a maintenant été trouvé, et c'est ce qui fait l'objet de la présente invention, que les produits de formule générale (I) dans laquelle :
R représente un radical -NR₁R₂ ou -SR₃ pour lequel :
   R₁ et R₂ identiques ou différents représentent un atome d'hydrogène, un radical alcoyle (1 à 8 carbones) éventuellement substitué par hydroxy, alcényle (3 à 8 carbones), cycloalcoyle (3 à 8 carbones), alcoyloxy (1 à 8 carbones), dialcoylamino, phénylalcoyle éventuellement substitué [par un ou plusieurs atomes d'halogène ou radicaux alcoyle, hydroxyalcoyle, alcoyloxy ou dialcoylamino], hétérocyclylalcoyle saturé ou insaturé (3 à 8 chaînons) contenant 1 ou plusieurs hétéroatomes choisis parmi l'azote, le soufre ou l'oxygène, ou dialcoylaminoalcoyle, ou bien
   R₁ et R₂ forment ensemble avec l'atome d'azote auquel ils sont attachés, un hétérocycle mono ou polycyclique, saturé, partiellement saturé ou insaturé, de 3 à 12 chaînons, contenant éventuellement un autre hétéroatome choisi parmi l'oxygène le soufre ou l'azote et éventuellement substitué [par un ou plusieurs radicaux hydroxy, alcoyle, phényle éventuellement substitué par un atome d'halogène, phénylalcoyle, phénylalcényle (alcényle contenant 2 à 4 carbones), hydroxyalcoyle, acyle, alcoyloxycarbonyle, ou hétérocyclyle ou hétérocyclylcarbonyle dont la partie hétérocyclyle est saturée ou insaturée (4 à 6 chaînons) et contient 1 ou plusieurs hétéroatomes choisis parmi l'oxygène le soufre ou l'azote],
R₃ est un radical alcoyle (contenant 1 à 8 atomes de carbone) ou cycloalcoyle (contenant 3 à 8 atomes de carbone) substitués par un radical -NR₁R₂ pour lequel R₁ et R₂ identiques ou différents représentent un atome d'hydrogène ou un radical alcoyle ou forment ensemble avec l'atome d'azote auquel ils sont attachés, un hétérocycle tel que défini ci-dessus, ou bien R₃ représente un radical hétérocyclyle mono ou polycyclique ou hétérocyclylméthyle saturé ou insaturé contenant 3 à 7 chaînons et éventuellement un autre hétéroatome choisi parmi l'oxygène le soufre ou l'azote et éventuellement substitué par un radical alcoyle. 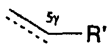
représente le reste d'un cycle insaturé non substitué en 5γ : 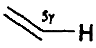 ou le reste d'un cycle saturé substitué en 5γ par un radical fluoro : 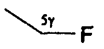 Ra est un radical méthyle ou éthyle, et
Rb, Rc et Rd ont les définitions ci-après :
1) Rb et Rc sont des atomes d'hydrogène et Rd est un atome d'hydrogène ou un radical méthylamirio ou diméthylamino,
2) Rb est un atome d'hydrogène, Rc est un atome d'hydrogène, de chlore ou de brome, ou représente un radical alcényle (3 à 5C), et Rd est un radical -NMe-R''' pour lequel R''' représente un radical alcoyle, hydroxyalcoyle (2 à 4C), ou alcényle (2 à 8C) éventuellement substitué par phényle, cycloalcoyl. (3 à 6C) méthyle, benzyle, benzyle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R''' représente un radical cyanométhyle ou carboxyméthyle, ou représente -CORe ou -CH₂CORe pour lesquels soit Re est -OR'e, R'e étant alcoyle (1 à 6 carbones), alcényle (2 à 6 carbones), benzyle, phényle, tolyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle
7) Rc est un atome d'hydrogène et Rb et Rd représentent un radical méthyle,
manifestent des activités particulièrement intéressantes, à la fois par voie orale et parentérale.

Les dérivés de streptogramine de formule générale (I) sont en effet particulièrement intéressants du fait de leur activité puissante à la fois par voie orale et parentérale ce qui leur apporte un avantage indéniable dans le cas notamment de traitements d'infections graves, en milieu hospitalier par voie injectable, suivis d'un traitement ambulatoire par voie orale plus facile à administrer aux patients. Ainsi le praticien ne se trouve plus dans l'obligation de changer le patient de classe de médicament entre la fin du traitement hospitalier et la fin globale du traitement.

Dans la formule générale (I) ci-dessus, les atomes d'halogène peuvent être choisis parmi le fluor, le chlore, le brome ou l'iode ; les radicaux alcoyle ou acyle sont droits ou ramifiés et, sauf mention spéciale, contiennent 1 à 4 atomes de carbone. Les radicaux alcényle peuvent être également en chaîne droite ou ramifiée et contiennent 2 à 4 atomes de carbone.

Il est également entendu que, dans la définition ci-dessus, lorsque R₁ et R₂ représentent hétérocyclylalcoyle,comprennent un radical hétérocyclyle ou forment ensemble avec l'atome d'azote un hétérocycle, ou lorsque R₃ représente un radical hétérocyclyle ou hétérocyclylméthyle, le radical hétérocyclyle peut être saturé ou insaturé et éventuellement polycyclique (bi ou tricyclique notamment).

A titre non limitatif, parmi les radicaux hétérocyclyle mentionnés ci-avant, peuvent être cités notamment pyrrolyle, pyrrolidinyle, pipéridinyle, pyrazinyle, pyrimidinyle, pipérazinyle, pyridyle, tétrahydropyridyle, tétrahydroquinolyle, tétrahydroisoquinolyle, morpholinyle, thiomorpholinyle, thiazolyle, oxazolyle, imidazolidinyle, imidazolyle, benzimidazolyle, furyle, thiényle, dioxolanyle.

Selon l'invention les produits de formule générale (I) peuvent être préparés par action d'un agent de fluoration sur le dérivé de synergistine du groupe B de formule générale : 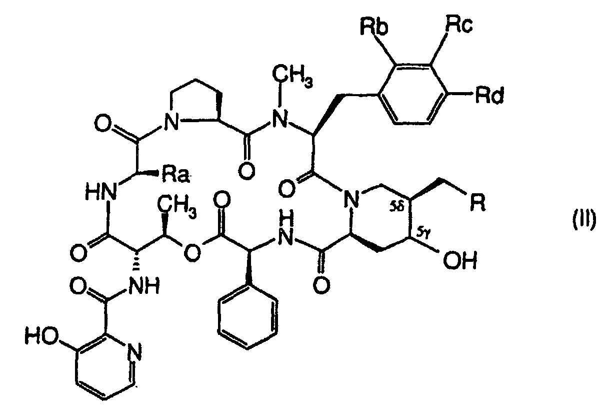 dans laquelle R, Ra, Rb, Rc et Rd sont définis comme ci-dessus, suivie de la séparation du dérivé fluoré ou du dérivé insaturé en position 5γ-5δ.

La réaction s'effectue généralement par action d'un agent de fluoration comme un fluorure de soufre [par exemple trifluorure d'aminosoufre : trifluorure de morpholino soufre, trifluorure de diéthylamino soufre (Tetrahedron, 44, 2875 (1988)), trifluorure de bis(2-méthoxyéthyl)amino soufre (Deoxofluor®), ou par exemple tétrafluorure de soufre (J. Org. Chem., 40, 3808 (1975)] ou un agent comme l'hexafluoropropyl diéthylamine (JP 2 039 546) ou la N-(chloro-2 trifluoro-1,1,2 éthyl) diéthylamine. La réaction est mise en oeuvre dans un solvant organique comme par exemple un solvant chloré (dichlorométhane, dichloréthane, chloroforme notamment), à une température comprise entre -70 et 50°C et de préférence en milieu inerte (sous argon ou sous azote par exemple).

La séparation du dérivé fluoré et du dérivé insaturé pour lequel 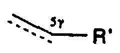 représente : 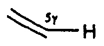 s'effectue selon les méthodes habituelles qui n'altèrent pas le reste de la molécule. Par exemple en opérant par chromatographie ou par cristallisation.

Selon une autre alternative de l'invention, les dérivés de synergistine de formule générale (I) pour lesquels 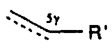 représente : 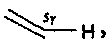 peuvent être préparés par action d'un halogénure de thionyle, en présence d'une base azotée, sur le dérivé de synergistine du groupe B de formule générale (II).

La réaction est mise en oeuvre par traitement par le chlorure ou le bromure de thionyle en présence d' une base azotée (comme par exemple la triéthylamine ou la pyridine) à une température comprise entre -50 et +80°C, dans un solvant chloré (dichlorométhane, 1,2-dichloréthane, chloroforme notamment) ou un éther (comme par exemple le tétrahydrofurane [THF]).

Le dérivé de synergistine du groupe B de formule générale (II) peut être préparé par réduction de la fonction cétone en 5γ d'un dérivé de streptogramine de formule générale : 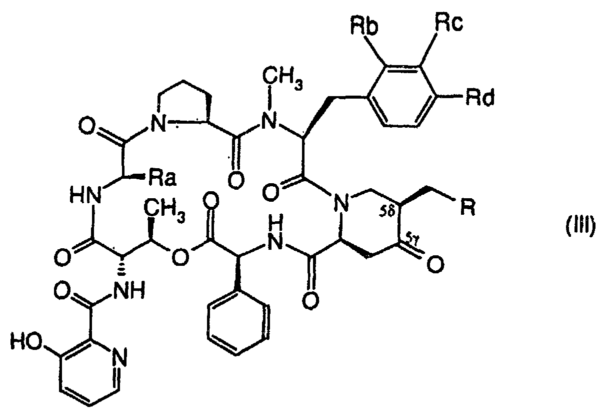 dans laquelle R, Ra, Rb, Rc et Rd sont définis comme ci dessus, selon les méthodes habituelles qui n'altèrent pas le reste de la molécule.

On opère par traitement par un agent réducteur comme un hydrure, par exemple un borohydrure alcalin (borohydrure de sodium, borohydrure de potassium, triacétoxy-borohydrure de sodium, cyanoborohydrure de sodium notamment), à une température comprise entre -70 et 60°C, dans un solvant organique comme un éther (par exemple THF,) ou un alcool (par exemple méthanol, éthanol) ou un solvant chloré (par exemple dichorométhane).

Le dérivé de streptogramine de formule générale (III) peut être préparé selon ou par analogie avec les méthodes décrites dans les brevets européens EP 133097, EP 133098, EP 248703, EP 432029, EP 770132 ou EP 772630.

Selon l'invention les dérivés de streptogramine de formule générale (I) pour lesquels le symbole 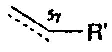 représente 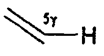 peuvent également être préparés par action d'une amine HNR₁R₂ ou d'un thiol HS-R₃ sur un dérivé halogéné de streptogramine de formule générale : 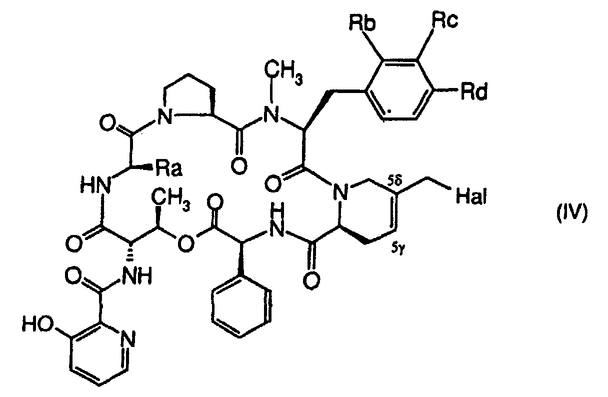 dans laquelle Ra, Rb, Rc et Rd sont définis comme ci dessus, et Hal représente un atome d'halogène.

De préférence le symbole Hal représente un atome de chlore ou de brome.

La réaction des amines R₁R₂NH s'effectue dans un solvant organique comme un amide (diméthylformamide par exemple), ou un nitrile (acétonitrile par exemple), ou un solvant chloré (chloroforme par exemple) à une température comprise entre 0 et 80°C. Eventuellement, on opère en présence de triéthylamine. Lorsque l'on fait réagir un thiol HS-R₃, on opère en milieu basique, par exemple en présence d'un hydrure alcalin (hydrure de sodium par exemple), dans un solvant organique comme un amide (diméthylformamide par exemple), ou un nitrile (acétonitrile par exemple), éventuellement en présence de triéthylamine, à une température comprise entre 0 et 80°C.

Le dérivé de streptogramine de formule générale (IV) peut être préparé par traitement par un agent d'halogénation, d'une 5δ-méthylène streptogramine de formule générale : 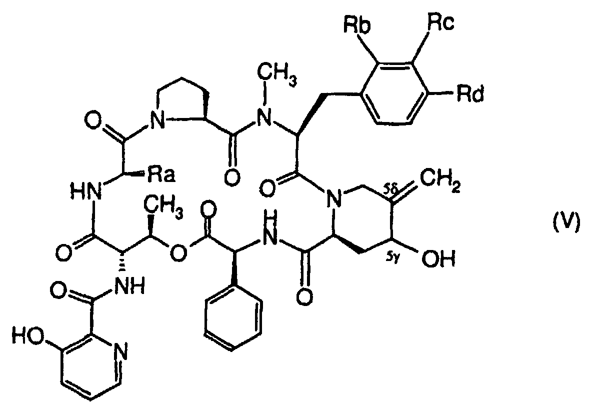 dans laquelle Ra, Rb, Rc et Rd sont définis comme ci dessus.

On opère avantageusement au moyen des agents d'halogénation habituels qui n'altèrent pas le reste de la molécule. Notamment on fait agir le chlorure ou le bromure de thionyle dans un solvant organique comme un solvant chloré (dichlorométhane, dichloroéthane, chloroforme par exemple), ou comme un éther (tétrahydrofurane par exemple) ou l'on opère dans un mélange de ces solvants, à une température comprise entre -60 et 80°C.

Le dérivé de streptogramine de formule générale (V) peut être préparé par réduction de la fonction cétone en 5γ de la synergistine de formule générale : 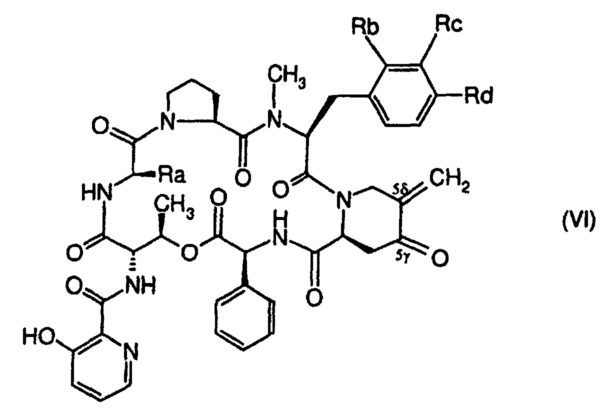 dans laquelle Ra, Rb, Rc et Rd sont définis comme ci dessus.

La réaction s'effectue dans des conditions analogues aux conditions décrites pour obtenir un dérivé de streptogramine de formule générale (II) à partir d'un dérivé de formule générale (III). On opère avantageusement dans un solvant organique comme un alcool (méthanol par exemple) ou un solvant chloré (dichlorométhane, dichloréthane, chloroforme par exemple), ou dans mélange de solvants alcool/solvant chloré (par exemple comme le méthanol/dichlorométhane), en présence de chlorure de cérium anhydre, à une température comprise entre -60 et 60°C.

Le dérivé de streptogramine de formule générale (VI) peut être préparé selon les méthodes décrites dans les brevets européens EP 133098 et EP 432029, ou par analogie avec ces méthodes ou les méthodes décrites dans EP 248703, EP 770132, EP 772630, EP 821697 ou WO 99/43699 ou décrites ci-après dans les exemples.

Les dérivés de streptogramine de formule générale (I) ou (IV) peuvent être purifiés le cas échéant par des méthodes physiques telles que la cristallisation ou la chromatographie.

Les dérivés de streptogramine de formule générale (II) pour lesquels R, Ra, Rb, Rc et Rd sont définis comme précédemment sont des produits nouveaux. Il est entendu que ces produits entrent aussi dans le cadre de la présente invention.

Certains des dérivés de streptogramine de formule générale (I) peuvent être transformés à l'état de sels d'addition avec les acides, par les méthodes connues. Il est entendu que ces sels lorsqu'ils existent entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Le cas échéant, les dérivés portant un substituant carboxy peuvent être transformés en sels métalliques ou en sels d'addition avec les bases azotées selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les dérivés de streptogramine selon la présente invention présentent des propriétés antibactériennes et des propriétés synergisantes de l'activité antibactérienne des dérivés de streptogramine du groupe A. Ils sont particulièrement intéressants du fait de leur activité seuls ou associés à des composantes du groupe A des streptogramines et surtout du fait de leur activité à la fois par voie orale et parentérale ce qui ouvre la voie à un traitement relai ambulatoire sans modifier la nature du médicament.

Lorsqu'ils sont associés avec une composante ou un dérivé du groupe A des streptogramines, ces derniers peuvent être notamment choisis selon que l'on désire obtenir une forme administrable par voie orale ou parentérale, parmi les composantes naturelles : pristinamycine IIA, pristinamycine IIB, pristinamycine IIC, pristinamycine IID, pristinamycine IIE, pristinamycine IIF, pristinamycine IIG ou parmi des dérivés d'hémisynthèse tels que décrits dans les brevets ou demandes de brevet US 4 590 004 et EP 191662 ou encore parmi les dérivés d'hémisynthèse de formule générale : 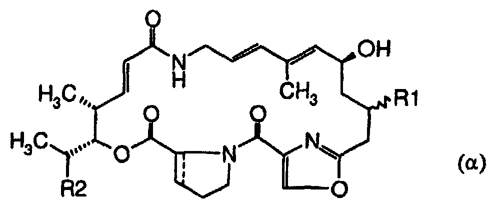 décrits dans la demande internationale WO 99/051265, dans laquelle R₁ est un radical -NR'R" pour lequel R' est un atome d'hydrogène ou un radical méthyle, et R" est un atome d'hydrogène, un radical alcoyle, cycloalcoyle, allyle, propargyle, benzyle, ou -OR''', R''' étant un atome d'hydrogène, un radical alcoyle, cycloalcoyle, allyle, propargyle ou benzyle, ou -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, R₂ est un atome d'hydrogène ou un radical méthyle ou éthyle, et la liaison --- représente une liaison simple ou une liaison double, ainsi que leurs sels. Les dérivés du groupe A pouvant leur être associés peuvent être également choisis parmi des dérivés d'hémisynthèse de formule générale : 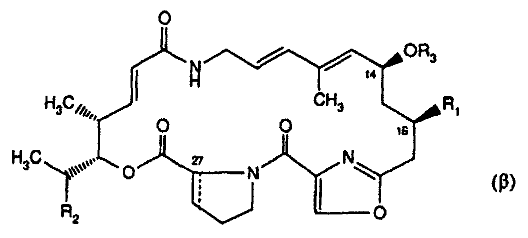 dans laquelle R₁ représente un atome d'halogène ou un radical azido ou thiocyanato, R₂ représente un atome d'hydrogène ou un radical méthyle ou éthyle, R₃ représente un atome d'hydrogène, ou le reste d'un ester aliphatique, cycloaliphatique, aromatique, araliphatique, hétérocyclique ou hétérocyclylaliphatique pouvant être substitué, et la liaison --- représente une liaison simple (stéréochimie 27R) ou une liaison double, et leurs sels lorsqu'ils existent. Et notamment les produits de formule générale (β) pour lesquels le reste d'ester R₃ peut être choisi parmi : des radicaux R'₃-CO- pour lesquels R'₃ est phényle ou phénylalcoyle non substitués ou substitués sur le radical phényle [par un ou plusieurs radicaux choisis parmi alcoyle, portant éventuellement un radical NR"R''' dont les radicaux R" et R''' identiques ou différents peuvent être des atomes d'hydrogène ou des radicaux alcoyle pouvant former ensemble avec l'atome d'azote auquel ils sont attachés un radical hétérocyclyle saturé ou insaturé de 3 à 8 chaînons, comprenant éventuellement un autre hétéroatome choisi parmi l'oxygène, le soufre ou l'azote, ledit hétérocycle pouvant être lui même substitué par un ou plusieurs radicaux (alcoyle, hydroxyalcoyle, alcoyloxyalcoyle, alcoyloxy-carbonylalcoyle, aryle, hétérocyclyle, hétérocyclylalcoyle saturés ou insaturés de 3 à 8 chaînons ou -CH₂-CO-NR"R"'), ou bien R" et/ou R''' peuvent être un radical hydroxyalcoyle, phényle, hétérocyclylalcoyle saturé ou insaturé de 3 à 8 chaînons, -CO-NR"R''' pour lequel NR"R"' est défini comme précédemment, ou alcoyle ou acyle substitués par NR''R''' défini tel que ci-dessus], ou bien R'₃ peut être choisi parmi des radicaux phényle ou phénylalcoyle substitués sur le radical phényle par un ou plusieurs radicaux [choisis parmi alcoyle, pouvant être substitués par un radical alcoyloxy ou alcoylthio éventuellement portant eux-même un radical carboxy ou un radical NR"R''' tel que défini ci-dessus, ou choisis parmi acyloxy pouvant être substitué par NR"R''' défini tel que précédemment], ou bien R'₃ peut être choisi parmi des radicaux alcoyle ou cycloalcoyle éventuellement substitués [par un radical carboxy, carboxyalcoyldisulfanyle ou par un radical NR"R"', -CH₂-NR"R''', -CO-NR"R''', ou par un radical alcoyloxycarbonyle, alcoyloxy, ou alcoyldisulfanyle éventuellement substitués par NR"R''' ou -CO-NR"R''' pour lesquels NR"R"' est défini tel que précédemment], ou bien R'₃ peut être choisi parmi des radicaux hétérocyclyle saturés ou insaturés de 3 à 8 chaînons éventuellement substitués [par alcoyle ou acylè eux-même éventuellement substitués par NR"R'''].

Il est entendu dans la formule générale (β) ci-dessus que lorsque R₁ est halogène, il peut être choisi parmi chlore, brome, fluor ou iode, et que les associations des dérivés selon l'invention et des streptogramines du groupe A entrent également dans le cadre de la présente invention.

In vitro sur *Staphylococcus aureus* 209P, les dérivés de streptogramine selon l'invention se sont montrés actifs à des concentrations comprises entre 0,25 et 32 µg/ml associés à un dérivé du groupe A des streptogramines comme la pristinamycine IIB et à des concentrations comprises entre 0,5 et 32 µg/ml sur *Staphylococcus aureus* Schiclia (résistant à la méticilline) associés à la pristinamycine IIB ; in vivo, ils synergisent l'activité antimicrobienne de la pristinamycine II_{B} sur les infections expérimentales de la souris à *Staphylococcus aureus* IP8203 à des doses comprises entre 10 et 150 mg/kg par voie sous cutanée (DC₅₀) et par voie orale, à des doses comprises entre 24 et 150 mg/kg (DC₅₀) [associations 30/70].

Enfin, les produits selon l'invention sont particulièrement intéressants du fait de leur faible toxicité. Aucun des produits n'a manifesté de toxicité à à la dose de 150 mg/kg par voie orale (2 administrations).

Parmi ces produits les produits de formule générale (I) dans laquelle :
R représente un radical -NR₁R₂ ou -SR₃ pour lequel :
   R₁ et R₂ identiques ou différents représentent un atome d'hydrogène, un radical alcoyle (1 à 8 carbones) éventuellement substitué par hydroxy, alcényle (3 à 8 carbones), cycloalcoyle (3 à 8 carbones), alcoyloxy (1 à 8 carbones), dialcoylamino, phénylalcoyle éventuellement substitué [par un ou plusieurs atomes d'halogène ou radicaux alcoyle, hydroxyalcoyle, alcoyloxy ou dialcoylamino], hétérocyclylalcoyle saturé ou insaturé (3 à 8 chaînons) contenant 1 ou plusieurs hétéroatomes choisis parmi l'azote, le soufre ou l'oxygène, ou dialcoylaminoalcoyle, ou bien
   R₁ et R₂ forment ensemble avec l'atome d'azote auquel ils sont attachés, un hétérocycle mono ou polycyclique, saturé, partiellement saturé ou insaturé, de 3 à 12 chaînons, contenant éventuellement un autre hétéroatome choisi parmi l'oxygène le soufre ou l'azote et éventuellement substitué [par un ou plusieurs radicaux hydroxy, alcoyle, phényle éventuellement substitué par un atome d'halogène, phénylalcoyle, hydroxyalcoyle, acyle, alcoyloxycarbonyle, ou hétérocyclyle ou hétérocyclylcarbonyle dont la partie hétérocyclyle est saturée ou insaturée (4 à 6 chaînons) et contient 1 ou plusieurs hétéroatomes choisis parmi l'oxygène le soufre ou l'azote],
   R₃ est un radical alcoyle (contenant 1 à 8 atomes de carbone) substitué par un radical -NR₁R₂ pour lequel R₁ et R₂ identiques ou différents représentent un radical alcoyle ou forment ensemble avec l'atome d'azote auquel ils sont attachés, un hétérocycle tel que défini ci-dessus, ou bien R₃ représente un radical hétérocyclyle mono ou polycyclique ou hétérocyclylméthyle saturé ou insaturé contenant 3 à 7 chaînons et éventuellement un autre hétéroatome choisi parmi l'oxygène le soufre ou l'azote et éventuellement substitué par un radical alcoyle. 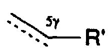
représente le reste d'un cycle insaturé non substitué en 5γ : 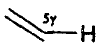 ou le reste d'un cycle saturé substitué en 5γ par un radical fluoro : 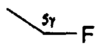 Ra est un radical éthyle, et
Rb, Rc et Rd ont les définitions ci-après :
1) Rb et Rc sont des atomes d'hydrogène et Rd est radical méthylamino ou diméthylamino,
2) Rb est un atome d'hydrogène, Rc est un atome d'hydrogène ou de chlore et Rd est un radical -NMe-R"' pour lequel R''' représente un radical alcényle (2 à 8C), hétérocyclylméthyle ou représente -COOR'e ou R'e est alcoyle (1 à 6 carbones), alcényle (2 à 6 carbones), phényle ou tolyle
3) Rb est un atome d'hydrogène, Rd est un radical -NHCH₃ ou -N(CH₃)₂ et Rc est un atome de chlore
sont particulièrement intéressants.

Et parmi ces produits, notamment les
- 5δ-(1-morpholino)méthyl 5δ-5γ-déhydro pristinamycine I_{E}
- 5δ-[N-méthyl N-2-(1,3-dioxolanyl)méthyl]aminométhyl-5δ,5γ-déhydro pristinamycine I_{E}
- 5δ-morpholinométhyl-4ζ-méthylamino-4ζ-désdiméthylamino-5δ,5γ-déhydro pristinamycine I_{E}
- 5δ-morpholinométhyl-4ζ-méthylamino-4ζ-désdiméthylamino-5δ,5γ-déhydro-4ε-chloro pristinamycine I_{E}
- 5δ-[bis-(2-méthoxyéthyl)aminométhyl]-5δ,5γ-déhydro pristinamycine I_{E}
sont plus spécialement actifs.

Les produits cités dans les exemples sont particulièrement préférés ; les dérivés de streptogramines ci-après sont également des produits intéressants :
- 4ε-chloro-5δ-(diéthylaminoéthylthiométhyl)-5δ,5γ-déhydro pristinamycine I_{E}
- 5δ-(diéthylaminoéthylthiométhyl)-4ζ-méthylamino-4ζ-désdiméthylamino-5δ,5γ-déhydro pristinamycine I_{E}
- 4ε-chloro-5δ-(diéthylaminoéthylthiométhyl)-4ζ-méthylamino)-4ζ-désdiméthylamino-5δ,5γ-déhydro pristinamycine I_{E}
- 5δ-(3-diéthylaminopropylthiométhyl)-5δ,5γ-déhydro pristinamycine I_{E}
- 4ε-chloro-5δ-(3-diéthylaminopropylthiométhyl)-5δ,5γ-déhydro pristinamycine I_{E}
- 5δ-(3-diéthylaminopropylthiométhyl)-4ζ-méthylamino-4ζ-désdiméthylamino-5δ,5γ-déhydro pristinamycine I_{E}
- 4ε-chloro-5δ-(3-diéthylaminopropylthiométhyl)-4ζ-méthylamino-4ζ-désdiméthylamino-5δ,5γ déhydro pristinamycine I_{E}
- 5δ-(diméthylaminoéthylthiométhyl)-5δ,5γ-déhydro pristinamycine I_{E}
- 4ε-chloro-5δ-(diméthylaminoéthylthiométhyl)-5δ,5γ-déhydro pristinamycine I_{E}
- 5δ-(diméthylaminoéthylthiométhyl)-4ζ-méthylamino-4ζ-désdiméthylamino-5δ,5γ-déhydro pristinamycine I_{E}
- 4ε-chloro-5δ-(diméthylaminoéthylthiométhyl)-4ζ-méthylamino-4-ζ-désdiméthylamino-5δ,5γ-déhydro pristinamycine I_{E}
- 4ε-chloro-5δ-(1-méthyl-2-imidazolylthiométhyl)-5δ,5γ-déhydropristinamycine I_{E}
- 5δ-(1-méthyl-2-imidazolylthiométhyl)-4ζ-méthylamino-4ζ-désdiméthylamino-5δ,5γ déhydro pristinamycine I_{E}
- 4ε-chloro-5δ-(1-méthyl-2-imidazolylthiométhyl)-4ζ-méthylamino-4ζ-désdiméthylamino-5δ,5γ-déhydro pristinamycine I_{E}
- 5δ-(morpholinoéthylthiométhyl)-5δ,5γ-déhydro pristinamycine I_{E}
- 4ε-chloro-5δ-(morpholinoéthylthiométhyl)-5δ,5γ-déhydro pristinamycine I_{E}
- 5δ-(morpholinoéthylthiométhyl)-4ζ-méthylamino-4ζ-désdiméthylamino-5δ,5γ-déhydro pristinamycine I_{E}
- 4ε-chloro-5δ-(morpholinoéthylthiométhyl)-4-ζ-méthylamino-4ζ-désdiméthylamino-5δ,5γ-déhydro pristinamycine I_{E}
- 4ε-chloro-5δ-(2-pipéridinoéthyl)thiométhyl-5δ,5γ-déhydro pristinamycine I_{E}
- 5δ-(2-pipéridinoéthyl)thiométhyl-4ζ-méthylamino-4ζ-désdiméthylamino-5δ,5γ-déhydro pristinamycine I_{E}
- 4ε-chloro-5δ-(2-pipéridinoéthyl)thiométhyl-4ζ-méthylamino-4ζ-désdiméthylamino-5δ,5γ-déhydro pristinamycine I_{E}
- 5δ-(3-pipéridinopropyl)thiométhyl-5δ,5γ-déhydro pristinamycine I_{E}
- 4ε-chloro-5δ-(3-pipéridinopropyl)thiométhyl-5δ,5γ-déhydro pristinamycine I_{E}
- 5δ-(3-pipéridinopropyl)thiométhyl-4ζ-méthylamino-4-ζdésdiméthylamino-5δ,5γ-déhydro pristinamycine I_{E}
- 4ε-chloro-5δ-(3-pipéridinopropyl)thiométhyl-4ζ-méthylamino-4ζ-désdiméthylamino-5δ,5γ-déhydro pristinamycine I_{E}
- 4ε-chloro-5δ-(4-pyridylméthylthiométhyl)-5δ,5γ-déhydro pristinamycine I_{E}
- 5δ-(4-pyridylméthylthiométhyl)-4ζ-méthylamino-4ζ-désdiméthylamino-5δ,5γ-déhydro pristinamycine I_{E}
- 4ε-chloro-5δ-(4-pyridylméthylthiométhyl-4ζ-méthylamino-4ζ-désdiméthylamino-5δ,5γ-déhydro pristinamycine I_{E}
- 4ε-chloro-5δ-(3-pyridylméthylthiométhyl)-5δ,5γ-déhydro pristinamycine I_{E}
- 5δ-(3-pyridylméthylthiométhyl)-4ζ-méthylamino-4ζ-désdiméthylamino-5δ,5γ-déhydro pristinamycine I_{E}
- 4ε-chloro-5δ-(3-pyridylméthylthiométhyl)-4ζ-méthylamino-4ζ-désdiméthylamino-5δ,5γ-déhydro pristinamycine I_{E}
- 5δ-(2-pyridylméthylthiométhyl)-5δ,5γ-déhydro pristinamycine I_{E}
- 4ε-chloro-5δ-(2-pyridylméthylthiométhyl)-5δ,5γ-déhydro pristinamycine I_{E}
- 5δ-(2-pyridylméthylthiométhyl)-4ζ-méthylamino-4ζ-désdiméthylamino-5δ,5γ-déhydro pristinamycine I_{E}
- 4ε-chloro-5δ-(2-pyridylméthylthiométhyl)-4ζ-méthylamino-4ζ-désdiméthylamino-5δ,5γ-déhydro pristinamycine I_{E}
- 5δ-{[2-(4-méthylpipérazin-1-yl)éthyl]thiométhyl}-5δ,5γ-déhydro pristinamycine I_{E}
- 4ε-chloro-5δ-{[2-(4-méthylpipérazin-1-yl)éthyl]thiométhyl}-5δ,5γ-déhydro pristinamycine I_{E}
- 5δ-{[2-(4-méthylpipérazin-1-yl)éthyl]thiométhyl}-4ζ-méthylamino-4ζ-désdiméthylamino-5δ,5γ-déhydro pristinamycine I_{E}
- 4ε-chloro-5δ-{[2-(4-méthylpipérazin-1-yl)éthyl]thiométhyl}-4ζ-méthylamino-4ζ-désdiméthylamino-5δ,5γ-déhydro pristinamycine I_{E}
- 5δ-(butoxycarbonylaminoéthylthiométhyl)-5δ,5γ-déhydro pristinamycine I_{E}
- 4ε-chloro-5δ-(butoxycarbonylaminoéthylthiométhyl)-5δ,5γ-déhydro pristinamycine I_{E}
- 5δ-(butoxycarbonylaminométhylthioéthyl)-4ζ-méthylamino-4ζ-désdiméthylamino-5δ,5γ-déhydro pristinamycine I_{E}
- 4ε-chloro-5δ-(butoxycarbonylaminoéthylthiométhyl)-4ζ-méthylamino-4ζ-désdiméthylamino 5δ,5γ-déhydro pristiriamycine I_{E}
- 5δ-(aminoéthylthiométhyl)-5δ,5γ-déhydro pristinamycine I_{E}
- 4ε-chloro-5δ-(aminoéthylthiométhyl)-5δ,5γ-déhydro pristinamycine I_{E}
- 5δ-(aminométhylthioéthyl)-4ζ-méthylamino-4ζ-désdiméthylamino-5δ,5γ-déhydro pristinamycine I_{E}
- 4ε-chloro-5δ-(aminoéthylthiométhyl)-4ζ-méthylamino-4ζ-désdiméthylamino-5δ,5γ-déhydro pristinamycine I_{E}
- 5δ-(pyrrolidinoéthylthiométhyl)-5δ,5γ-déhydro pristinamycine I_{E}
- 4ε-chloro-5δ-(pyrrolidinoéthylthiométhyl)-5δ,5γ-déhydro pristinamycine I_{E}
- 5δ-(pyrrolidinoéthylthiométhyl)-4ζ-méthylamino-4ζ-désdiméthylamino-5δ,5γ-déhydro pristinamycine I_{E}
- 4ε-chloro-5δ-(pyrrolidinoéthylthiométhyl)-4ζ-méthylamino-4ζ-désdiméthylamino-5δ,5γ-déhydro pristinamycine I_{E}
- 5δ-(diisopropylaminoéthylthiométhyl)-5δ,5γ-déhydro pristinamycine I_{E}
- 4ε-chloro-5δ-(diisopropylaminoéthylthiométhyl)-5δ,5γ-déhydro pristinamycine I_{E}
- 5δ-(diisopropylaminoéthylthiométhyl)-4ζ-méthylamino-4ζ-désdiméthylamino-5δ,5γ-déhydro pristinamycine I_{E}
- 4ε-chloro-5δ-(diisopropylaminoéthylthiométhyl)-4ζ-méthylamino)-4ζ-désdiméthylamino-5δ,5γ-déhydro pristinamycine I_{E}
- 5δ-(N-éthyl N-méthylaminoéthylthiométhyl)-5δ,5γ-déhydro pristinamycine I_{E}
- 4ε-chloro-5δ-(N-éthyl N-méthylaminoéthylthiométhyl)-5δ,5γ-déhydro pristinamycine I_{E}
- 5δ-(N-éthyl N-méthylaminoéthylthiométhyl)-4ζ-méthylamino-4ζ-désdiméthylamino-5δ,5γ-déhydro pristinamycine I_{E}
- 4ε-chloro-5δ-(N-éthyl N-méthylaminoéthylthiométhyl)-4ζ-méthylamino)-4ζ-désdiméthylamino-5δ,5γ-déhydro pristinamycine I_{E}
- 5δ-((2R)-3-diéthylaminopropyl-2-thiométhyl)-5δ,5γ-déhydro pristinamycine I_{E}
- 4ε-chloro-5δ-((2R)-3-diéthylaminopropyl-2-thiométhyl)-5δ,5γ-déhydro pristinamycine I_{E}
- 5δ-((2R)-3-diéthylaminopropyl-2-thiométhyl)-4ζ-méthylamino-4ζ-désdiméthylamino-5δ,5γ-déhydro pristinamycine I_{E}
- 4ε-chloro-5δ-((2R)-3-diéthylaminopropyl-2-thiométhyl)-4ζ-méthylamino)-4ζ-désdiméthylamino-5δ,5γ-déhydro pristinamycine I_{E}
- 5δ-((2S)-3-diéthylaminopropyl-2-thiométhyl)-5δ,5γ-déhydro pristinamycine I_{E}
- 4ε-chloro-5δ-((2S)-3-diéthylaminopropyl-2-thiométhyl)-5δ,5γ-déhydro pristinamycine I_{E}
- 5δ-((2S)-3-diéthylaminopropyl-2-thiométhyl)-4ζ-méthylamino-4ζ-désdiméthylamino-5δ,5γ-déhydro pristinamycine I_{E}
- 4ε-chloro-5δ-((2S)-3-diéthylaminopropyl-2-thiométhyl)-4ζ-méthylamino)-4ζ-désdiméthylamino-5δ,5γ-déhydro pristinamycine I_{E}

Les dérivés de streptogramine de formule générale (α) sont décrits, ainsi que leur préparation, dans la demande internationale WO 99/05165.

Les dérivés de streptogramine de formule générale (β) décrits dans la demande internationale WO 01/02427 sont préparés par halogénation, par transformation en azide ou par transformation en thiocyanate, d'un dérivé de streptogramine de formule générale : 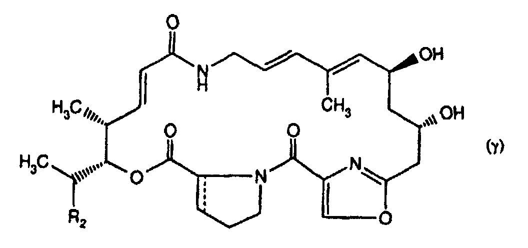 dans laquelle R₂ est défini comme précédemment, la liaison --- représente une liaison simple (stéréochimie 27R) ou une liaison double, et dont la fonction hydroxy en position 14 a été préalablement protégée, suivie de l'élimination du radical protecteur et le cas échéant, pour obtenir un dérivé (β) pour lequel R₃ est autre que l'atome d'hydrogène, par introduction du reste d'ester aliphatique, cycloaliphatique, aromatique, araliphatique, hétérocyclique ou hétérocyclylaliphatique pouvant être substitué (R₃) selon les méthodes habituelles qui n'altèrent pas le reste de la molécule.

Les réactions d'halogénation, de transformation en azide ou de transformation en thiocyanate peuvent être mises en oeuvre en présence d'un trifluorure d'aminosoufre (trifluorure de diéthylamino soufre, trifluorure de bis(2-méthoxyéthyl)amino soufre (Deoxofluor®), trifluorure de morpholino soufre) ou alternativement en présence de tétrafluorure de soufre, au moyen d'un réactif comme un halogénure, un azoture ou un thiocyanate de tétra alkylammonium (tétra méthylammonium, tétra éthylammonium, tétra propylammonium, tétra butylammonium), de tri alkyl benzylammonium ou de tri alkyl phénylammonium ou au moyen d'un halogénure, d'un azoture ou d'un thiocyanate de métal alcalin additionné éventuellement d'un éther couronne. La réaction s'effectue dans un solvant organique chloré (dichlorométhane, dichloréthane, chloroforme) ou dans un éther (tétrahydrofurane) entre -78 et 40°C, de préférence sous argon ou sous azote. La mise en oeuvre du dérivé hydroxy de configuration (16S) conduit au dérivé de configuration (16R). La protection et la déprotection du radical hydroxy en position 14 s'effectue selon les méthodes habituelles qui n'affectent pas le reste de la molécule [T.W. Greene et P.G.M. Wuts, Protective Groups in Organic Synthesis (2^{ème} éd.), A. Wiley - Interscience Publication (1991)].

Pour préparer un produit (β) pour lequel R₃ est un ester aliphatique, cycloaliphatique, aromatique, araliphatique, hétérocyclique ou hétérocyclylaliphatique pouvant être substitué, l'estérification est mise en oeuvre par réaction de l'acide ou d'un dérivé réactif de l'acide (chlorure d'acide, ester réactif, anhydride), en présence ou non d'un agent de couplage (carbodiimide : dicyclohexylcarbodiimide) et d'une amine tertiaire (trialcoylamine : triéthylamine, diisopropyléthylamine, pyridine ou un un dérivé) et éventuellement un catalyseur comme la 4-N-diméthylaminopyridine, à une température comprise entre -40 et + 80°C dans un solvant organique tel qu'un amide (diméthylformamide ou N-méthyl 2-pyrrolidinone par exemple), la pyridine, un solvant halogéné (dichlorométhane, dichloroéthane ou chloroforme par exemple) ou un éther (tétrahydrofurane, dioxane, diméthoxyéthane). Les fonctions pouvant interférer avec la réaction sont préalablement protégées.

Les exemples suivants donnés à titre non limitatif illustrent la présente invention.

Dans les exemples qui suivent, les spectres de RMN ont été étudiés dans le deutérochloroforme, la nomenclature utilisée est celle de J.O. Anteuriii et coll., Eur. Biochem., 58, 259 (1975) et notamment :

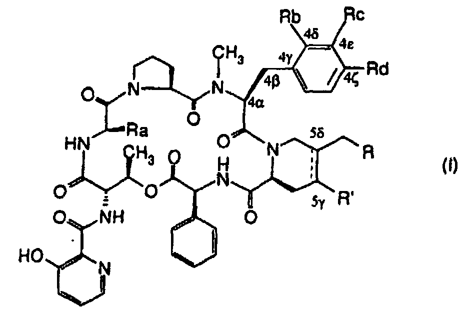

Les chromatographies sur colonne sont réalisées, sauf exception mentionnée, sous pression atmosphérique en utilisant une silice 0,063-0,02 mm. Dans quelques cas précisés, les purifications sont faites par chromatographie-flash en utilisant une silice de 0,04-0,063 mm, ou par chromatographie liquide haute performance (CLHP) sur silice greffée C₈ ou C₁₈. Au fur et à mesure de la chromatographie, les fractions sont analysées par chromatographie en couche mince (CCM), sur plaques de silice Merck 60F254 ou par HPLC analytique. Les fractions correspondants à un même Rf ou à un même temps de rétention sont regroupées puis concentrées à sec, sous pression réduite (30-45°C; 2,7 kPa). Les produits ainsi obtenus sont analysés par les techniques spectroscopiques habituelles (RMN; IR; MS), ce qui permet d'identifier les produits attendus.

### Exemple 1

Dans un tricol contenant 500 cm³ d'acétonitrile, on introduit 60 g d'un mélange brut contenant 33 % molaire de 5δ-chlorométhyl-5δ,5γ-déhydro pristinamycine I_{E} préparée dans les conditions décrites ci-après, puis 18 cm³ de morpholine. Le mélange est chauffé 5 heures à reflux puis est concentré à sec à 45°C sous pression réduite (2,7 kPa) pour donner 68,8 g d'un solide qui est repris par 200 cm³ d'eau saturée en bicarbonate de sodium et 200 cm³ de dichlorométhane. La phase organique est décantée puis séchée sur sulfate de sodium, filtrée et concentrée à sec à 45°C (2,7 kPa) pour donner 56,4 g d'un solide jaune auquel on ajoute 300 cm³ d'eau et 140 cm³ d'acide chlorhydrique 1N. La phase aqueuse obtenue est extraite successivement par 4 fois 100 cm³ d'acétate d'éthyle et 100 cm³ de dichlorométhane puis est amenée à pH 7-8 par ajout de 12 g de bicarbonate de sodium et extraite par 400 cm³ de dichlorométhane. La phase organique est décantée, séchée sur sulfate de sodium, filtrée puis concentrée à sec pour donner 41,3 g d'un solide jaune qui est purifié par chromatographie sur 200 g de silice (éluant : dichlorométhane/méthanol 98/2 en volumes). On obtient ainsi un solide qui est cristallisé dans un mélange méthanol/eau (90/10 en volumes). Une seconde cristallisation de 5,05 g du solide ainsi cristallisé dans 20 cm³ de méthanol conduit, après filtration et séchage à 45°C (90 Pa), à 4,5 g de 5δ-(1-morpholino)méthyl 5δ,5γ-déhydro pristinamycine I_{E}, sous forme d'une poudre blanchâtre fondant à 180°C.

Spectre de R.M.N. ¹H (400 MHz, CDCl₃) : 0,92 (t, J = 7,5 Hz, 3H : CH₃ en 2γ) ; 1,08 (d très large, J = 16,5 Hz, 1H : 1H du CH₂ en 5β) ; 1,27 (mt, 2H : 1H du CH₂ en 3β et 1H du CH₂ en 3γ) ; 1,30 (d, J = 7 Hz, 3H : CH₃ en 1γ) ; 1,57 (mt, 1H : l'autre H du CH₂ en 3γ) ; 1,66 et 1,72 (2 mts, 1H chacun : CH₂ en 2β) ; 2,00 (mt, 1H : l'autre H du CH₂ en 3β) ; 2,36 (mf, 4H : NCH₂ de la morpholine) ; 2,47 (dd large, J = 16,5 et 5 Hz, 1H : l'autre H du CH₂ en 5β) ; 2,85 (s, 2H : CH₂N) ; 2,94 (s, 6H : ArN(CH₃)₂) ; 2,99 (dd, J = 14 et 6,5 Hz, 1H : 1H du CH₂ en 4β) ; 3,17 (mt, 1H : l'autre H du CH₂ en 4β) ; 3,17 (s, 3H : NCH₃) ; 3,27 (mt, 1H : 1H du CH₂ en 3δ) ; 3,34 (d large, J = 18 Hz, 1H : 1H du CH₂ en 5ε) ; 3,47 (mt, 1H : l'autre H du CH₂ en 3δ) ; 3,70 (mt, 4H : CH₂O de la morpholine) ; 4,57 (dd, J = 8 et 5 Hz, 1H : CH en 3α) ; 4,78 (mt, 1H : CH en 2α) ; 4,82 (d large, J = 18 Hz, 1H : l'autre H du CH₂ en 5ε) ; 4,89 (dd, J = 10 et 1Hz, 1H : CH en 1α) ; 5,11 (d, J = 5 Hz, 1H : CH en 5α) ; 5,27 (dd, J = 9 et 6,5 Hz, 1H : CH en 4α) ; de 5,50 à 5,55 (mt, 1H : CH en 5γ) ; 5,52 (d, J = 8 Hz, 1H : CH en 6α) ; 5,87 (q dédoublé, J = 7 et 1 Hz, 1H : CH en 1β) ; 6,58 (d, J = 8 Hz, 2H : H aromatiques en 4ε) ; 6,65 (d, J = 9,5 Hz, 1H : CONH en 2) ; 6,92 (d, J = 8 Hz, 2H : H aromatiques en 4δ) ; de 7,20 à 7,45 (mt : les 7H correspondant aux H aromatiques en 6α - au 1' H₄ et au 1' H₅) ; 7,76 (dd, J = 4 et 1 Hz, 1H : 1' H₆) ; 8,47 (d, J = 10 Hz, 1H : CONH en 1) ; 8,51 (d, J = 8 Hz, 1H : CONH en 6) ; 11,69 (s, 1H : OH).

La 5δ-chlorométhyl-5δ,5γ-déhydro pristinamycine I_{E} peut être obtenue de la façon suivante :

Dans un tricol contenant 100 cm³ de tétrahydrofurane, on introduit 5,9 g de (5γR, 5γS)-5γ-hydroxy-5γ-désoxy-5δ-méthylène pristinamycine I_{A} (mélange 50/50 des deux isomères) puis 1 cm³ de chlorure de thionyle. Le mélange est agité une nuit à 20°C puis filtré. Le filtrat est concentré à sec à 45°C sous pression réduite pour donner un solide qui est repris par 100 cm³ d'eau saturée en bicarbonate de sodium et 100 cm³ de dichlorométhane. La phase organique est décantée, séchée sur sulfate de sodium, filtrée et concentrée à sec. On obtient ainsi 1,45 g d'un produit brut qui est purifié par deux chromatographies successives sur silice (éluant : dichlorométhane/méthanol 98/2 en volumes) pour donner 1,24 g de mélange brut contenant 50 % molaire de 5δ-chlorométhyl-5δ,5γ-déhydro pristinamycine I_{E} sous forme d'un solide jaune.

Les (5γR) et (5γS)-5γ-hydroxy-5γ-désoxy-5δ-méthylène pristinamycine I_{A} peuvent être obtenus de la façon suivante :

Dans un tricol contenant 100 cm³ de méthanol et 50 cm³ de dichlorométhane, on introduit 10 g de 5δ-méthylène pristinamycine I_{A} et 2,8 g de chlorure de cérium anhydre. A ce mélange refroidi à 0°C, on additionne, par petites portions, 0,47 g de borohydrure de sodium. Le mélange réactionnel est ensuite agité 3 heures puis dilué avec 100 cm³ d'eau. Le pH de la phase aqueuse est ajusté à 5 par addition d'acide acétique. Le mélange résultant est alors concentré sous pression réduite. La phase aqueuse résiduelle est amenée à pH 7 par addition d'eau saturée en bicarbonate de sodium puis extraite par 2 fois 50 cm³ de dichlorométhane. Les phases organiques sont rassemblées puis séchées sur sulfate de sodium, filtrées et concentrées à sec à 45°C sous pression réduite (2,7 kPa) pour donner 7,6 g d'un solide dont 2 g sont purifiés par 3 chromatographies liquides hautes performances (CLHP) préparatives successives sur 450 g de silice C₈ 10µm (éluant : eau-acétonitrile 70/30 en volumes, contenant 0,1 % d'acide trifluoroacétique). Les fractions contenant l'isomère 5γS attendu sont rassemblées, l'acétonitrile éliminé à 40°C sous pression réduite (2,7 kPa) et le pH de la phase aqueuse ajusté à 7 par addition d'eau saturée en bicarbonate de sodium. Le précipité apparu est filtré puis agité dans 20 cm³ d'éther diéthylique. Le solide résultant est filtré et séché à 40°C sous pression réduite (90 Pa) pour donner 0,19 g de (5γS)-5γ-hydroxy-5γ-désoxy 5δ-méthylène pristinamycine I_{A}, sous forme d'un solide blanc fondant à 166°C. En opérant de même pour les fractions contenant l'isomère 5γR, on obtient 0,18 g de (5γ-R)-5γ-hydroxy-5γ-désoxy-5δ-méthylène pristinamycine I_{A}, sous forme d'un solide blanc fondant à 246°C.

(5γS)-5γ-hydroxy-5γ-désoxy-5δ-méthylène pristinamycine I_{A} :

Spectre de R.M.N. ¹H (400 MHz, CDCl₃) : 0,47 (dt, J = 15 et 5,5 Hz, 1H : 1H du CH₂ en 5β) ; 0,92 (t, J = 7,5 Hz, 3H : CH₃ en 2γ) ; 1,10 (mt, 1H : 1H du CH₂ en 3β) ; de 1,25 à 1,40 (mt, 1H : 1H du CH₂ en 3γ) ; 1,35 (d, J = 7 Hz, 3H : CH₃ en 1γ) ; de 1,55 à 1,80 (mt : les 3H correspondant à l'autre H du CH₂ en 3γ et au CH₂ en 2β) ; 1,99 (mt, 1H : l'autre H du CH₂ en 3β) ; 2,13 (d large, J = 15 Hz, 1H : l'autre H du CH₂ en 5β) ; 2,90 (dd, J = 13 et 5 Hz, 1H : 1H du CH₂ en 4β) ; 2,98 (s, 6H : ArN(CH₃)₂) ; de 3,15 à 3,35 (mt, 2H : l'autre H du CH₂ en 4β et 1 H du CH₂ en 3δ) ; 3,20 (s, 3H : NCH₃) ; 3,38 (d, J = 14,5 Hz, 1H : 1H du CH₂ en 5ε) ; 3,52 (mt, 1H : l'autre H du CH₂ en 3δ) ; 3,90 (mt, 1H : CH en 5γ) ; 4,53 (t, J = 7,5 Hz, 1H : CH en 3α) ; 4,81 (mt, 1H : CH en 2α) ; 4,88 (d, J = 14,5 Hz, 1H : l'autre H du CH₂ en 5ε) ; 4,91 (d large, J = 10 Hz, 1H : CH en 1α) ; 5,03 (d large, J = 5,5 Hz, 1H : CH en 5α) ; 4,95 et 5,00 (2 s larges, 1H chacun : =CH₂) ; 5,17 (dd, J = 11 et 5 Hz, 1H : CH en 4α) ; 5,70 (d, J = 8 Hz, 1H : OH en 5γ) ; 5,77 (d, J = 8,5 Hz, 1H : CH en 6α) ; 5,92 (q large, J = 7 Hz, 1H : CH en 1β) ; 6,54 (d, J = 9,5 Hz, 1H : CONH en 2) ; 6,60 (d, J = 8 Hz, 2H : H aromatiques en 4ε) ; 6,92 (d, J = 8 Hz, 2H : H aromatiques en 4δ) ; de 7,20 à 7,35 (mt : les 5H correspondant aux H aromatiques en 6α) ; 7,38 (AB limite, 2H 1' H₄ et 1' H₅) ; 7,78 (mt, 1H : 1' H₆) ; 8,44 (d, J = 10 Hz, 1H : CONH en 1) ; 9,10 (d, J = 8,5 Hz : CONH en 6) ; 11,66 (s, 1H : OH).

(5γR)-5γ-hydroxy 5γ-désoxy 5δ-méthylène pristinamycine I_{A} :

Spectre de R.M.N. ¹H (400 MHz, CDCl₃) : 0,17 (t dédoublé, J = 12 et 5,5 Hz, 1H : 1H du CH₂ en 5β) ; 0,91 (t, J = 7,5 Hz, 3H : CH₃ en 2γ) ; 1,18 (mt, 1H : 1H du CH₂ en 3β) ; 1,28 (mt, 1H : 1H du CH₂ en 3γ) ; 1,34 (d, J = 7 Hz, 3H : CH₃ en 1γ) ; 1,57 (mt, 1H : l'autre H du CH₂ en 3γ) ; de 1,60 à 1,80 (mt : 2H correspondant au CH₂ en 2β) ; 1,99 (mt, 1H : l'autre H du CH₂ en 3β) ; 2,39 (dd, J = 12 et 6 Hz, 1H : l'autre H du CH₂ en 5β) ; de 2,90 à 3,00 (mt, 1H : 1H du CH₂ en 4 β) ; 2,96 (s, 6H : ArN(CH₃)₂) ; 3,06 (d, J = 14 Hz, 1H : 1H du CH₂ en 5ε) ; 3,17 (s, 3H : NCH₃) ; 3,20 (dd, J = 13,5 et 10 Hz, 1H : l'autre H du CH₂ en 4β) ; 3,28 et 3,49 (2 mts, 1H chacun : CH₂ en 3δ) ; 4,55 (dd, J = 8 et 7 Hz, 1H : CH en 3α) ; 4,70 (mt, 1H : CH en 5γ) ; 4,79 (mt, 1H : CH en 2α) ; 4,87 (d large, J = 10 Hz, 1H : CH en 1α) ; de 5,00 à 5,15 (mt, 3H : CH en 5α - l'autre H du CH₂ en 5ε et 1H du =CH₂) ; 5,17 (s large, 1H : l'autre H du =CH₂) ; 5,21 (dd, J = 10 et 6,5 Hz, 1H : CH en 4α) ; 5,65 (d, J = 8,5 Hz, 1H : CH en 6α) ; 5,90 (mt, 1H : CH en 1β) ; 6,56 (d, J =10 Hz, 1H : CONH en 2) ; 6,59 (d, J = 8 Hz, 2H : H aromatiques en 4ε) ; 6,91(d, J = 8 Hz, 2H : H aromatiques en 4δ) ; de 7,20 à 7,45 (mt : les 7H correspondant aux H aromatiques en 6α - au l'H₄ et au l' H₅) ; 7,76 (d large, J = 4 Hz, 1H : l'H₆) ; 8,41 (d, J = 10 Hz, 1H : CONH en 1) ; 8,61 (d, J = 8,5 Hz : CONH en 6) ; 11,66 (s, 1H : OH).

### Exemple 2

Dans un tricol contenant 350 cm³ de dichlorométhane, on introduit 21,2 g de 5δ-(1-morpholino)méthyl-5γ-désoxy-5γ-hydroxy pristinamycine I_{A} (mélange des isomères en 5δ et 5γ). Au mélange refroidi à 0°C, on ajoute 14,3 cm³ de diéthylaminosulfure trifluorure. Après addition, le mélange réactionnel est agité à 20°C durant 18 heures puis versé sur 400 cm³ d'eau. Le pH de la phase aqueuse est amené à 7 par addition d'eau saturée en bicarbonate de sodium. La phase organique est décantée, lavée par 200 cm³ d'eau, séchée sur sulfate de sodium, filtrée, concentrée à sec et reprise par 200 cm³ d'acétate d'éthyle. L'insoluble apparu est filtré et le filtrat concentré à sec à 45°C, sous pression réduite (2,7 kPa). On obtient 10,6 g d'un produit brut qui est purifié par chromatographie sur 250 g de silice (éluant : dichlorométhane/méthanol 97,5/2,5 en volumes) pour donner 4,5 g d'un solide qui est purifié en deux lots (1,5 g et 3 g), par deux CLHP préparatives successives sur 450 g de silice C₈ 10µm (éluant : eau-acétonitrile 70/30 en volumes, contenant 0,1 % d'acide trifluoroacétique). Les fractions, respectivement 4 à 8 et 3 à 5, sont rassemblées, l'acétonitrile éliminé à 40°C sous pression réduite (2,7 kPa) et le pH de la phase aqueuse résiduelle ajusté à 7 par addition d'eau saturée en bicarbonate de sodium. Le précipité apparu est filtré, rincé par 20 cm³ d'eau et 20 cm³ d'éther diisopropylique puis séché à 40°C sous pression réduite (90 Pa) pour donner 0,35 g (1,7 %) de 5δ-(1-morpholino)méthyl-5δ,5γ-déhydro pristinamycine I_{E}.

Spectre de R.M.N. ¹H (400 MHz, CDCl₃) : 0,92 (t, J = 7,5 Hz, 3H : CH₃ en 2γ) ; 1,08 (d très large, J = 16,5 Hz, 1H : 1H du CH₂ en 5β) ; 1,27 (mt, 2H : 1H du CH₂ en 3β et 1H du CH₂ en 3γ) ; 1,30 (d, J = 7 Hz, 3H : CH₃ en 1γ) ; 1,57 (mt, 1H : l'autre H du CH₂ en 3γ) ; 1,66 et 1,72 (2 mts, 1H chacun : CH₂ en 2β) ; 2,00 (mt, 1H : l'autre H du CH₂ en 3β) ; 2,36 (mf, 4H : NCH₂ de la morpholine) ; 2,47 (dd large, J = 16,5 et 5 Hz, 1H : l'autre H du CH₂ en 5β) ; 2,85 (s, 2H : CH₂N) ; 2,94 (s, 6H : ArN(CH₃)₂) ; 2,99 (dd, J = 14 et 6,5 Hz, 1H : 1H du CH₂ en 4β) ; 3,17 (mt, 1H : l'autre H du CH₂ en 4β) ; 3,17 (s, 3H : NCH₃) ; 3,27 (mt, 1H : 1H du CH₂ en 3δ) ; 3,34 (d large, J = 18 Hz, 1H : 1H du CH₂ en 5ε) ; 3,47 (mt, 1H : l'autre H du CH₂ en 3δ) ; 3,70 (mt, 4H : CH₂O de la morpholine) ; 4,57 (dd, J = 8 et 5 Hz, 1H : CH en 3α) ; 4,78 (mt, 1H : CH en 2α) ; 4,82 (d large, J = 18 Hz, 1H : l'autre H du CH₂ en 5ε) ; 4,89 (dd, J = 10 et 1Hz, 1H : CH en 1α) ; 5,11 (d, J = 5 Hz, 1H : CH en 5α) ; 5,27 (dd, J = 9 et 6,5 Hz, 1H : CH en 4α) ; de 5,50 à 5,55 (mt, 1H : CH en 5γ) ; 5,52 (d, J = 8 Hz, 1H : CH en 6α) ; 5,87 (q dédoublé, J = 7 et 1 Hz, 1H : CH en 1β) ; 6,58 (d, J = 8 Hz, 2H : H aromatiques en 4ε) ; 6,65 (d, J = 9,5 Hz, 1H : CONH en 2) ; 6,92 (d, J = 8 Hz, 2H : H aromatiques en 4δ) ; de 7,20 à 7,45 (mt : les 7H correspondant aux H aromatiques en 6α - au 1' H₄ et au 1' H₅) ; 7,76 (dd, J = 4 et 1 Hz, 1H : 1' H₆) ; 8,47 (d, J = 10 Hz, 1H : CONH en 1) ; 8,51 (d, J = 8 Hz, 1H : CONH en 6) ; 11,69 (s, 1H : OH).

La 5δ-(1-morpholino)méthyl-5γ-désoxy-5γ-hydroxy pristinamycine I_{A} (mélange des isomères en 5δ et 5γ) peut être obtenu de la façon suivante :

On place dans un ballon 11 g de 5δ-(1-morpholino)méthyl pristinamycine I_{A} (mélange d'isomères 5δS et 5δR 90/10) dans 120 cm³ de 1,2-diméthoxyéthane puis on ajoute 0,42 g de borohydrure de sodium. Après 1 nuit d'agitation à 20°C, on ajoute 60 cm³ d'isopropanol et 0,42 g de borohydrure de sodium supplémentaire, puis l'agitation est poursuivie 5 heures. Le mélange réactionnel est concentré à sec sous pression réduite, dilué par 40 cm³ de dichlorométhane et 400 cm³ d'eau distillée additionnée d'acide chlorhydrique 1N de façon à ajuster le pH à 3. La phase aqueuse est décantée puis lavée par 3 fois 30 cm³ de dichlorométhane. Les phases organiques sont réunies, séchées sur sulfate de sodium, filtrées et concentrée à sec sous pression réduite pour donner 10,84 g d'un solide qui est purifiée par chromatographie flash (éluant en gradient : CH₂Cl₂: MeOH 98/2 à 96/4 en volumes) pour donner 1,6 g d'un produit qui est agité dans 50 cm³ d'éther diéthylique. Après filtration et séchage à 50°C sous pression réduite (90 Pa), on obtient 1,14 g de 5δ-(1-morpholino)méthyl-5γ-désoxy-5γ-hydroxy pristinamycine IA (mélange des isomères 90/10 en 5δ et 50/50 en 5γ), sous forme d'un solide blanc fondant vers 180°C (peu net).

Spectre de R.M.N. ¹ H (400 MHz, CDCl₃, d en ppm). 0,42 (dt, J = 15 et 5 Hz, 1H : 1H du CH₂ en 5 b) ; 0,89 (t, J = 7,5 Hz, 3H : CH₃ en 2 g) ; 1,02 (mt, 1H : 1H du CH₂ en 3 b) ; de 1,15 à 1,35 (mt, 1H : 1H du CH₂ en 3 g) ; 1,33 (d, J = 7 Hz, 3H : CH₃ en 1 g) ; de 1,45 à 1,80 (mt : les 4H correspondant au CH en 5 d - à l'autre H du CH₂ en 3 g et au CH₂ en 2 b) ; 1,97 (mt, 1H : l'autre H du CH₂ en 3 b) ; 2,13 (d large, J = 15 Hz, 1H : l'autre H du CH₂ en 5 b) ; 2,27 (dd, J = 12 et 6 Hz, 1H : 1H du CH₂N) ; de 2,30 à 2,50 (mt, 4H : l'autre H du CH₂N - 1H des 2 NCH₂ de la morpholine et 1H du CH₂ en 5 e) ; de 2,45 à 2,60 (mt, 2H : l'autre H des 2 NCH₂ de la morpholine) ; de 2,80 à 3,00 (mt, 1H : 1H du CH₂ en 4 b) ; 2,96 (s, 6H : ArN(CH₃)₂) ; de 3,10 à 3,30 (mt, 2H : 1H du CH₂ en 3 d et l'autre H du CH₂ en 4 b) ; 3,21 (s, 3H : NCH₃) ; de 3,40 à 3,60 (mt, 2H : l'autre 1H du CH₂ en 3 d et CH en 5 g) ; 3,76 (mt, 4H : les 2 OCH₂ de la morpholine) ; 4,46 (d large, J = 13 Hz, 1H : l'autre H du CH₂ en 5 e) ; 4,50 (t, J = 8 Hz, 1H : CH en 3 a) ; 4,81 (mt, 1H : CH en 2 a) ; 4,90 (dd, J = 10 et 1 Hz, 1H : CH en 1 a) ; 5,04 (d large, J = 5 Hz, 1H : CH en 5 a) ; de 5,25 à 5,35 (mt, 2H : OH en 5 g et CH en 4 a) ; 5,80 (d, J = 9 Hz, 1H : CH en 6 a) ; 5,98 (dq, J = 7 et 1 Hz, 1H : CH en 1 b) ; 6,56 (d, J = 10 Hz, 1H : CONH en 2) ; 6,59 (d, J = 8,5 Hz, 2H : H aromatiques en 4 e) ; 6,99 (d, J = 8,5 Hz, 2H : H aromatiques en 4 d) ; de 7,15 à 7,35 (mt : les 5H aromatiques en 6) ; 7,40 (AB limite, 2H : 1' H₄ et 1' H₅) ; 7,86 (dd, J = 4 et 2 Hz, 1H : 1' H₆) ; 8,48 (d, J = 10 Hz, 1H : CONH en 1) ; 9,15 (d, J = 9 Hz, 1H : CONH en 6) ; 11,66 (s, 1H : OH).

La 5δ-(1-morpholino)méthyl pristinamycine I_{A} (mélange d'isomères 5δR et 5δS 90/10) peut être obtenue par la méthode suivante : on place dans un ballon maintenu sous atmosphère d'azote 4 g de 5δ-méthylène pristinamycine IA en solution dans un mélange de 10 cm³ de dichlométhane et de 50 cm³ de méthanol puis on ajoute 1,8 cm³ de morpholine. Le mélange est laissé 4 jours sous agitation, concentré à sec sous pression réduite à 30°C, puis agité dans 40 cm³ d'éther diéthylique. Le surnageant est soutiré puis le solide résultant agité dans 40 cm³ d'éther diéthylique, filtré puis séché sous pression réduite (2,7 kPa) pour donner 3,54 g de 5δ-(1-morpholino)méthyl pristinamycine IA (mélange 90/10 d'isomères 5δR et 5δS), sous forme d'un solide blanchâtre contenant une mole de morpholine par mole de produit. Ce solide est utilisé tel quel.

Spectre de R.M.N. ¹H (400 MHz, CDCl₃, d en ppm). 0,68 (dd, J = 15 et 5,5 Hz, 1H : 1H du CH₂ en 5 b) ; 0,91 (t, J = 7,5 Hz, 3H : CH₃ en 2 g) ; 1,12 (mt, 1H : 1H du CH₂ en 3 b) ; de 1,15 à 1,35 (mt, 1H : 1H du CH₂ en 3 g) ; 1,32 (d, J = 7 Hz, 3H : CH₃ en 1 g) ; de 1,45 à 1,70 (mt, 2H : l'autre H du CH₂ en 3 g et 1H du CH₂ en 2 b) ; 1,75 (mt, 1H : l'autre H du CH₂ en 2 b) ; 2,01 (mt, 1H : l'autre H du CH₂ en 3 b) ; de 2,20 à 2,45 (mt, 5H : CH en 5 d - l'autre H du CH₂ en 5 b - 1H du NCH₂ et 1H des 2 NCH₂ de la morpholine) ; de 2,40 à 2,60 (mt, 3H : 1H du CH₂ en 5 e et l'autre H des 2 NCH₂ de la morpholine) ; 2,79 (dd, J = 12,5 et 4 Hz, 1H : l'autre H du NCH₂) ; de 2,80 à 2,95 (mt, 1H : 1H du CH₂ en 4 b) ; 2,92 (s, 6H : ArN(CH₃)₂) ; de 3,15 à 3,25 (mt, 1H : 1H du CH₂ en 3 d) ; 3,25 (s, 3H : NCH₃) ; 3,32 (t, J = 12 Hz, 1H : l'autre H du CH₂ en 4 b) ; 3,53 (mt, 1H : l'autre H du CH₂ en 3 d) ; 3,72 (mt : les 4H correspondant aux 2 CH₂O de la morpholine) ; 4,54 (t, J = 8 Hz, 1H : CH en 3 a) ; 4,82 (mt, 1H : CH en 2 a) ; 4,87 (dd, J = 10 et 1 Hz, 1H : CH en 1 a) ; 4,95 (dd large, J = 13 et 6 Hz, 1H : l'autre H du CH₂ en 5 e) ; 5,25 (dd, J = 12 et 4 Hz, 1H : CH en 4 a) ; 5,29 (d large, J = 5,5 Hz, 1H : CH en 5 a) ; 5,85 (d, J = 9,5 Hz, 1H : CH en 6 a) ; 5,89 (mt, 1H : CH en 1 b) ; 6,49 (d, J = 10 Hz, 1H : CONH en 2) ; 6,61 (d, J = 8 Hz, 2H : H aromatiques en 4 e) ; 7,04 (d, J = 8 Hz, 2H : H aromatiques en 4 d ) ; de 7,10 à 7,35 (mt : les 5H aromatiques en 6) ; 7,44 (AB limite, 2H : l'H₄ et l'H₅) ; 7,83 (dd, J = 4 et 1,5 Hz, 1H : 1' H₆) ; 8,40 (d, J = 10 Hz, 1H : CONH en 1) ; 8,75 (d, J = 9,5 Hz, 1H : CONH en 6).

### Exemple 3

Les fractions, respectivement 10 à 14 et 7 à 13 de la chromatographie de l'exemple 2, sont traitées dans les mêmes conditions qu'à l'exemple 2 pour donner 0,37 g (1,8 %) de (5δR,5γS)-5γ-désoxy-5γ-fluoro-5δ-(1-morpholino)méthyl pristinamycine I_{A}, sous forme d'un solide blanc fondant à 162°-164°C.

Spectre de R.M.N. ¹H (400 MHz, CDCl₃) : 0,29 (mt, 1H : 1H du CH₂ en 5β) ; 0,90 (t, J = 7,5 Hz, 3H : CH₃ en 2γ) ; 1,10 (mt, 1H : 1H du CH₂ en 3β) ; 1,26 (mt, 1H : 1H du CH₂ en 3γ) ; 1,33 (d, J = 7 Hz, 3H : CH₃ en 1γ) ; 1,55 (mt, 1H : l'autre H du CH₂ en 3γ) ; 1,65 (mt, 1H : 1H du CH₂ en 2β) ; 1,75 (mt, 2H : CH en 5δ et l'autre H du CH₂ en 2β) ; 1,98 (mt, 1H : l'autre H du CH₂ en 3β) ; 2,05 (t, J = 13,5 Hz, 1H : 1H du CH₂ en 5ε) ; 2,22 (t large, J = 11,5 Hz, 1H : 1H du CH₂N) ; de 2,30 à 2,45 (mt, 3H : l'autre H du CH₂ en 5β et 2H des 2 CH₂N de la morpholine) ; de 2,55 à 2,65 (mt, 3H : l'autre H du CH₂N et les deux autres H des 2 CH₂N de la morpholine) ; de 2,90 à 3,00 (mt, 1H : 1H du CH₂ en 4β) ; 2,95 (s, 6H : ArN(CH₃)₂) ; de 3,15 à 3,30 (mt, 2H : 1H du CH₂ en 3δ et l'autre H du CH₂ en 4β) ; 3,18 (s, 3H : NCH₃) ; 3,49 (mt, 1H : l'autre H du CH₂ en 3δ) ; 3,77 (mt, 4H : les 2 CH₂O de la morpholine) ; 4,53 (t, J = 7,5 Hz, 1H : CH en 3α) ; de 4,65 à 4,90 (mt, 2H : CH en 5γ et l'autre H du CH₂ en 5ε) ; 4,79 (mt, 1H : CH en 2α) ; 4,88 (d large, J = 10 Hz, 1H : CH en 1α) ; 5,09 (mt, 1H : CH en 5α) ; 5,27 (dd, J = 10 et 6 Hz, 1H : CH en 4α) ; 5,65 (d, J = 8 Hz, 1H : CH en 6α) ; 5,89 (q large, 1H : CH en 1β) ; 6,55 (d, J = 10 Hz, 1H : CONH en 2) ; 6,64 (d, J = 8 Hz, 2H : H aromatiques en 4ε) ; 6,97 (d, J = 8 Hz, 2H : H aromatiques en 4δ) ; de 7,15 à 7,45 (mt : les 5H correspondant aux H aromatiques en 6α) ; 7,36 (AB limite, 2H : 1' H₄ et 1' H₅) ; 7,84 (dd, J = 4 et 2 Hz, 1H : 1' H₆) ; 8,42 (d, J = 10 Hz, 1H : CONH en 1) ; 8,70 (d, J = 8 Hz, 1H : CONH en 6) ; 11,66 (s, 1H : OH).

### Exemple 4

On opère comme à l'exemple 1, mais à partir de 100 cm³ d'acétonitrile, de 5 g d'un mélange brut contenant 50 % molaire de 5δ-chlorométhyl-5δ,5γ-déhydro pristinamycine I_{E}, de 1,2 cm³ de di n-propylamine. Le mélange réactionnel est chauffé à reflux durant 2 heures puis concentré sous pression réduite pour donner 5,2 g d'un solide qui est purifié par chromatographie sur silice (éluant : dichlorométhane/méthanol 98/2 en volumes). On obtient ainsi 1,35 g d'un solide qui est purifié, en deux fois (0,5 g et 0,75 g), par 2 chromatographies CLHP préparatives successives sur 450 g de silice C₈ 10µm (éluant: eau-acétonitrile 60/40 en volumes et contenant 0,1 % d'acide trifluoroacétique). Pour chaque lot, les fractions contenant le produit attendu sont rassemblées et l'acétonitrile éliminé à 40°C sous pression réduite (2,7 kPa). La phase aqueuse résiduelle est amenée à pH 7-8 par addition d'eau saturée en bicarbonate de sodium puis extraite par 400 cm³ de dichlorométhane. La phase organique est décantée, séchée sur sulfate de sodium, filtrée, concentrée à sec sous pression réduite. Les 2 solides ainsi obtenus sont cristallisés chacun dans 100 cm³ de cyclohexane pour donner respectivement 0,3 g et 0,28 g d'un solide. Les 2 lots sont réunis, dissous dans 10 cm³ de dichlorométhane et 3 cm³ d'éthanol, concentrés à sec puis agité dans 20 cm³ d'éther diisopropylique. Le précipité est filtré et séché à 40°C sous pression réduite (90 Pa) pour donner 0,35 g de 5δ-dipropylaminométhyl-5δ,5γ-déhydro pristinamycine I_{E}, sous forme de cristaux blancs fondant à 200°-202°C.

Spectre de R.M.N. ¹H (400 MHz, CDCl₃) : de 0,85 à 0,95 (mt, 9H : CH₃ en 2γ et les 2 CH³ de la dipropylamine) ; 1,10 (d très large, J = 16 Hz, 1H : 1H du CH₂ en 5β) ; 1,25 (mt, 2H : 1H du CH₂ en 3β et 1H du CH₂ en 3γ) ; 1,32 (d, J = 7 Hz, 3H : CH₃ en 1γ) ; 1,45 (mt, 4H : les 2 CH₂ centraux de la dipropylamine) ; de 1,50 à 1,65 (mt : 1H correspondant à l'autre H du CH₂ en 3γ) ; 1,66 et 1,74 (2 mts, 1H chacun : CH₂ en 2β) ; 1,99 (mt, 1H : l'autre H du CH₂ en 3β) ; 2,30 (mt, 4H : les 2 NCH₂ de la dipropylamine) ; 2,47 (dd large, J = 16 et 4,5 Hz, 1H : l'autre H du CH₂ en 5β) ; de 2,80 à 3,05 (mt, 3H : 1H du CH₂ en 4β et CH₂N) ; 2,94 (s, 6H : ArN(CH₃)₂) de 3,15 à 3,30 (mt, 2H : 1H du CH₂ en 3δ et l'autre H du CH₂ en 4β) ; 3,17 (s, 3H : NCH₃) ; 3,33 (d large, J = 18 Hz, 1H : 1H du CH₂ en 5ε) ; 3,46 (mt, 1H : l'autre H du CH₂ en 3δ) ; 4,57 (dd, J = 8 et 6 Hz, 1H : CH en 3α) ; 4,78 (mt, 1H : CH en 2α) ; 4,84 (d large, J = 18 Hz, 1H : l'autre H du CH₂ en 5ε) ; 4,89 (d large, J = 10 Hz, 1H : CH en 1α) ; 5,13 (d large, J = 5 Hz, 1H : CH en 5α) ; 5,24 (dd, J = 10 et 8 Hz, 1H : CH en 4α) ; 5,47 (mt, 1H : CH en 5γ) ; 5,56 (d, J = 8 Hz, 1H : CH en 6α) ; 5,88 (q large, J = 7 Hz, 1H : CH en 1β) ; 6,57 (d, J = 10 Hz, 1H : CONH en 2) ; 6,60(d, J = 8 Hz, 2H : H aromatiques en 4ε) ; 6,92 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,70 (d large, J = 4 Hz, 1H : 1' H₆) ; 8,39 (d, J = 10 Hz, 1H : CONH en 1) ; 8,42 (d, J = 8 Hz, 1H : CONH en 6) ; 11,66 (s, 1H : OH).

### Exemple 5

On opère comme à l'exemple 1, mais à partir de 70 cm³ d'acétonitrile, de 5 g d'un mélange brut contenant 50% molaire de 5δ-chlorométhyl-5δ,5γ-déhydro pristinamycine I_{E} et de 0,7 cm³ de pipéridine. Le mélange réactionnel est chauffé à reflux durant 45 minutes puis concentré sous pression réduite pour donner 5,7 g d'un solide qui est purifié par chromatographie sur silice (éluant : gradient dichlorométhane/méthanol 99/1 à 95/5 en volumes). Les fractions contenant le produit attendu sont réunies puis concentrées à sec. Le solide est agité dans 100 cm³ de cyclohexane, filtré et séché sous pression réduite pour donner 0,58 g de solide qui est cristallisé dans 50 cm³ de cyclohexane puis dans 40 cm³ du même solvant. Le solide ainsi obtenu est filtré et séché à 40°C sous pression réduite (90 Pa) pour donner 0,37 g de 5δ-pipéridinométhyl-5δ,5γ-déhydro pristinamycine I_{E}, sous forme d'un solide cotonneux blanc fondant à 200-202°C.

Spectre de R.M.N. ¹H (400 MHz, CDCl₃) : 0,92 (t, J = 7,5 Hz, 3H : CH₃ en 2γ) ; 1,08 (d très large, J = 16,5 Hz, 1H : 1H du CH₂ en 5β) ; de 1,15 à 1,35 (mt, 2H : 1H du CH₂ en 3β et 1H du CH₂ en 3γ) ; 1,31 (d, J = 7 Hz, 3H : CH₃ en 1γ) ; de 1,35 à 1,75 (mt les 8H correspondant à l'autre H du CH₂ en 3γ - aux CH₂CH₂CH₂ de la pipéridine et à 1H du CH₂ en 2β); 1,75 (mt, 1H : l'autre H du CH₂ en 2β); 2,00 (mt, 1H : l'autre H du CH₂ en 3β); 2,29 (mf, 4H : NCH₂ de la pipéridine); 2,48 (dd large, J = 16,5 et 5 Hz, 1H : l'autre H du CH₂ en 5β) ; 2,81 (s, 2H : CH₂N) ; 2,94 (s, 6H : ArN(CH₃)₂) ; 2,98 (dd, J = 14 et 6,5 Hz, 1H : 1H du CH₂ en 4β) ; de 3,10 à 3,30 (mt, 2H : l'autre H du CH₂ en 4β et 1H du CH₂ en 3δ) ; 3,18 (s, 3H : NCH₃) ; 3,3.6 (d large, J = 18 Hz, 1H : 1H du CH₂ en 5ε) ; 3,46 (mt, 1H : l'autre H du CH₂ en 3δ) ; 4,59 (dd, J = 8 et 5,5 Hz, 1H : CH en 3α) ; 4,78 (mt, 1H : CH en 2α) ; 4,83 (d large, J = 18 Hz, 1H : l'autre H du CH₂ en 5ε) ; 4,88 (d large, J = 10 Hz, 1H : CH en 1α) ; 5,12 (d, J = 5 Hz, 1H : CH en 5α) ; 5,23 (dd, J = 9 et 6,5 Hz, 1H : CH en 4α) ; 5,47 (mt, 1H : CH en 5γ) ; 5,53 (d, J = 8 Hz, 1H : CH en 6α) ; 5,89 (q large, J = 7 Hz, 1H : CH en 1β) ; 6,58 (d, J = 9,5 Hz, 1H : CONH en 2) ; 6,60 (d, J = 8 Hz, 2H : H aromatiques en 4ε) ; 6,93 (d, J= 8 Hz, 2H: H aromatiques en 4δ) ; de 7,20 à 7,40 (mt : les 7H correspondant aux H aromatiques en 6α - au 1' H₄ et au 1' H₅) ; 7,72 (d large, J = 4 Hz, 1H : 1' H₆) ; de 8,35 à 8,45 (mt, 2H : CONH en 1 et CONH en 6) ; 11,68 (s, 1H : OH).

### Exemple 6

On opère comme à l'exemple 1, mais à partir de 50 cm³ d'acétonitrile, de 5,3 g d'un mélange brut contenant 25% molaire de 5δ-chlorométhyl-5δ,5γ-déhydro pristinamycine I_{E} préparé dans des conditions identiques à celles décrites à l'exemple 1, et de 0,4 cm³ de pyrrolidine. Le mélange réactionnel est chauffé à reflux durant 45 minutes puis est concentré sous pression réduite à 45°C (2,7 kPa). Le solide obtenu est agité dans 100 cm³ de cyclohexane et 100 cm³ d'éther diéthylique. Le précipité apparu est filtré, lavé par 25 cm³ d'éther diéthylique puis chromatographié par CLHP préparative sur 450 g de silice C₈ 10µm (éluant: eau-acétonitrile 65/35 en volumes contenant 0,1 % d'acide trifluoroacétique). Les fractions contenant l'attendu sont rassemblées et l'acétonitrile éliminé à 40°C sous pression réduite (2,7 kPa). La solution aqueuse résiduelle est ajustée à pH 7-8 par addition d'eau saturée en bicarbonate de sodium puis extraite par 100 cm³ de dichlorométhane. La phase organique est séchée sur sulfate de sodium, filtrée puis concentrée à sec sous pression réduite pour donner un solide qui est agité dans 20 cm³ d'éther diisopropylique, filtré puis séché sous pression réduite à 45°C (90 Pa). On obtient ainsi 0,49 g de 5δ-(1-pyrrolidinylméthyl)-5δ,5γ-déhydro pristinamycine I_{E}, sous forme de cristaux blancs fondant à 164°-166°C.

Spectre de R.M.N. ¹H (400 MHz, CDCl₃) : 0,92 (t, J = 7,5 Hz, 3H : CH₃ en 2γ) ; 1,11 (d très large, J = 17 Hz, 1H : 1H du CH₂ en 5β) ; 1,24 (mt, 2H : 1H du CH₂ en 3β et 1H du CH₂ en 3γ) ; 1,31 (d, J = 7 Hz, 3H : CH₃ en 1γ) ; 1,54 (mt, 1H : l'autre H du CH₂ en 3γ) ; de 1,60 à 1,85 (mt : les 6H correspondant au CH₂ en 2β et au CH₂ de la pyrrolidine) ; 1,99 (mt, 1H : l'autre H du CH₂ en 3β) ; 2,42 (mt, 4H : NCH₂ de la pyrrolidine) ; 2,48 (dd large, J = 17 et 5,5 Hz, 1H : l'autre H du CH₂ en 5β) ; de 2,90 à 3,05 (mt, 1H : 1H du CH₂ en 4β) ; 2,93 (s, 6H : ArN(CH₃)₂) ; 2,98 (s, 2H : CH₂N) ; de 3,15 à 3,30 (mt, 2H : l'autre H du CH₂ en 4β et 1H du CH₂ en 3δ) ; 3,16 (s, 3H : NCH₃) ; 3,38 (d large, J = 18 Hz, 1H : 1H du CH₂ en 5ε) ; 3,45 (mt, 1H : l'autre H du CH₂ en 3δ) ; 4,58 (dd, J = 8,5 et 5,5 Hz, 1H : CH en 3α) ; 4,77 (mt, 1H : CH en 2α) ; de 4,80 à 4,95 (mt, 2H : l'autre H du CH₂ en 5ε et CH en 1α) ; 5,10 (d, J = 5,5 Hz, 1H : CH en 5α) ; 5,24 (dd, J = 10 et 7 Hz, 1H : CH en 4α) ; 5,50 (mt, 1H : CH en 5γ) ; 5,54 (d, J = 8 Hz, 1H : CH en 6α) ; 5,86 (q dédoublé, J = 7 et 1 Hz, 1H : CH en 1β) ; 6,57 (d, J = 9,5 Hz, 1H : CONH en 2) ; 6,59 (d, J = 8 Hz, 2H : H aromatiques en 4ε) ; 6,91 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,71 (d large, J = 4 Hz, 1H : 1' H₆) ; 8,39 (d, J = 10 Hz, 1H : CONH en 1) ; 8,43 (d, J = 8 Hz, 1H : CONH en 6) ; 11,66 (s large, 1H :OH).

### Exemple 7

On opère comme à l'exemple 1, mais à partir de 100 cm³ d'acétonitrile, de 10 g d'un mélange brut contenant 50% molaire de 5δ-chlorométhyl-5δ,5γ-déhydro pristinamycine I_{E} et de 3,7 cm³ de 2,6-diméthylmorpholine (mélange d'isomères cis et trans). Le mélange réactionnel est chauffé à reflux I heure puis concentré sous pression réduite à 45°C (2,7 kPa) pour donner 13,4 g d'un solide qui est repris par 100 cm³ d'eau saturée en bicarbonate de sodium. Le mélange résultant est extrait par 2 fois 100 cm³ de dichlorométhane. Les phases organiques sont rassemblées, séchées sur sulfate de sodium, filtrées puis concentrées à sec pour donner 12,1 g de solide jaune qui est purifié par 2 chromatographies successives sur silice (éluant : dichlorométhane/méthanol 98/2 en volumes) pour donner un solide qui est agité dans 30 cm³ d'éther diéthylique, filtré puis séché sous pression réduite à 40°C (90 Pa). On obtient ainsi 0,5 g de 5δ-(2,6-diméthyl-morpholinométhyl)-5δ,5γ-déhydro pristinamycine I_{E} (mélange d'isomères), sous forme d'un solide crème fondant vers 165°C.

Spectre de R.M.N. ¹H (400 MHz, CDCl₃). Nous observons un mélange des deux diastéréoisomères cis et trans sur la morpholine : 0,92 (t, J = 7,5 Hz, 3H : CH₃ en 2γ) ; 1,02 (d très large, J = 17 Hz, 1H : 1H du CH₂ en 5β) ; 1,15 et 1,20 (2 d, J = 7 Hz, 3H chacun : CH₃ de la 2,6 diméthyl morpholine) ; de 1,20 à 1,45 (mt, 2H : 1H du CH₂ en 3β et 1H du CH₂ en 3γ) ; 1,30 (d, J = 7 Hz, 3H : CH₃ en 1γ) ; 1,55 (mt, 1H : l'autre H du CH₂ en 3γ) ; de 1,60 à 1,75 (mt : les 3H correspondant à 1H du CH₂ en 2β et à 2H des NCH₂ de la 2,6 diméthyl morpholine) ; 1,74 (mt, 1H : l'autre H du CH₂ en 2β) ; 2,00 (mt, 1H : l'autre H du CH₂ en 3β) ; 2,45 (dd large, J = 17 et 5,5 Hz, 1H : l'autre H du CH₂ en 5β) ; 2,68 (mt, 2H : les 2 autres H des NCH₂ de la 2,6 diméthyl morpholine) ; 2,77 et 2,86 (2 d, J = 13 Hz, 1H chacun : CH₂N) ; de 2,90 à 3,00 (mt, 1H : 1H du CH₂ en 4β) ; 2,95 (s, 6H : ArN(CH₃)₂) ; de 3,15 à 3,30 (mt, 2H : l'autre H du CH₂ en 4β et 1H du CH₂ en 3δ) ; 3,17 (s, 3H : NCH₃) ; 3,32 (d large, J = 18 Hz, 1H : 1H du CH₂ en 5ε) ; 3,47 (mt, 1H : l'autre H du CH₂ en 3δ) ; 3,60 et 3,70 (2 mts, 1H chacun : CHO de la morpholine) ; 4,58 (dd, J = 8,5 et 5,5 Hz, 1H : CH en 3α) ; 4,77 (mt, 1H : CH en 2α) ; 4,84 (d large, J = 18 Hz, 1H : l'autre H du CH₂ en 5ε) ; 4,88 (dd, J = 10 et 1Hz, 1H : CH en 1α) ; 5,10 (d, J = 5,5 Hz, 1H : CH en 5α) ; 5,21 (dd, J = 9 et 6,5 Hz, 1H : CH en 4α) ; 5,48 (mt, 1H : CH en 5γ) ; 5,52 (d, J = 8 Hz, 1H : CH en 6α) ; 5,87 (q dédoublé, J = 7 et 1 Hz, 1H : CH en 1β) ; 6,56 (d, J = 9,5 Hz, 1H : CONH en 2) ; 6,58 (d, J = 8 Hz, 2H : H aromatiques en 4ε) ; 6,90 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,72 (dd, J = 4 et 1 Hz, 1H : 1' H₆) ; 8,39 (d, J = 10 Hz, 1H : CONH en 1) ; 8,48 (d, J = 8 Hz, 1H : CONH en 6) ; 11,66 (s, 1H :OH).

### Exemple 8

On opère comme à l'exemple 1, mais à partir de 100 cm³ d'acétonitrile, de 10 g d'un mélange brut contenant 40 % molaire de 5δ-chlorométhyl-5δ,5γ-déhydro pristinamycine I_{E} et de 3,8 g de chlorhydrate de 4-(4-fluorophényl)-1,2,3,6-tétrahydropyridine et de 2,75 cm³ de triéthylamine. Le mélange réactionnel est chauffé à reflux 1 heure puis concentré sous pression réduite (45°C ; 2,7 kPa) pour donner 14,3 g de solide marron qui est purifié par 2 chromatographies préparatives CLHP sur 450 g de silice C₈ 10µm (éluant: eau-acétonitrile 65/35 en volumes contenant 0,1 % d'acide trifluoroacétique). Les fractions contenant le produit attendu sont rassemblées et l'acétonitrile éliminé à 40°C, sous pression réduite (2,7 kPa). Le pH de la phase aqueuse résiduelle est ajusté à 7-8 par addition d'eau saturée en bicarbonate de sodium. Le précipité obtenu est filtré, rincé par 50 cm³ d'éther diisopropylique puis séché à 40°C (90 Pa) pour donner 0,63 g de 5δ-[4-(4-fluorophényl)-1,2,3,6-tétrahydropyridylméthyl]-5δ,5γ-déhydro pristinamycine I_{E}, sous forme d'une solide jaune fondant à 172°C.

Spectre de R.M.N. ¹H (600 MHz, CDCl₃) : 0,90 (t, J = 7,5 Hz, 3H : CH₃ en 2γ) ; 1,13 (d très large, J = 16 Hz, 1H : 1H du CH₂ en 5β) ; 1,21 (mt, 2H : 1H du CH₂ en 3β et 1H du CH₂ en 3γ) ; 1,30 (d, J = 7 Hz, 3H : CH₃ en 1γ) ; 1,53 (mt, 1H : l'autre H du CH₂ en 3γ), 1,65 et 1,73 (2 mts : les 2H correspondant au CH₂ en 2β) ; 1,98 (mt, 1H : l'autre H du CH₂ en 3β) ; de 2,40 à 2,65 (mt, 5H : NCH₂CH₂ de la 1,2,3,6-tétrahydropyridine et l'autre H du CH₂ en 5β) ; de 2,90 à 3,05 (mt, 3H : CH₂N et 1 H du CH₂ en 4β) ; 2,93 (s, 6H : ArN(CH₃)₂) ; 3,06 (mt, 2H : NCH₂ de la 1,2,3,6-tétrahydropyridine) ; de 3,10 à 3,30 (mt, 2H : l'autre H du CH₂ en 4β et 1H du CH₂ en 3δ) ; 3,15 (s, 3H : NCH₃) ; 3,40 (d large, J = 18 Hz, 1H : 1H du CH₂ en 5ε) ; 3,45 (mt, 1H : l'autre H du CH₂ en 3δ) ; 4,56 (dd, J = 8 et 6 Hz, 1H : CH en 3α) ; 4,76 (mt, 1H : CH en 2α) ; 4,83 (d, J = 18 Hz, 1H : l'autre H du CH₂ en 5ε) ; 4,86 (d large, J = 10 Hz, 1H : CH en 1α) ; 5,12 (d, J = 5,5 Hz, 1H : CH en 5α) ; 5,22 (dd, J = 9 et 7 Hz, 1H : CH en 4α) ; 5,54 (d, J = 8 Hz, 1H : CH en 6α) ; 5,56 (mt, 1H : CH en 5γ) ; 5,87 (q large, J = 7 Hz, 1H : CH en 1β) ; 6,00 (mt, 1H : CH= de la 1,2,3,6-tétrahydropyridine ) ; 6,56 (d, J = 9,5 Hz, 1H : CONH en 2) ; 6,59 (d, J = 8 Hz, 2H : H aromatiques en 4ε) ; 6,92 (d, J = 8 Hz, 2H : H aromatiques en 4δ) ; 6,97 (t, J = 8,5 Hz, 2H : H aromatiques en ortho du F) ; de 7,20 à 7,30 (mt : les 5H correspondant aux H aromatiques en 6α) ; de 7,30 à 7,40 (mt, 4H : H aromatiques en méta du F - 1' H₄ et 1' H₅) ; 7,71 (d, J = 4 Hz, 1H : 1' H₆) ; 8,48 (d, J= 10 Hz, 1H : CONH en 1) ;8,45 (d, J = 8 Hz, 1H : CONH en 6) ; 11,65 (s, 1H : OH).

### Exemple 9

On opère comme à l'exemple 1, mais à partir de 50 cm³ d'acétonitrile, de 5 g d'un mélange brut contenant 50 % molaire de 5δ-chlorométhyl-5δ,5γ-déhydro pristinamycine I_{E} et de 0,6 g de thiomorpholine. Le mélange réactionnel est chauffé 2 heures puis concentré sous pression réduite à 45°C (2,7 kPa). Le solide est repris par 100 cm³ d'eau. L'insoluble est filtré, lavé par 20 cm³ d'eau pour donner 5,4 g de solide marron qui est purifié par chromatographie sur silice (éluant : gradient dichlorométhane/méthanol 99/1 à 98/2 en volumes) puis par CLHP préparative sur 450 g de silice C₈ 10µm (éluant: eau-acétonitrile 65/35 en volumes contenant 0,1 % d'acide trifluoroacétique). Les fractions contenant l'attendu sont rassemblées, l'acétonitrile éliminé à 40°C sous pression réduite (2,7 kPa) et le pH de la phase aqueuse résiduelle ajusté à 7-8 par addition d'eau saturée en bicarbonate de sodium. Le précipité obtenu est filtré, lavé par 20 cm³ d'eau et séché à 45°C (90 Pa), pour donner 0,31 g de 5δ-thiomorpholinométhyl-5δ,5γ-déhydro pristinamycine I_{E}, sous forme d'un solide blanc fondant à 160°C.

Spectre de R.M.N. ¹H (400 MHz, CDCl₃): 0,91 (t, J = 7,5 Hz, 3H : CH₃ en 2γ) ; 1,04 (d très large, J = 16 Hz, 1H : 1H du CH₂ en 5β) ; de 1,15 à 1,35 (mt, 2H : 1H du CH₂ en 3β et 1H du CH₂ en 3γ) ; 1,31 (d, J = 7 Hz, 3H : CH₃ en 1γ) ; 1,55 (mt, 1H : l'autre H du CH₂ en 3γ) ; de 1,60 à 1,85 (mt : les 2H correspondant au CH₂ en 2β) ; 2,00 (mt, 1H : l'autre H du CH₂ en 3β) ; 2,46 (d très large, J = 16 Hz, 1H : l'autre H du CH₂ en 5β) ; 2,65 (mf, 8H : NCH₂CH₂S de la thiomorpholine) ; 2,90 (s large, 2H : CH₂N) ; 2,95 (s, 6H : ArN(CH₃)₂) ; de 2,95 à 3,05 (mt, 1H : 1H du CH₂ en 4β) ; de 3,10 à 3,25 (mt, 2H : l'autre H du CH₂ en 4β et 1H du CH₂ en 3δ) ; 3,19 (s, 3H : NCH₃) ; 3,29 (d large, J = 18 Hz, 1H : 1H du CH₂ en 5ε) ; 3,47 (mt, 1H : l'autre H du CH₂ en 3δ) ; 4,59 (dd, J = 8 et 6,5 Hz, 1H : CH en 3α) ; de 4,75 à 4,85 (mt, 1H : CH en 2α) ; 4,82 (d large, J = 18 Hz, 1H : l'autre H du CH₂ en 5ε) ; 4,89 (d large, J = 10 Hz, 1H : CH en 1α) ; 5,11 (d large, J = 5 Hz, 1H : CH en 5α) ; 5,24 (mt, 1H : CH en 4α) ; 5,50 (mt, 1H : CH en 5γ) ; 5,53 (d, J = 8,5 Hz, 1H : CH en 6α) ; 5,89 (q large, J = 7 Hz, 1H : CH en 1β) ; de 6,55 à 6,65 (mt, 1H : CONH en 2) ; 6,59 (d, J = 8 Hz, 2H : H aromatiques en 4ε) ; 6,92 (d, J = 8 Hz, 2H : H aromatiques en 4δ) ; de 7,20 à 7,45 (mt : les 7H correspondant aux H aromatiques en 6α - au 1' H₄ et au 1' H₅) ; 7,74 (mt, 1H : 1' H₆) ; 8,41 (d, J = 10 Hz, 1H : CONH en 1) ; 8,51 (d, J = 8,5 Hz, 1H : CONH en 6) ; 11,68 (s, 1H : OH).

### Exemple 10

En opérant comme à l'exemple 1, mais à partir de 200 cm³ d'acétonitrile, de 6 g d'un mélange brut contenant 50% molaire de 5δ-chlorométhyl 5δ,5γ-déhydro pristinamycine I_{E} et de 4,8 g de 4-acétyl-4-phényl pipéridine. Le mélange réactionnel est chauffé 3 heures puis concentré sous pression réduite à 45°C (2,7 kPa). Le solide est repris par 100 cm³ d'eau et 100 cm³ de dichlorométhane. La phase organique est décantée puis séchée sur sulfate de sodium, filtrée et concentrée à sec pour donner 10,6 g d'un solide marron qui est purifié par 2 chromatographies successives sur silice (éluant : dichlorométhane/méthanol 97/3 en volumes). On obtient ainsi un solide qui est agité dans 30 cm³ d'éther diéthylique, filtré puis séché à 45°C (90 Pa) pour donner 0,46 g de 5δ-(4-acétyl-4-phényl pipéridinométhyl)-5δ,5γ-déhydro pristinamycine I_{E}, sous forme d'un solide jaune fondant à 171°C.

Spectre de R.M.N. ¹H (400 MHz, CDCl₃) : 0,93 (t, J = 7,5 Hz, 3H : CH, en 2γ) ; 1,06 (d très large, J = 16 Hz, 1H : 1H du CH₂ en 5β) ; de 1,15 à 1,35 (mt, 2H : 1H du CH₂ en 3β et 1H du CH₂ en 3γ) ; 1,31 (d, J = 7 Hz, 3H : CH₃ en 1γ) ; 1,53 (mt, 1H : l'autre H du CH₂ en 3γ) ; de 1,60 à 1,85 (mt : les 2H correspondant au CH₂ en 2β) ; de 1,90 à 2,25 - 2,45 et 2,62 (3 séries de mt, 8H en totalité : NCH₂CH₂ de la pipéridine) ; 1,91 (s, 3H : COCH₃) ; 2,00 (mt, 1H : l'autre H du CH₂ en 3β) ; de 2,40 à 2,45 (mt, 1H : l'autre H du CH₂ en 5β) ; 2,82 (s large, 2H : CH₂N) ; de 2,85 à 3,05 (mt, 1H : 1H du CH₂ en 4β) ; 2,94 (s, 6H : ArN(CH₃)₂) ; de 3,10 à 3,30 (mt, 2H : l'autre H du CH₂ en 4β et 1H du CH₂ en 3δ) ; 3,16 (s, 3H : NCH₃) ; 3,31 (d large, J = 18 Hz, 1H : 1H du CH₂ en 5ε) ; 3,48 (mt, 1H : l'autre H du CH₂ en 3δ) ; 4,58 (dd, J = 8 et 5,5 Hz, 1H : CH en 3α) ; de 4,70 à 4,85 (mt, 2H : CH en 2α et l'autre H du CH₂ en 5ε) ; 4,89 (d large, J = 10 Hz, 1H : CH en 1α) ; 5,10 (d large, J = 5,5 Hz, 1H : CH en 5α) ; 5,23 (dd, J = 9 et 7 Hz, 1H : CH en 4α) ; 5,47 (mt, 1H : CH en 5γ) ; 5,56 (d, J = 8 Hz, 1H : CH en 6α) ; 5,89 (q large, J = 7 Hz, 1H : CH en 1β) ; de 6,55 à 6,65 (mt, 1H : CONH en 2) ; 6,60 (d, J = 8 Hz, 2H : H aromatiques en 4ε) ; 6,91 (d, J = 8 Hz, 2H : H aromatiques en 4δ) ; de 7,15 à 7,40 (mt : les 12H correspondant aux H aromatiques en 6α - aux H aromatiques du phényle - au 1' H₄ et au 1' H₅) ; 7,74 (d large, J = 4 Hz, 1H : 1' H₆) ; 8,42 (d, J = 10 Hz, 1H : CONH en 1) ; 8,49 (d, J = 8 Hz, 1H : CONH en 6) ; 11,67 (s, 1H : OH).

### Exemple 11

On opère comme à l'exemple 1, mais à partir de 100 cm³ d'acétonitrile, de 6 g d'un mélange brut contenant 40 % molaire de 5δ-chlorométhyl-5δ,5γ-déhydro pristinamycine I_{E} et de 2,4 cm³ de N-méthylbutylamine. Le mélange réactionnel est chauffé 3 heures puis concentré sous pression réduite à 45°C (2,7 kPa). Le solide est repris par 100 cm³ d'eau et le mélange résultant est extrait par 2 fois 70 cm³ de dichlorométhane. La phase organique est décantée puis séchée sur sulfate de sodium, filtrée et concentrée à sec pour donner 6,5 g de brut qui est purifié par 2 chromatographies successives sur silice (éluant : dichlorométhane/méthanol 97/3 en volumes). On obtient ainsi un solide qui est agité dans 30 cm³ d'éther diéthylique, filtré puis séché à 40°C (90 Pa) pour donner 0,50 g de 5δ N-méthyl-N-butylaminométhyl-5δ,5γ-déhydro pristinamycine I_{E}, sous forme d'un solide jaune fondant à 168°C.

Spectre de R.M.N. ¹H (500 MHz, CDCl₃) : de 0,85 à 0,95 (mt, 6H : CH₃ en 2γ et CH, du butyle) ; 1,10 (d très large, J = 16 Hz, 1H : 1H du CH₂ en 5β) ; de 1,15 à 1,30 (mt, 2H : 1H du CH₂ en 3β et 1H du CH₂ en 3γ) ; 1,30 (d, J = 7 Hz, 3H : CH₃ en 1γ) ; 1,34 et 1,44 (2 mts, 2H chacun : CH₂CH₂ centraux du butyle) ; 1,54 (mt, 1H : l'autre H du CH₂ en 3γ) ; 1,66 (mt, 1H : 1H du CH₂ en 2β) ; 1,74 (mt : 1H correspondant à l'autre H du CH₂ en 2β) ; 1,99 (mt, 1H : l'autre H du CH₂ en 3β) ; 2,11 (s, 3H : NCH₃) ; 2,27 (mt, 2H : NCH₂ du butyle) ; 2,47 (dd large, J = 16 et 5 Hz, 1H : l'autre H du CH₂ en 5β) ; 2,83 (AB, J = 13 Hz, 2H : CH₂N) ; 2,93 (s, 6H : ArN(CH₃)₂) ; 2,98 (dd, J = 14 et 6,5 Hz, 1H : 1H du CH₂ en 4β) ; de 3,15 à 3,30 (mt, 2H : 1H du CH₂ en 3δ et l'autre H du CH₂ en 4β) ; 3,17 (s, 3H : NCH₃) ; 3,34 (d large, J = 18 Hz, 1H : 1H du CH₂ en 5ε) ; 3,46 (mt, 1H : l'autre H du CH₂ en 3δ) ; 4,57 (dd, J = 8 et 5,5 Hz, 1H : CH en 3α) ; de 4,75 à 4,85 (mt, 1H : CH en 2α) ; 4,80 (d large, J = 18 Hz, 1H : l'autre H du CH₂ en 5ε) ; 4,87 (dd, J = 10 et 1 Hz, 1H : CH en 1α) ; 5,11 (d large, J = 5 Hz, 1H : CH en 5α) ; 5,24 (dd, J = 9 et 6,5 Hz, 1H : CH en 4α) ; 5,48 (mt, 1H : CH en 5γ) ; 5,56 (d, J = 8 Hz, 1H : CH en 6α) ; 5,87 (q dédoublé, J = 7 et 1 Hz, 1H : CH en 1β) ; 6,60 (d, J = 8 Hz, 2H : H aromatiques en 4ε) ; 6,52 (d, J = 10 Hz, 1H : CONH en 2) ; 6,92 (d, J= 8 Hz, 2H : H aromatiques en 4δ) ; de 7,20 à 7,35 (mt : les 7H correspondant aux H aromatiques en 6α - au 1' H₄ et au 1' H₅) ; 7,72 (d large, J = 4 Hz, 1H : 1' H₆) ; 8,39 (d, J = 10 Hz, 1H : CONH en 1) ; 8,43 (d, J = 8 Hz, 1H : CONH en 6) ; 11,68 (s large, 1H : OH).

### Exemple 12

On opère comme à l'exemple 1, mais à partir de 100 cm³ d'acétonitrile, de 6 g d'un mélange brut contenant 40 % molaire de 5δ-chlorométhyl-5δ,5γ-déhydro pristinamycine I_{E} et de 2 cm³ de (S)-prolinol. Le mélange réactionnel est chauffé 3 heures puis concentré sous pression réduite à 45°C (2,7 kPa). Le solide est repris par 200 cm³ d'eau et 100 cm³ de dichlorométhane. La phase organique est décantée puis séchée sur sulfate de sodium, filtrée et concentrée à sec pour donner 6,2 g de brut qui est chromatographié sur silice (éluant : dichlorométhane/méthanol 95/5 en volumes). On obtient un solide qui est agité dans 30 cm³ d'éther diéthylique et 30 cm³ d'éther de pétrole, filtré puis séché à 40°C (90 Pa) pour donner 0,67 g de 5δ-[(S)-2-hydroxyméthylpyrrolidino]méthyl-5δ,5γ-déhydro pristinamycine I_{E}, sous forme d'un solide jaune fondant à 147°C.

Spectre de R.M.N. ¹H (400 MHz, CDCl₃) : 0,92 (t, J = 7,5 Hz, 3H : CH₃ en 2γ) ; 1,01 (d très large, J = 17 Hz, 1H : 1H du CH₂ en 5β) ; de 1,15 à 1,30 (mt, 2H : 1H du CH₂ en 3β et 1H du CH₂ en 3γ) ; 1,31 (d, J = 7 Hz, 3H : CH₃ en 1γ) ; 1,57 (mt, 1H : l'autre H du CH₂ en 3γ) ; de 1,60 à 2,00 (mt : les 6H correspondant au CH₂ en 2β et aux CH₂ de la pyrrolidine) ; 2,02 (mt, 1H : l'autre H du CH₂ en 3β) ; 2,30 (mt, 1H : 1H du NCH₂ de la pyrrolidine) ; 2,44 (dd large, J = 17 et 5 Hz, 1H : l'autre H du CH₂ en 5β) ; 2,62 (mt, 1H : NCH de la pyrrolidine) ; 2,77 (d large, J = 12 Hz, 1H : 1H du CH₂N) ; de 2,85 à 3,05 (mt, 1H : 1H du CH₂ en 4β) ; 2,94 (s, 6H : ArN(CH₃)₂) ; de 3,05 à 3,35 (mt, 5H : l'autre H du NCH₂ de la pyrrolidine - l'autre H du CH₂ en 4β - l'autre H du CH₂N - 1H du CH₂ en 3δ et 1H du CH₂ en 5ε) ; 3,19 (s, 3H : NCH₃) ; 3,40 (mt, 1H : 1H du CH₂O) ; 3,46 (mt, 1H : l'autre H du CH₂ en 3δ) ; 3,64 (dd, J = 11,5 et 3 Hz, 1H : l'autre H du CH₂O) ; 4,56 (dd, J = 8 et 5,5 Hz, 1H : CH en 3α) ; 4,78 (mt, 1H : CH en 2α) ; 4,88 (dd, J = 10 et 1 Hz, 1H : CH en 1α) ; 4,94 (d large, J = 18 Hz, 1H : l'autre H du CH₂ en 5ε) ; 5,12 (d, J = 5,5 Hz, 1H : CH en 5α); 5,24 (dd, J = 9 et 6,5 Hz, 1H : CH en 4α) ; 5,51 (mt, 1H : CH en 5γ) ; 5,59 (d, J = 8 Hz, 1H : CH en 6α) ; 5,87 (q dédoublé, J = 7 et 1 Hz, 1H : CH en 1β) ; de 6,55 à 6,70 (mt, 1H : CONH en 2) ; 6,59 (d, J = 8 Hz, 2H : H aromatiques en 4ε) ; 6,94 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,74 (dd, J = 4 et 1,5 Hz, 1H : 1' H₆) ; 8,41 (d, J = 10 Hz, 1H: CONH en 1) ; 8,49 (d, J = 8 Hz, 1H: CONH en 6); 11,68 (s large, 1H : OH).

### Exemple 13

On opère comme à l'exemple 1, mais à partir de 50 cm³ d'acétonitrile, de 2 g d'un mélange brut contenant 40 % molaire de 5δ-chlorométhyl-5δ,5γ-déhydro pristinamycine I_{E} et de 0,75 cm³ de 2-(N-méthyl-N-aminométhyl)-1,3-dioxolanne. Le mélange réactionnel est chauffé 3 heures puis concentré sous pression réduite à 45°C (2,7 kPa). Le solide est repris par 100 cm³ d'eau et 100 cm³ de dichlorométhane. La phase organique est décantée puis séchée sur sulfate de sodium, filtrée et concentrée à sec pour donner 2,0 g de solide jaune qui est purifié par chromatographie sur silice (éluant : dichlorométhane/méthanol 97/3 en volumes). Après concentration des fractions, le solide obtenu est séché à 40°C (90 Pa), pour donner 0,24 g de 5δ-[N-méthyl N-2-(1,3-dioxolanyl)méthyl]aminométhyl-5δ,5γ-déhydro pristinamycine I_{E}, sous forme d'un solide jaune fondant à 149°C.

Spectre de R.M.N. ¹H (400 MHz, CDCl₃) : 0,91 (t, J = 7,5 Hz, 3H : CH₃ en 2γ) ; 1,01 (d très large, J = 16 Hz, 1H : 1H du CH₂ en 5β) ; de 1,15 à 1,35 (mt, 2H : 1H du CH₂ en 3β et 1H du CH₂ en 3γ) ; 1,30 (d, J = 7 Hz, 3H : CH₃ en 1γ) ; 1,53 (mt, 1H : l'autre H du CH₂ en 3γ) ; de 1,60 à 1,80 (mt : les 2H correspondant au CH₂ en 2β) ; 1,98 (mt, 1H : l'autre H du CH₂ en 3β) ; 2,28 (s, 3H : NCH₃) ; 2,44 (dd large, J = 16 et 5 Hz, 1H : l'autre H du CH₂ en 5β) ; 2,50 et 2,58 (2 dd, J = 13 et 4,5 Hz, 1H chacun : NCH₂) ; de 2,85 à 3,05 (mt, 3H : 1H du CH₂ en 4β et CH₂N en 5δ) ; 2,94 (s, 6H : ArN(CH₃)₂) ; de 3,10 à 3,30 (mt, 2H : l'autre H du CH₂ en 4β et 1H du CH₂ en 3) ; 3,17 (s, 3H : NCH₃) ; 3,36 (d large, J = 18 Hz, 1H : 1H du CH₂ en 5ε) ; 3,44 (mt, 1H : l'autre H du CH₂ en 3δ) ; de 3,75 à 4,00 (mt, 4H : OCH₂CH₂O) ; 4,56 (dd, J = 8 et 5,5 Hz, 1H : CH en 3α) ; 4,76 (mt, 1H : CH en 2α) ; 4,83 (d large, J = 18 Hz, 1H : l'autre H du CH₂ en 5ε) ; 4,88 (dd, J= 10 et 1 Hz, 1H : CH en 1α) ; 4,97 (t, J = 4,5 Hz, 1H : OCHO) ; 5,11 (d, J = 5,5 Hz, 1H : CH en 5α) ; 5,21 (dd, J = 9 et 6 Hz, 1H : CH en 4α) ; 5,48 (mt, 1H : CH en 5γ) ; 5,57 (d, J = 8 Hz, 1H : CH en 6α) ; 5,87 (q dédoublé, J = 7 et 1 Hz, 1H : CH en 1β) ; 6,57 (d, J = 9 Hz, 1H : CONH en 2); 6,62 (d, J= 8 Hz, 2H : H aromatiques en 4ε) ; 6,93 (d, J= 8 Hz, 2H : H aromatiques en 4δ) ; de 7,20 à 7,40 (mt : les 7H correspondant aux H aromatiques en 6α - au l'H₄ et au l'H₅) ; 7,72 (dd, J = 4 et 1 Hz, 1H : l'H₆) ; 8,39 (d, J = 10 Hz, 1H : CONH en 1) ; 8,44 (d, J = 8 Hz, 1H : CONH en 6) ; 11,67 (s large, 1H : OH).

### Exemple 14

On opère comme à l'exemple 1, mais à partir de 100 cm³ d'acétonitrile, de 10 g d'un mélange brut contenant 33 % molaire de 5δ-chlorométhyl-5δ,5γ-déhydro pristinamycine I_{E} et de 3,5 g de 4-pipéridine éthanol. Le mélange réactionnel est chauffé 2 heures puis concentré sous pression réduite à 45°C (2,7 kPa). Le solide obtenu est repris par 50 cm³ d'eau et 50 cm³ de dichlorométhane. La phase organique est décantée, séchée sur sulfate de sodium, filtrée et concentrée à sec pour donner 6,2 g de solide marron qui est purifié sur silice (éluant : dichlorométhane/méthanol 95/5 en volumes) puis par CLHP sur 450 g de silice C₈ 10µm (éluant: eau-acétonitrile 70/30 en volumes contenant 0,1% d'acide trifluoroacétique). Les fractions contenant l'attendu sont rassemblées, l'acétonitrile éliminé à 40°C sous pression réduite (2,7 kPa) et le pH de la phase aqueuse résiduelle ajusté à 7-8 par addition d'eau saturée en bicarbonate de sodium. La phase aqueuse est extraite par 2 fois 50 cm³ de dichlorométhane. Les phases organiques sont rassemblées puis séchées sur sulfate de sodium, filtrées et concentrées à sec. Le solide obtenu est agité dans 25 cm³ d'éther diéthylique, filtré, lavé par 10 cm³ d'éther diisopropylique puis séché à 40°C. (90 Pa) pour donner 0,70 g de 5δ-[4-(2-hydroxyéthyl)pipéridino]méthyl-5δ,5γ-déhydro pristinamycine I_{E}, sous forme d'un solide blanc fondant à 240°C.

Spectre de R.M.N. ¹H (500 MHz, CDCl₃) : 0,93 (t, J = 7,5 Hz, 3H : CH₃ en 2γ) ; 1,10 (d très large, J= 16,5 Hz, 1H : 1H du CH₂ en 5β); de 1,15 à 1,35 (mt, 2H : 1H du CH₂ en 3β et 1H du CH₂ en 3γ) ; de 1,15 à 1,80 (mt, 5H : CH₂ de la pipéridine et CH de la pipéridine) ; 1,32 (d, J = 7 Hz, 3H : CH₃ en 1γ) ; de 1,45 à 1,60 (mt, 1H : l'autre H du CH₂ en 3γ) ; 1,51 (q, J = 7 Hz, 2H : CH₂ de l'hydroxyéthyle) ; de 1,60 à 1,80 (mt, 2H : CH₂ en 2β) ; 1,84 (t large, J= 11Hz, 2H : H axiaux des NCH₂ de la pipéridine) ; 2,00 (mt, 1H : l'autre H du CH₂ en 3β) ; 2,49 (dd large, J = 16,5 et 5,5 Hz, 1H : l'autre H du CH₂ en 5β) ; de 2,75 à 2,90 (mt, 4H : CH₂N et H équatoriaux des NCH₂ de la pipéridine) ; 2,95 (s, 6H : ArN(CH₃)₂) ; 2,99 (dd, J = 14 et 6,5 Hz, 1H : 1H du CH₂ en 4β) ; de 3,15 à 3,35 (mt, 2H : l'autre H du CH₂ en 4β et 1H du CH₂ en 3δ) ; 3,18 (s, 3H : NCH,) ; 3,35 (d large, J = 17,5 Hz, 1H : 1H du CH₂ en 5ε) ; 3,47 (mt, 1H : l'autre 1H du CH₂ en 3δ) ; 3,70 (t, J = 7 Hz, 2H : CH₂O) ; 4,59 (dd, J = 8,5 et 6 Hz, 1H : CH en 3α) ; 4,78 (mt, 1H : CH en 2α) ; 4,83 (d large, J = 17,5 Hz, 1H : l'autre H du CH₂ en 5ε) ; 4,87 (d large, J = 10 Hz, 1H : CH en 1α) ; 5,12 (d, J = 5,5 Hz, 1H : CH en 5α) ; 5,24 (dd, J = 8 et 6,5 Hz, 1H : CH en 4α) ; 5,48 (mt, 1H : CH en 5γ) ; 5,54 (d, J = 7,5 Hz, 1H : CH en 6α) ; 5,89 (q, J = 7 Hz, 1H : CH en 1β) ; de 6,55 à 6,65 (mt, 1H : CONH en 2) ; 6,59 (d, J = 8 Hz, 2H : H aromatiques en 4ε) ; 6,92 (d, J = 8 Hz, 2H: H aromatiques en 4δ) ; de 7,25 à 7,40 (mt : les 7H correspondant aux H aromatiques en 6α - au 1' H₄ et au 1' H₅) ; 7,73 (d large, J = 4 Hz, 1H : 1' H₆) ; 8,39 (d, J = 10 Hz, 1H : CONH en 1) ; 8,43 (d, J = 7,5 Hz, 1H : CONH en 6) ; 11,67 (s large, 1 H :OH).

### Exemple 15

On opère comme à l'exemple 1, mais à partir de 100 cm³ d'acétonitrile, de 7 g d'un mélange brut contenant 33 % molaire de 5δ-chlorométhyl 5δ,5γ-déhydro pristinamycine I_{E} et de 1,25 cm³ de 1-(2-pyridyl)pipérazine. Le mélange réactionnel est chauffé 2 heures à 60°C, puis concentré sous pression réduite à 45°C (2,7 kPa). Le solide est repris par 50 cm³ d'eau et 100 cm³ de dichlorométhane. La phase aqueuse est réextraite par 30 cm³ de dichlorométhane. Les phases organiques sont rassemblées, séchées sur sulfate de magnésium, filtrées et concentrées à sec pour donner 7,6 g d'un solide beige qui est purifié par 2 chromatographies flash successives sur silice (éluant : dichlorométhane/méthanol 95/5 puis 98/2 en volumes). On obtient ainsi un solide qui est agité dans 60 cm³ d'éther diéthylique, filtré et séché à 40°C (90 Pa) pour donner 1,5 g de 5δ-[4-(2-pyridyl)pipérazin-1-yl]méthyl 5δ,5γ-déhydro pristinamycine I_{E}, sous forme d'une poudre beige fondant à 148°C.

Spectre de R.M.N. ¹H (400 MHz, CDCl₃) : 0,91 (t, J = 7,5 Hz, 3H : CH₃ en 2γ) ; 1,06 (d très large, J = 16 Hz, 1H : 1H du CH₂ en 5β) ; 1,22 (mt, 2H : 1H du CH₂ en 3β et 1H du CH₂ en 3γ) ; 1,32 (d, J = 7 Hz, 3H : CH₃ en 1γ) ; 1,55 (mt, 1H : l'autre H du CH, en 3γ) ; de 1,60 à 1,80 (mt : les 2H correspondant au CH₂ en 2β) ; 1,98 (mt, 1H : l'autre H du CH₂ en 3β) ; de 2,40 à 2,55 (mt, 5H : l'autre H du CH₂ en 5β et CH₂N de la pipérazine) ; 2,90 (s, 2H : CH₂N) ; 2,95 (s, 6H : ArN(CH₃)₂) ; 2,98 (dd, J = 14 et 6,5 Hz, 1H : 1H du CH₂ en 4β) ; de 3,10 à 3,30 (mt, 2H : 1H du CH₂ en 3δ et l'autre H du CH₂ en 4β) ; 3,18 (s, 3H : NCH₃) ; 3,36 (d large, J = 18 Hz, 1H : 1H du CH₂ en 5ε) ; de 3,40 à 3,65 (mt, 5H : l'autre H du CH₂ en 3δ et CH₂NAr de la pipérazine) ; 4,60 (dd, J = 8 et 5,5 Hz, 1H : CH en 3α) ; 4,78 (mt, 1H : CH en 2α) ; 4,88 (d large, J = 18 Hz, 1H : l'autre H du CH₂ en 5ε) ; 4,88 (dd, J = 10 et 1 Hz, 1H : CH en 1α) ; 5,12 (d large, J = 5,5 Hz, 1H : CH en 5α) ; 5,24 (dd, J = 9 et 6,5 Hz, 1H : CH en 4α) ; 5,51 (mt, 1H : CH en 5γ) ; 5,54 (d, J= 8,5 Hz, 1H : CH en 6α) ; 5,88 (q dédoublé, J = 7 et 1 Hz, 1H : CH en 1β) ; de 6,50 à 6,70 (mt, 3H : CONH en 2 - H en 3 de la pyridine et H en 5 de la pyridine) ; 6,60 (d, J = 8 Hz, 2H : H aromatiques en 4ε) ; 6,92(d, J = 8 Hz, 2H : H aromatiques en 4δ) ; de 7,20 à 7,40 (mt : les 7H correspondant aux H aromatiques en 6α - au 1' H₄ et au 1' H₅) ; 7,46 (t dédoublé, J = 8 et 2 Hz, 1H : H en 4 de la pyridine) ; 7,73 (dd, J = 4 et 1,5 Hz, 1H : 1' H₆) ; 8,18 (dd, J = 5 et 2 Hz, 1H : H en 6 de la pyridine) ; 8,40 (d, J = 10 Hz, 1H : CONH en 1) ; 8,48 (d, J = 8,5 Hz, 1H : CONH en 6) ; 11,67 (s, 1H : OH).

### Exemple 16

On opérant comme à l'exemple 1, mais à partir de 30 cm³ d'acétonitrile, de 3 g d'un mélange brut contenant 33 % molaire de 5δ-chlorométhyl-5δ,5γ-déhydro pristinamycine I_{E} et de 1,4 cm³ de N-benzyléthanolamine. Le mélange réactionnel est chauffé 8 heures à reflux, puis concentré sous pression réduite à 45°C (2,7 kPa). Le solide est repris par 50 cm³ d'eau et 10 cm³ d'acide chlorhydrique 2 N. La phase aqueuse est extraite par 2 fois 100 cm³ d'acétate d'éthyle et 2. fois 100 cm³ d'éther diéthylique puis est additionnée de 2 g de bicarbonate de sodium. Le précipité blanc apparu est filtré puis repris par 50 cm³ de dichlorométhane. La solution obtenue est lavée successivement par 3 fois 50 cm³ d'eau et 3 fois 50 cm³ d'eau saturée en chlorure de sodium puis séchée sur sulfate de magnésium, filtrée et concentrée à sec pour donner 2 g de produit blanc qui sont purifiés par chromatographie sur silice (éluant : gradient dichlorométhane/méthanol 99/1 à 96/4 en volumes). On obtient ainsi un solide qui est agité dans 60 cm³ d'éther diéthylique, filtré et séché à 40°C (90 Pa) pour donner 0,68 g de 5δ-[N-benzyl N-(2-hydroxyéthyl)]aminométhyl-5δ,5γ-déhydro pristinamycine I_{E}, sous forme d'un solide fondant à 148°C.

A ce solide dissous dans 10 cm³ d'acétate d'éthyle, on ajoute 0,063g d'acide méthanesulfonique. Le mélange obtenu est agité 1 heure puis dilué par 10 cm³ d'éther diéthylique. Après une nuit sous agitation, le précipité est filtré, lavé par 2 fois 5 cm³ d'éther diéthylique puis séché sous pression réduite, à 20°C (90 Pa), sur pentoxyde de phosphore. On obtient ainsi 0,5 g de méthanesulfonate de 5δ-[N-benzyl N-(2-hydroxyéthyl)]aminométhyl-5δ,5γ-déhydro pristinamycine I_{E}, sous forme de cristaux blancs fondants à 165°C.

Spectre de R.M.N. ¹H (400 MHz, CDCl₃) : De 0,85 à 1,05 (mt, 1H : 1H du CH₂ en 5β) ; 0,91 (t, J = 7,5 Hz, 3H : CH₃ en 2γ) ; de 1,10 à 1,35 (mt, 2H : 1H du CH₂ en 3β et 1H du CH₂ en 3γ) ; 1,30 (d, J = 7 Hz, 3H : CH₃ en 1γ) ; 1,56 (mt, 1H : l'autre H du CH₂ en 3γ) ; 1,65 et 1,72 (2 mts, 1H chacun : CH₂ en 2β) ; 2,02 (mt, 1H : l'autre H du CH₂ en 3β) ; 2,44 (d très large, J = 16 Hz, 1H : l'autre H du CH₂ en 5β) ; 2,84 (s, 3H : SO₂CH₃) ; de 2,90 à 3,30 (mt, 2H : NCH₂) ; de 2,95 à 3,05 (mt, 1H : 1H du CH₂ en 4β) ; 2,98 (s large, 6H : ArN(CH₃)₂) ; de 3,10 à 3,25 (mt, 2H : 1H du CH₂ en 3δ et l'autre H du CH₂ en 4β) ; 3,18 (s, 3H : NCH₃) ; de 3,35 à 4,00 (2 mfs étalés, 2H en totalité : CH₂N) ; de 3,40 à 3,55 (mt, 2H : 1H du CH₂ en 5ε et l'autre H du CH₂ en 3δ) ; 3,90 (s très large, 2H : CH₂O) ; 4,40 et 4,54 (2 mts, 1H chacun : ArCH₂N) ; 4,54 (mt, 1H : CH en 3α) ; 4,76 (mt, 1H : CH en 2α) ; de 4,85 à 4,95 (mt, 1H : l'autre H du CH₂ en 5ε) ; 4,87(d large, J = 10 Hz, 1H : CH en 1α) 5,13 (d large, J = 5 Hz, 1H : CH en 5α) ; 5,18 (dd, J = 9,5 et 5,5 Hz, 1H : CH en 4α) ; 5,66 (mt, 1H : CH en 6α) ; de 5,80 à 5,95 (mf, 1H : CH en 5γ) ; 5,86 (q.large, J = 7 Hz, 1H : CH en 1β) ; 6,53 (d, J = 9 Hz, 1H : CONH en 2) ; de 6,55 à 6,95 (mf étalé, 2H : H aromatiques en 4ε) ; 6,98 (mf, 2H : H aromatiques en 4δ) ; de 7,20 à 7,55 (mt : les 12H correspondant aux H aromatiques en 6α - aux H aromatiques du benzyle - au 1' H₄ et au 1' H₅) ; 7,73 (mf, 1H : 1' H₆) ; 8,34 (d, J = 10 Hz, 1H : CONH en 1) ; 8,72 (mf étalé, 1H : CONH en 6) ; de 9,60 à 10,50 (mf très étalé, 1H : OH du methanesulfonate) ;11,64 (s, 1H : OH).

### Exemple 17

On opère comme à l'exemple 1, mais à partir de 80 cm³ d'acétonitrile, de 8 g d'un mélange brut contenant 33 % molaire de 5δ-chlorométhyl-5δ,5γ-déhydro pristinamycine I_{E} et de 1,48 g de N-éthyloxycarbonylpipérazine. Le mélange réactionnel est chauffé à reflux 3 heures puis concentré sous pression réduite à 45°C (2,7 kPa). Le solide est repris par 200 cm³ de dichlorométhane. La solution obtenue est lavée par 2 fois 100 cm³ d'eau, décantée, puis séchée sur sulfate de magnésium, filtrée et concentrée à sec, pour donner 7,4 g d'un solide vert qui est purifié par 2 chromatographies flash sur silice (éluant : dichlorométhane/méthanol 95/5 puis 98/2 en volumes) puis par 2 chromatographies CLHP préparatives sur 500 g de silice 20-45 µm (éluant: dichlorométhane/méthanol 98/2 et 99/1) et enfin par CLHP préparative sur 450 g de silice C₈ 10µm (éluant: eau-acétonitrile 60/40 en volumes contenant 0,1 % d'acide trifluoroacétique). Les fractions contenant l'attendu sont rassemblées, l'acétonitrile éliminé à 40°C sous pression réduite (2,7 kPa) et le pH de la phase aqueuse ajusté à 7 par addition d'eau saturée en bicarbonate de sodium. Le précipité apparu est filtré, lavé par 30 cm³ d'eau, rincé par 2 fois 30 cm³ d'éther diisopropylique puis séché à 40°C (90 Pa) pour donner 0,36 g de 5δ-[4-éthyloxycarbonylpipérazin-1-yl]méthyl-5δ,5γ-déhydro pristinamycine I_{E}, sous forme d'une poudre blanche fondant vers 165°C.

Spectre de R.M.N. ¹H (400 MHz, CDCl₃) : 0,91 (t, J = 7,5 Hz, 3H : CH₃ en 2γ) ; 1,06 (d très large, J = 16 Hz, 1H : 1H du CH₂ en 5β) ; de 1,15 à 1,35 (mt, 2H : 1H du CH₂ en 3β et 1H du CH₂ en 3γ) ; 1,25 (t, J = 7,5 Hz, 3H : CH₃ de l'éthyle) ; 1,31 (d, J = 7 Hz, 3H : CH₃ en 1γ) ; 1,55 (mt, 1H : l'autre H du CH₂ en 3γ) ; de 1,60 à 1,80 (mt : les 2H correspondant au CH₂ en 2β) ; 1,98 (mt, 1H : l'autre H du CH₂ en 3β) ; de 2,15 à 2,60 (mf étalé, 4H : CH₂N de la pipérazine) ; 2,46 (dd, J = 16 et 5,5 Hz, 1H : l'autre H du CH₂ en 5β) ; de 2,85 à 3,05 (mt, 3H : CH₂N et 1H du CH₂ en 4β) ; 2,93 (s, 6H : ArN(CH₃)₂); de 3,15 à 3,30 (mt, 2H : 1H du CH₂ en 3δ et l'autre H du CH₂ en 4β) ; 3,17 (s, 3H : NCH₃) ; de 3,30 à 3,75 (mf étalé, 4H : CH₂NCO de la pipérazine) ; 3,34 (d large, J = 18 Hz, 1H : 1H du CH₂ en 5ε) ; 3,46 (mt, 1H : l'autre H du CH₂ en 3δ) ; 4,13 (q, J = 7,5 Hz, 2H : COOCH₂) ; 4,57 (dd, J = 8 et 5,5 Hz, 1H : CH en 3α) ; 4,77 (mt, 1H : CH en 2α) ; 4,83 (d large, J = 18 Hz, 1H : l'autre H du CH₂ en 5ε) ; 4,87 (dd, J = 10 et 1 Hz, 1H : CH en 1α) ; 5,11 (d large, J = 5,5 Hz, 1H : CH en 5α) ; 5,21 (dd, J = 9 et 6,5 Hz, 1H : CH en 4α) ; de 5,50 à 5,60 (mt, 1H : CH en 5γ) ; 5,53 (d, J = 7,5 Hz, 1H : CH en 6α) ; 5,88 (q dédoublé, J = 7 et 1 Hz, 1H : CH en 1β) ; de 6,50 à 6,60 (mt, 1H : CONH en 2) ; 6,58 (d, J = 8 Hz, 2H : H aromatiques en 4ε) ; 6,90 (d, J = 8 Hz, 2H: H aromatiques en 4δ); de 7,20 à 7,40 (mt: les 7H correspondant aux H aromatiques en 6α - au 1' H₄ et au 1' H₅) ; 7,72 (dd, J = 4 et 1,5 Hz, 1H : 1' H₆) ; 8,38 (d, J = 10 Hz, 1H : CONH en 1) ; 8,51 (d, J = 7,5 Hz, 1H : CONH en 6) ; 11,66 (s, 1H : OH).

### Exemple 18

On opère comme à l'exemple 1, mais à partir de 30 cm³ d'acétonitrile, de 3 g d'un mélange brut contenant 33 % molaire de 5δ-chlorométhyl-5δ,5γ-déhydro pristinamycine I_{E} et de 1,8 g de 1-(2-furoyl)pipérazine. Le mélange réactionnel est chauffé à reflux 24 heures puis concentré sous pression réduite à 45°C (2,7 kPa). Le solide obtenu est repris par 50 cm³ d'eau et 10 cm¹ d'acide chlorhydrique 2 N. La solution obtenue est extraite par 3 fois 100 cm³ d'acétate d'éthyle et 2 fois 100 cm³ d'éther diéthylique puis additionnée de 2 g de bicarbonate de sodium. Le précipité blanc apparu est filtré, lavé par 6 fois 20 cm³ d'eau puis dissous dans 80 cm³ de dichlorométhane. La solution obtenue est lavée par 3 fois 50 cm³ d'eau et 2 fois 50 cm³ d'eau saturée en chlorure de sodium puis concentrée à sec pour donner 2,15 g de solide qui est chromatographié sur silice (éluant : gradient dichlorométhane/méthanol 100/0 à 97/3 en volumes). On obtient un solide qui est repris par 80 cm³ d'éther diéthylique, filtré, rincé par 3 fois 10 cm³ d'éther diéthylique puis séché à 40°C (90 Pa) pour donner 0,73 g de 5δ-[4-(2-furoyl)pipérazin-1-yl]méthyl-5δ,5γ-déhydro pristinamycine I_{E}, sous forme de cristaux blanc cassé fondant vers 148°C.

Spectre de R.M.N. ¹H (400 MHz, CDCl₃) : 0,91 (t, J = 7,5 Hz, 3H : CH₃ en 2γ) ; 1,08 (d très large, J = 16 Hz, 1H : 1H du CH₂ en 5β) ; 1,25 (mt, 2H : 1H du CH₂ en 3β et 1H du CH₂ en 3γ) ; 1,30 (d, J = 7 Hz, 3H : CH₃ en 1γ) ; 1,56 (mt, 1H : l'autre H du CH₂ en 3γ) ; de 1,60 à 1,80 (mt : les 2H correspondant au CH₂ en 2β) ; 2,00 (mt, 1H : l'autre H du CH₂ en 3β) ; de 2,25 à 2,55 (mf étalé, 4H : les 2 CH₂N de la pipérazine) ; 2,46 (dd, J = 16 et 5 Hz, 1H : l'autre H du CH₂ en 5β) ; 2,88 (s, 2H : CH₂N) ; 2,93 (s, 6H : ArN(CH₃)₂) ; 2,98 (dd, J = 14 et 6,5 Hz, 1H : 1H du CH₂ en 4β) ; de 3,15 à 3,30 (mt, 2H : 1H du CH₂ en 3δ et l'autre H du CH₂ en 4β) ; 3,19 (s, 3H : NCH₃) ; 3,34 (d large, J = 18 Hz, 1H : 1H du CH₂ en 5ε) ; 3,46 (mt, 1H : l'autre H du CH₂ en 3δ) ; de 3,50 à 4,10 (mf très étalé, 4H : les 2 CH₂NCO de la pipérazine) ; 4,58 (dd, J = 8 et 5,5 Hz, 1H : CH en 3α) ; 4,77 (mt, 1H : CH en 2α) ; 4,84 (d large, J= 18 Hz, 1H : l'autre H du CH₂ en 5ε) ; 4,87 (dd, J = 10 et 1 Hz, 1H : CH en 1α) ; 5,12 (d large, J = 5,5 Hz, 1H : CH en 5α) ; 5,24 (dd, J = 9,5 et 6,5 Hz, 1H : CH en 4α) ; 5,50 (mt, 1H : CH en 5γ) ; 5,53 (d, J = 8 Hz, 1H : CH en 6α) ; 5,87 (q dédoublé, J = 7 et 1 Hz, 1H : CH en 1β) ; 6,46 (dd, J = 3 et 2 Hz, 1H : H en 4 du furanne) ; de 6,55 à 6,65 (mt, 1H : CONH en 2) ; 6,58 (d, J = 8 Hz, 2H : H aromatiques en 4ε) ; 6,92 (d, J = 8 Hz, 2H : H aromatiques en 4δ) ; 6,98 (dd, J = 3 Hz, 1H : H en 3 du furanne) ; de 7,20 à 7,40 (mt : les 7H correspondant aux H aromatiques en 6α - au 1' H₄ et au 1' H₅) ; 7,48 (dd, J = 2 Hz, 1H : H en 5 du furanne) ; 7,73 (dd, J = 4 et 1,5 Hz, 1H : 1' H₆) ; 8,41 (d, J = 10 Hz, 1H: CONH en 1) ; 8,53 (d, J = 8 Hz, 1H: CONH en 6) ; 11,67 (s large, 1H : OH).

### Exemple 19

On opère comme à l'exemple 1, mais à partir de 60 cm³ d'acétonitrile, de 6 g d'un mélange brut contenant 33% molaire de 5δ-chlorométhyl-5δ,5γ-déhydro pristinamycine I_{E} et de 4,1 cm³ de N'-benzyl N,N-diméthyléthylènediamine. Le mélange réactionnel est chauffé à reflux 15 heures puis concentré à sec sous pression réduite à 45°C (2,7 kPa). Le solide obtenu est repris par 100 cm³ d'eau et par une solution d'acide chlorhydrique 2 N de façon à obtenir un pH de 1. La phase aqueuse est extraite par 2 fois 100 cm³ d'éther diéthylique et 2 fois 100 cm³ d'acétate d'éthyle, ajustée à pH 7 par addition d'eau saturée en bicarbonate de sodium puis extraite par 2 fois 100 cm³ de dichlorométhane. La phase organique est séchée sur sulfate de sodium, filtrée et concentrée à sec sous pression réduite pour donner 7,4 g de solide qui est chromatographié sur silice (éluant : dichlorométhane/méthanol 98/2 en volumes). On obtient un solide qui est agité dans 50 cm³ d'éther puis filtré et séché pour donner 0,64 g d'un solide blanc qui est repris par 20 cm³ d'eau et par une solution d'acide chlorhydrique de façon à obtenir un pH de 1. On extrait par 2 fois 20 cm³ d'éther diéthylique. A la phase aqueuse on ajoute une solution saturée de bicarbonate de sodium et le précipité est filtré et séché 20°C (90 Pa) sur P₂O₅, pour donner 0,14 g de 5δ-N-benzyl-N-(2-diméthylaminoéthyl)aminométhyl-5δ,5γ-déhydro pristinamycine I_{E}, sous forme d'une poudre blanche fondant à 182°C.

Spectre de R.M.N. ¹H (400 MHz, CDCl₃) : 0,92 (t, J = 7,5 Hz, 3H : CH₃ en 2γ) ; 1,06 (d très large, J = 16 Hz, 1H : 1H du CH₂ en 5β) ; de 1,20 à 1,35 (mt, 2H : 1H du CH₂ en 3β et 1H du CH₂ en 3γ) ; 1,29 (d, J = 7 Hz, 3H : CH₃ en 1γ) ; de 1,50 à 1,85 (mt : les 3H correspondant à l'autre H du CH₂ en 3γ et au CH₂ en 2β) ; 2,02 (mt, 1H : l'autre H du CH₂ en 3β) ; 2,33 (mf, 6H : les 2 NCH₃ de la diméthylamine) ; 2,43 (dd large, J = 16 et 5 Hz, 1H : l'autre H du CH₂ en 5β) ; 2,59 (mf, 4H : NCH₂CH₂N) ; de 2,85 à 3,05 (mt, 1H : 1H du CH₂ en 4β) ; 2,91 (s, 6H : ArN(CH₃)₂) ; 3,00 (s, 2H : CH₂N) ; de 3,15 à 3,30 (mt, 2H : 1H du CH₂ en 3δ et l'autre H du CH₂ en 4β) ; 3,17 (s, 3H : NCH₃) ; 3,33 (d large, J = 18 Hz, 1H : 1H du CH₂ en 5ε) ; 3,46 (mt, 1H : l'autre H du CH₂ en 3δ) ; 3,55 (s, 2H : ArCH₂N) ; 4,57 (dd, J = 8 et 6 Hz, 1H : CH en 3α) ; 4,78 (mt, 1H : CH en 2α) ; 4,87 (d large, J = 18 Hz, 1H : l'autre H du CH₂ en 5ε) ; 4,87 (dd, J = 10 et 1 Hz, 1H : CH en 1α) ; 5,13 (d large, J = 5 Hz, 1H : CH en 5α) ; 5,24 (dd, J = 10 et 6 Hz, 1H : CH en 4α) ; 5,46 (d, J = 8 Hz, 1H : CH en 6α) ; 5,55 (mt, 1H : CH en 5γ) ; 5,85 (q dédoublé, J = 7 et 1 Hz, 1H : CH en 1β) ; 6,54 (d, J = 8 Hz, 2H : H aromatiques en 4ε) ; 6,57 (d, J = 10 Hz, 1H: CONH en 2) ; 6,88 (d, J = 8 Hz, 2H : H aromatiques en 4δ) ; de 7,15 à 7,40 (mt : les 7H correspondant aux H aromatiques en 6α - au 1' H₄ et au 1' H₅) ; 7,71 (dd, J = 4 et 1,5 Hz, 1H : 1' H₆) ; 8,38 (d, J = 10 Hz, 1H : CONH en 1) ; 8,53 (d, J = 8 Hz, 1H : CONH en 6) ; 11,66 (mf, 1H : OH).

### Exemple 20

On opère comme à l'exemple 1, mais à partir de 100 cm³ d'acétonitrile, de 20 g d'un mélange brut contenant 33 % molaire de 5δ-chlorométhyl-5δ,5γ-déhydro pristinamycine I_{E} et de 4,5 g de chlorhydrate de 4-phényl-1,2,3,6 tétrahydropyridine et de 3,25 cm³ de triéthylamine. Le mélange réactionnel est chauffé à reflux 1,5 heure puis concentré à sec, sous pression réduite à 45°C (2,7 kPa). Le solide obtenu est repris par 100 cm³ d'eau puis filtré, lavé par 20 cm³ d'eau et séché sous pression réduite, pour donner 26 g de solide. Ce solide est purifié par chromatographie sur silice (éluant : dichlorométhane/méthanol 97/3 en volumes) puis par CLHP préparative sur 450 g de silice C₈ 10 µm (éluant: eau-acétonitrile 65/35 en volumes contenant 0,1 % d'acide trifluoroacétique). Les fractions contenant l'attendu sont rassemblées, l'acétonitrile éliminé à 40°C sous pression réduite (2,7 kPa) et le pH de la solution aqueuse ajusté à 7-8 par addition d'eau saturée en bicarbonate de sodium. Le précipité apparu est filtré, lavé par 20 cm³ d'eau et séché sous pression réduite à 40°C (90 Pa), pour donner 0,47 g de 5δ-[1-(4-phényl)-1,2,3,6 tétrahydropyridyl] méthyl-5δ,5γ-déhydro pristinamycine I_{E}, sous forme d'un solide blanc fondant à 154°C.

Spectre de R.M.N. ¹H (400 MHz, CDCl₃) : 0,92 (t, J = 7,5 Hz, 3H : CH₃ en 2γ) ; de 1,00 à 1,15 (mf étalé, 1H : 1H du CH₂ en 5β) ; 1,22 (mt, 2H : 1H du CH₂ en 3β et 1H du CH₂ en 3γ) ; 1,32 (d, J = 7 Hz, 3H : CH₃ en 1γ) ; 1,54 (mt, 1H : l'autre H du CH₂ en 3γ), de 1,60 à 1,85 (mt : les 2H correspondant au CH₂ en 2β) ; 1,99 (mt, 1H : l'autre H du CH₂ en 3β) ; de 2,40 à 2,80 (mt, 5H : NCH₂CH₂ de la 1,2,3,6-tétrahydropyridine et l'autre H du CH₂ en 5β) ; de 2,90 à 3,30 (mt, 7H : CH₂N - CH₂ en 4β - NCH₂ de la 1,2,3,6-tétrahydropyridine et 1H du CH₂ en 3δ) ; 2,95 (s, 6H : ArN(CH₃)₂) ; 3,17 (s, 3H : NCH₃) ; de 3,35 à 3,55 (mt, 2H : 1H du CH₂ en 5ε et l'autre H du CH₂ en 3δ) ; 4,57 (dd, J = 8 et 5,5 Hz, 1H : CH en 3α) ; 4,77 (mt, 1H : CH en 2α) ; de 4,80 à 4,95 (mt, 1H : l'autre H du CH₂ en 5ε) ; 4,87 (dd, J = 10 et 1 Hz, 1H : CH en 1α) ; 5,14 (d, J = 5,5 Hz, 1H : CH en 5α) ; 5,22 (mt, 1H : CH en 4α) ; de 5,50 à 5,65 (mf, 1H : CH en 5γ) ; 5,57 (d, J = 8 Hz, 1H : CH en 6α) ; 5,88 (q dédoublé, J = 7 et 1 Hz, 1H : CH en 1β) ; 6,06 (mt, 1H : CH= de la 1,2,3,6-tétrahydropyridine ) ; 6,56 (d, J = 9 Hz, 1H : CONH en 2) ; 6,60 (d, J = 8 Hz, 2H : H aromatiques en 4ε) ; 6,93 (d, J = 8 Hz, 2H : H aromatiques en 4δ) ; de 7,20 à 7,45 (mt : les 12H correspondant aux H aromatiques en 6α - aux H aromatiques du phényle - au 1' H₄ et au 1' H₅) ; 7,73 (dd, J = 4 et 1 Hz, 1H : 1' H₆) ; 8,39 (d, J = 10 Hz, 1H : CONH en 1) ; 8,49 (mf, 1H : CONH en 6) ; 11,66 (s, 1H : OH).

### Exemple 21

On opère comme à l'exemple 1, mais à partir de 30 cm³ d'acétonitrile, de 3 g d'un mélange brut contenant 33 % molaire de 5δ-chlorométhyl-5δ,5γ-déhydro pristinamycine I_{E} et de 1,9 g de 4-benzyl-4-hydroxy-pipéridine. Le mélange réactionnel est chauffé à reflux durant 20 heures puis concentré à sec sous pression réduite à 45°C (2,7 kPa). Le solide obtenu est repris par 50 cm³ d'eau et 10 cm³ d'acide chlorhydrique 2 N. La solution obtenue est extraite successivement par 4 fois 50 cm³ d'acétate d'éthyle et par 2 fois 50 cm³ d'éther diéthylique, ajustée à pH 7-8 par addition 2 g de bicarbonate de sodium puis extraite par 3 fois 100 cm³ de dichlorométhane. La phase organique est décantée, séchée sur sulfate de sodium, filtrée et concentrée à sec à 45°C sous pression réduite (2,7 kPa) pour donner 2,6 g de solide qui est chromatographié sur silice (éluant : gradient dichlorométhane/méthanol 99,5/0,5 puis 90/10 en volumes). On obtient un solide qui est agité dans 50 cm³ d'éther diisopropylique puis filtré et séché sous pression réduite à 40°C (90 Pa) pour donner 0,2 g de 5δ-(4-benzyl-4-hydroxy-pipéridinométhyl)-5δ,5γ-déhydro pristinamycine I_{E}, sous forme de cristaux blanc cassé fondant à 172°C.

Spectre de R.M.N. ¹H (400 MHz, CDCl₃) : 0,92 (t, J = 7,5 Hz, 3H : CH₃ en 2γ) ; 1,03 (d très large, J = 16 Hz, 1H : 1H du CH₂ en 5β) ; de 1,15 à 1,35 (mt, 2H : 1H du CH₂ en 3β et 1H du CH₂ en 3γ) ; 1,30 (d, J = 7 Hz, 3H : CH₃ en 1γ) ; de 1,50 à 1,90 (mt : les 3H correspondant à l'autre H du CH₂ en 3γ et au CH₂ en 2β) ; de 1,50 à 1,90 - de 2,10 à 2,35 et de 2,50 à 2,65 (3 séries de mt : les 10H correspondant aux NCH₂CH₂ de la pipéridine et CH₂Ar) ; 2,00 (mt, 1H : l'autre H du CH₂ en 3β) ; 2,45 (mt, 1H : l'autre H du CH₂ en 5β) ; de 2,75 à 3,05 (mt, 3H : CH₂N et 1H du CH₂ en 4β) ; 2,93 (s, 6H : ArN(CH₃)₂) ; de 3,15 à 3,30 (mt, 2H : l'autre H du CH₂ en 4β et 1H du CH₂ en 3δ) ; 3,18 (s, 3H : NCH₃) ; 3,30 (d large, J = 18 Hz, 1H : 1H du CH₂ en 5ε) ; 3,47 (mt, 1H : l'autre H du CH₂ en 3δ) ; 4,58 (dd, J = 8 et 5,5 Hz, 1H : CH en 3α) ; 4,78 (mt, 1H : CH en 2α) ; de 4,80 à 4,90 (mt, 1H : l'autre H du CH₂ en 5ε) ; 4,87 (d large, J = 10 Hz, 1H : CH en 1α) ; 5,11 (d large, J = 5,5 Hz, 1H : CH en 5α) ; 5,22 (dd, J = 9 et 7 Hz, 1H : CH en 4α) ; 5,47 (mt, 1H : CH en 5γ) ; 5,54 (d, J = 8 Hz, 1H : CH en 6α) ; 5,87 (q large, J = 7 Hz, 1H : CH en 1β) ; de 6,55 à 6,65 (mt, 1H : CONH en 2) ; 6,58 (d, J = 8 Hz, 2H : H aromatiques en 4ε) ; 6,91 (d, J = 8 Hz, 2H : H aromatiques en 4δ) ; de 7,10 à 7,40 (mt : les 12H correspondant aux H aromatiques en 6α - aux H aromatiques du phényle - au 1' H₄ et au 1' H₅) ; 7,72 (d large, J = 4 Hz, 1H : 1' H₆) ; 8,39 (d, J = 10 Hz, 1H : CONH en 1) ; 8,46 (d, J = 8 Hz, 1H : CONH en 6) ; 11,67 (s, 1H : OH).

### Exemple 22

En opérant comme à l'exemple 1, mais à partir de 30 cm³ d'acétonitrile, de 3 g d'un mélange brut contenant 33 % molaire de 5δ-chlorométhyl-5δ,5γ-déhydro pristinamycine I_{E} et de (5δR)-5δ-méthylène-5γ-chloro pristinamycine I_{E} (en proportion 33/67) et de 1,25 g de N-allyl-cyclopentylamine. Le mélange réactionnel est chauffé à reflux durant 28 heures puis concentré à sec sous pression réduite à 45°C (2,7 kPa). Le solide obtenu est repris par 50 cm³ d'eau et 10 cm³ d'acide chlorhydrique 2 N. La solution obtenue est extraite successivement par 4 fois 50 cm³ d'acétate d'éthyle et par 2 fois 50 cm³ d'éther diéthylique puis ajustée à pH 8 par addition de 2 g de bicarbonate de sodium. Le précipité apparu est filtré, lavé par de l'eau pour éliminer les sels minéraux restants éventuellement puis dissous dans 50 cm³ de dichlorométhane. La solution résultante est lavé par 4 fois 50 cm³ d'eau et par 2 fois 50 cm³ d'eau saturée en chlorure de sodium puis séchée sur sulfate de sodium, filtrée et concentrée à sec à 45°C sous pression réduite (2,7 kPa). On obtient ainsi 2,3 g de solide qui est chromatographié sur silice (éluant: gradient dichlorométhane/méthanol 100/0 puis 98/2 en volumes). On obtient un solide qui est agité dans 50 cm³ d'éther diisopropylique, filtré, lavé par 3 fois 10 cm³ d'éther diisopropylique puis séché sous pression réduite à 20°C (90 Pa), pour donner 0,59 g de 5δ-(N-allyl-N-cyclopentyl)aminométhyl-5δ,5γ-déhydro pristinamycine I_{E}, sous forme de cristaux blancs fondant à 140°C.

Spectre de R.M.N. ¹H (400 MHz, CDCl₃) : 0,93 (t, J = 7,5 Hz, 3H : CH₃ en 2γ) ; 1,07 (d très large, J = 16 Hz, 1H : 1H du CH₂ en 5β) ; de 1,15 à 1,35 (mt, 2H : 1H du CH₂ en 3β et 1H du CH₂ en 3γ) ; 1,31 (d, J = 7 Hz, 3H : CH₃ en 1γ) ; de 1,35 à 1,85 (mt : les 11H correspondant à l'autre H du CH₂ en 3γ - aux CH₂ du cyclopentane et au CH₂ en 2β) ; 2,00 (mt, 1H : l'autre H du CH₂ en 3β) ; 2,47 (mt, 1H : l'autre H du CH₂ en 5β) ; de 2,85 à 3,05 (mt, 3H : 1H du CH₂ en 4β - CH₂N) ; 2,93 (s, 6H : ArN(CH₃)₂) ; de 3,05 à 3,20 (mt, 3H : NCH et NCH₂ de l'allyle) ; de 3,15 à 3,45 (mt, 3H : 1H du CH₂ en 3δ - l'autre H du CH₂ en 4β et 1H du CH₂ en 5ε) ; 3,17 (s, 3H : NCH₃) ; 3,47 (mt, 1H : l'autre H du CH₂ en 3δ) ; 4,58 (dd, J = 8,5 et 6 Hz, 1H : CH en 3α) ; 4,78 (mt, 1H : CH en 2α) ; de 4,80 à 4,95 (mt, 1H : l'autre H du CH₂ en 5ε) ; 4,86 (d large, J = 10 Hz, 1H : CH en 1α) ; de 5,00 à 5,30 (mt, 4H : =CH₂ de l'allyle - CH en 5α et CH en 4α) ; 5,47 (mt, 1H : CH en 5γ) ; 5,55 (d, J = 8 Hz, 1H : CH en 6α) ; de 5,80 à 5,95 (mt, 21H : CH= de l'allyle et CH en 1β) ; 6,56 (d, J = 10 Hz, 1H : CONH en 2) ; 6,60 (d, J = 8 Hz, 2H : H aromatiques en 4ε) ; 6,91 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,73 (dd, J = 4 et 1 Hz, 1H : 1' H₆) ; de 8,35 à 8,45 (mt, 2H : CONH en 1 et CONH en 6) ; 11,66 (s, 1H : OH).

### Exemple 23

En opérant comme à l'exemple 1, mais à partir d'un mélange brut contenant la 5δ-chlorométhyl-5δ,5γ-déhydro-4ε-chloro pristinamycine I_{E}, on prépare 0,5 g de 5δ-morpholinométhyl-5δ,5γ-déhydro-4ε-chloro pristinamycine I_{E}, sous forme d'un solide blanc fondant à 150°C.

Spectre de R.M.N. ¹H (400 MHz, CDCl₃) : 0,92 (t, J = 7,5 Hz, 3H : CH₃ en 2γ); de 1,10 à 1,35 (mt, 3H : 1H du CH₂ en 5β - 1H du CH₂ en 3β et 1H du CH₂ en 3γ) ; 1,31 (d, J = 7 Hz, 3H : CH₃ en 1γ) ; 1,57 (mt, 1H : l'autre H du CH₂ en 3γ); 1,67 et 1,75 (2 mts, 1H chacun : CH₂ en 2β) ; 1,99 (mt, 1H : l'autre H du CH₂ en 3β) ; 2,35 (mf, 4H : NCH₂ de la morpholine) ; 2,57 (dd large, J = 16 et 5 Hz, 1H : l'autre H du CH₂ en 5β) ; 2,80 (s, 6H : ArN(CH₃)₂) ; 2,88 (AB limite, J = 14 Hz, 2H : CH₂N) ; 3,02 (dd, J = 14 et 7,5 Hz, 1H : 1H du CH₂ en 4β) ; de 3,10 à 3,25 (mt, 1H : l'autre H du CH₂ en 4β) ; 3,14 (s, 3H : NCH₃) ; 3,28 (mt, 1H : 1H du CH₂ en 3δ) ; 3,36 (d large, J = 18 Hz, 1H : 1H du CH₂ en 5ε) ; 3,48 (mt, 1H : l'autre H du CH₂ en 3δ) ; 3,70 (mt, 4H : CH₂O de la morpholine) ; 4,56 (dd, J = 8 et 6 Hz, 1H : CH en 3α) ; de 4,75 à 4,85 (mt, 2H : CH en 2α et l'autre H du CH₂ en 5ε) ; 4,87 (dd, J = 10 et 1Hz, 1H : CH en 1α) ; 5,13 (d, J = 5 Hz, 1H : CH en 5α) ; 5,26 (dd, J = 8 et 7,5 Hz, 1H : CH en 4α) ; 5,50 (d, J = 8 Hz, 1H : CH en 6α) ; 5,55 (mt, 1H : CH en 5γ) ; 5,88 (q dédoublé, J = 7 et 1 Hz, 1H : CH en 1β) ; 6,56 (d, J = 9,5 Hz, 1H : CONH en 2) ; 6,87 (AB limite, 2H : H aromatique en 4ε et H aromatique en 4δ et en para du Cl) ; 7,09 (d, J = 2 Hz, 1H : H aromatique en 4δ et en ortho du Cl) ; de 7,25 à 7,40 (mt : les 7H correspondant aux H aromatiques en 6α - au 1' H₄ et au 1' H₅) ; 7,69 (dd, J = 4 et 2 Hz, 1H : 1' H₆) ; 8,33 (d, J = 10 Hz, 1H : CONH en 1) ; 8,46 (d, J = 8 Hz, 1H : CONH en 6) ; 11,68 (s, 1H : OH).

La 5δ-chlorométhyl-5δ,5γ-déhydro-4ε-chloro pristinamycine peut être préparée à partir de la 4ε-chloro-5δ-méthylène pristinamycine IA par analogie avec la méthode décrite à l'exemple 1.

La 4ε-chloro-5δ-méthylène pristinamycine IA peut être obtenue de la manière suivante :

A 11,4 g de 5δ-méthylène pristinamycine IA en solution dans 120 cm³ d'acétonitrile, on ajoute 1,9 g de N-chlorosuccinimide sous atmosphère d'argon. Le mélange est agité au reflux pendant 2 heures puis additionné de 346 mg de N-chlorosuccinimide supplémentaires. Après 1,5 heure de reflux supplémentaire et agitation 18 heures à 20°C, le mélange réactionnel est concentré à sec sous pression réduite (2,7 kPa), à 30°C. Le solide obtenu est agité 4 heures dans 250 cm³ d'éther diéthylique, filtré, rincé puis séché sous hotte à 20°C pour donner 11,7 g de 4ε-chloro-5δ-méthylène pristinamycine IA sous forme d'une poudre rose utilisée telle quelle.

### Exemple 24

On opère comme à l'exemple 1 mais à partir d'un mélange brut contenant la 5δ-chlorométhyl-5δ,5γ-déhydro-4ε-chloro pristinamycine I_{E} pour donner 0,25 g de 5δ-pipéridinométhyl-5δ,5γ-déhydro-4ε-chloro pristinamycine I_{E}, sous forme d'un solide blanc se décomposant vers 160°C.

Spectre de R.M.N. ¹H (500 MHz, CDCl₃) : 0,91 (t, J = 7,5 Hz, 3H : CH₃ en 2γ) ; de 1,10 à 1,35 (mt, 3H : 1H du CH₂ en 5β - 1H du CH₂ en 3β et 1H du CH₂ en 3γ) ; 1,30 (d, J = 7 Hz, 3H : CH₃ en 1γ) ; de 1,35 à 1,75 (mt : les 8H correspondant à l'autre H du CH₂ en 3γ - aux CH₂CH₂CH₂ de la pipéridine et à 1H du CH₂ en 2β) ; 1,74 (mt, 1H : l'autre H du CH₂ en 2β) ; 1,98 (mt, 1H : l'autre H du CH₂ en 3β) ; 2,27 (mf, 4H : NCH₂ de la pipéridine) ;de 2,50 à 2,65 (mt, 1H : l'autre H du CH₂ en 5β) ; 2,78 (s, 6H : ArN(CH₃)₂) ; 2,80 (s, 2H : CH₂N) ; 3,01 (mt, 1H : 1H du CH₂ en 4β) ; de 3,10 à 3,25 (mt, 1H : l'autre H du CH₂ en 4β) ; 3,14 (s, 3H : NCH₃) ; 3,26 (mt, 1H : 1H du CH₂ en 3δ) ; 3,36 (mt, 1H : 1H du CH₂ en 5ε) ; 3,46 (mt, 1H : l'autre H du CH₂ en 3δ) ; 4,56 (dd, J = 7 et 6 Hz, 1H : CH en 3α) ; de 4,70 à 4,85 (mt, 2H : CH en 2α et l'autre H du CH₂ en 5ε) ; 4,86 (d large, J = 10 Hz, 1H : CH en 1α) ; 5,12 (d, J = 5 Hz, 1H : CH en 5α) ; 5,25 (mt, 1H : CH en 4α) ; de 5,45 à 5,55 (mt, 2H : CH en 5γ et CH en 6α) ; 5,87 (q large, J = 7 Hz, 1H : CH en 1β) ; 6,55 (d, J = 9 Hz, 1H : CONH en 2) ; 6,86 (mt, 2H : H aromatique en 4ε et H aromatique en 4δ en para du Cl) ; 7,09 (s large, 1H : H aromatique en 4δ en ortho du Cl) ; de 7,20 à 7,40 (mt : les 7H correspondant aux H aromatiques en 6α - au 1' H₄ et au 1' H₅) ; 7,69 (mt, 1H : 1' H₆) ; de 8,30 à 8,45 (mt, 2H : CONH en 1 et CONH en 6) ; 11,68 (s, 1H : OH).

### Exemple 25

On opère comme à l'exemple 1, mais à partir d'un mélange brut contenant la 5δ-chlorométhyl-5δ,5γ-déhydro-4ε-chloro pristinamycine I_{E} pour donner 0,35 g de 5δ-(2,6-diméthylmorpholino)méthyl-5δ,5γ-déhydro-4ε-chloro pristinamycine I_{E}, sous forme d'un solide blanc fondant à 179°C.

Spectre de R.M.N. ¹H (400 MHz, CDCl₃) : Nous observons un mélange des deux diastéréoisomères cis et trans sur la morpholine, ainsi que la présence de traces infimes d'autres pristinamycines non identifiées et de conformères.

0,92 (t, J = 7,5 Hz, 3H : CH₃ en 2γ) ; de 1,05 à 1,40 (mt, 3H : 1H du CH₂ en 5β - 1H du CH₂ en 3β et 1H du CH₂ en 3γ) ; 1,15 et 1,19 (2 d, J = 7 Hz, 3H chacun : CH₃ de la 2,6 diméthyl morpholine) ; 1,31 (d, J = 7 Hz, 3H : CH₃ en 1γ) ; de 1,50 à 1,75 (mt : les 4H correspondant à l'autre H du CH₂ en 3γ - à 1H du CH₂ en 2β et à 2H des NCH₂ de la 2,6 diméthyl morpholine) ; 1,75 (mt, 1H : l'autre H du CH₂ en 2β) ; 2,00 (mt, 1H : l'autre H du CH₂ en 3β); 2,57 (dd large, J = 16 et 5,5 Hz, 1H : l'autre H du CH₂ en 5β) ; 2,66 (mt, 2H : les 2 autres H des NCH₂ de la 2,6 diméthyl morpholine) ; 2,80 (s, 6H : ArN(CH₃)₂) ; 2,85 (s large, 2H : CH₂N) ; 3,03 (dd, J = 14 et 7 Hz, 1H : 1H du CH₂ en 4β) ; de 3,10 à 3,25 (mt, 1H : l'autre H du CH₂ en 4β) ; 3,16 (s, 3H : NCH₃) ; 3,30 (mt, 1H : 1H du CH₂ en 3δ) ; 3,35 (d large, J = 18 Hz, 1H : 1H du CH₂ en 5ε) ; 3,48 (mt, 1H : l'autre H du CH₂ en 3δ) ; 3,59 et 3,69 (2 mts, 1H chacun : CHO de la morpholine) ; 4,58 (dd, J = 7 et 5 Hz, 1H : CH en 3α) ; de 4,70 à 4,85 (mt, 2H : CH en 2α et l'autre H du CH₂ en 5ε) ; 4,87 (d large, J = 10 Hz, 1H : CH en 1α) ; 5,13 (d, J = 5,5 Hz, 1H: CH en 5α) ; 5,27 (mt, 1H : CH en 4α) ; 5,51 (d, J = 7,5 Hz, 1H : CH en 6α) ; 5,53 (mt, 1H : CH en 5γ) ; 5,88 (q large, J = 7 Hz, 1H : CH en 1β) ; 6,57 (d, J = 9,5 Hz, 1H : CONH en 2) ; 6,87 (mt, 2H : H aromatique en 4ε et H aromatique en 4δ en para du Cl) ; 7,11 (s large, 1H : H aromatique en 4δ en ortho du Cl) ; de 7,20 à 7,40 (mt : les 7H correspondant aux H aromatiques en 6α - au 1' H₄ et au 1' H₅) ; 7,70 (mt, 1H : 1' H₆) ; 8,35 (d, J = 10 Hz, 1H : CONH en 1) ; 8,46 (d, J = 8 Hz, 1H : CONH en 6) ; 11,68 (s, 1H : OH).

### Exemple 26

On opère comme à l'exemple 1, mais à partir d'un mélange brut contenant la 5δ-chlorométhyl-5δ,5γ-déhydro-4ε-chloro pristinamycine I_{E}. On obtient ainsi 0,46 g de 5δ-N,N-dipropylaminométhyl 5δ,5γ-déhydro-4ε-chloro pristinamycine I_{E}, sous forme d'un solide jaune fondant à 139°C.

Spectre de R.M.N. ¹H (400 MHz, CDCl₃) : de 0,85 à 0,95 (mt, 9H : CH₃ en 2γ et CH₃ de la dipropylamine) ; 1,14 (d très large, J = 16 Hz, 1H : 1H du CH₂ en 5β) ; de 1,20 à 1,35 (mt, 2H : 1H du CH₂ en 3β et 1H du CH₂ en 3γ) ; 1,30 (d, J = 7 Hz, 3H : CH₃ en 1γ) ; 1,44 (mt, 4H : CH₂ centraux de la dipropylamine); 1,57 (mt : 1H correspondant à l'autre H du CH₂ en 3γ); de 1,60 à 1,80 (2 mts: les 2H correspondant au CH₂ en 2β) ; 1,98 (mt, 1H : l'autre H du CH₂ en 3β) ; de 2,15 à 2,40 (mt, 4H : NCH₂ de la dipropylamine) ; 2,56 (dd large, J = 16 et 5,5 Hz, 1H : l'autre H du CH₂ en 5β) ; 2,78 (s, 6H : ArN(CH₃)₂) ; 2,84 et 2,98 (2 d, J = 13 Hz, 1H chacun : CH₂N) ; 3,02 (dd, J = 14 et 7 Hz, 1H : 1H du CH₂ en 4β) ; de 3,15 à 3,30 (mt, 2H : 1H du CH₂ en 3δ et l'autre H du CH₂ en 4β) ; 3,15 (s, 3H : NCH₃) ; 3,38 (d large, J = 18 Hz, 1H : 1H du CH₂ en 5ε) ; 3,47 (mt, 1H : l'autre H du CH₂ en 3δ) ; 4,55 (dd, J = 8 et 5,5 Hz, 1H : CH en 3α) ; de 4,70 à 4,85 (mt, 2H : CH en 2α et l'autre H du CH₂ en 5ε) ; 4,88 (dd, J = 10 et 1 Hz, 1H : CH en 1α) ; 5,14 (d large, J = 5 Hz, 1H : CH en 5α) ; 5,27 (dd, J = 7,5 et 7 Hz, 1H : CH en 4α) ; 5,49 (mt, 1H : CH en 5γ) ; 5,54 (d, J = 8 Hz, 1H : CH en 6α) ; 5,88 (q dédoublé, J = 7 et 1 Hz, 1H : CH en 1β) ; 6,57 (d, J = 9,5 Hz, 1H : CONH en 2) ; 6,86 (d, J = 8 Hz, 1H : H aromatique en 4ε) ; 6,89 (dd, J = 8 et 1,5 Hz, 1H : H aromatique en 4δ en para du Cl) ; 7,11 (d, J = 1,5 Hz, 1H : H aromatique en 4δ en ortho du Cl) ; de 7,20 à 7,40 (mt : les 7H correspondant aux H aromatiques en 6α - au 1' H₄ et au 1' H₅) ; 7,67 (mt, 1H : 1' H₆) ; 8,34 (d, J = 10 Hz, 1H : CONH en 1) ; 8,40 (d, J = 8 Hz, 1H : CONH en 6) ; 11,68 (mf, 1H : OH).

### Exemple 27

On opère comme à l'exemple 1, mais à partir d'un mélange brut contenant la 5δ-chlorométhyl-4ζ-méthylamino-4ζ-désdiméthylamino-5δ,5γ-déhydro pristinamycine I_{E}. On obtient 0,5 g de 5δ-morpholinométhyl-4ζ-méthylamino-4ζ-désdiméthylamino-5δ,5γ-déhydro pristinamycine I_{E}, sous forme d'un solide jaune fondant à 173°C.

Spectre de R.M.N. ¹H (400 MHz, CDCl₃) : 0,91 (t, J = 7,5 Hz, 3H : CH₃ en 2γ) ; de 1,15 à 1,40 (mt, 2H : 1H du CH₂ en 3β et 1H du CH₂ en 3γ) ; 1,16 (d très large, J = 16,5 Hz, 1H : 1H du CH₂ en 5β) ; 1,31 (d, J = 7 Hz, 3H : CH₃ en 1γ) ; 1,56 (mt, 1H : l'autre H du CH₂ en 3γ) ; de 1,60 à 1,80 (mt : les 2H correspondant au CH₂ en 2β) ; 2,00 (mt, 1H : l'autre H du CH₂ en 3β) ; 2,35 (mf, 4H : NCH₂ de la morpholine) ; 2,49 (dd large, J = 16,5 et 5 Hz, 1H : l'autre H du CH₂ en 5β) ; 2,84 et 2,86 (2 s, 5H en totalité : ArNCH₃ et CH₂N) ; 2,97 (dd, J = 14 et 6,5 Hz, 1H : 1H du CH₂ en 4β) ; de 3,10 à 3,20 (mt, 1H : l'autre H du CH₂ en 4β) ; 3,17 (s, 3H : NCH₃) ; 3,26 (mt, 1H : 1H du CH₂ en 3δ) ; 3,33 (d large, J = 18 Hz, 1H : 1H du CH₂ en 5ε) ; 3,47 (mt, 1H : l'autre H du CH₂ en 3δ) ; 3,70 (mt, 4H : CH₂O de la morpholine) ; 4,56 (dd, J = 8 et 5,5 Hz, 1H : CH en 3α) ; 4,77 (mt, 1H : CH en 2α) ; 4,82 (d large, J = 18 Hz, 1H : l'autre H du CH₂ en 5ε) ; 4,87 (dd, J= 10 et 1Hz, 1H : CH en 1α) ; 5,12 (d, J = 5 Hz, 1H : CH en 5α) ; 5,25 (dd, J = 9 et 6,5 Hz, 1H : CH en 4α) ; 5,52 (d, J = 8 Hz, 1H : CH en 6α) ; 5,53 (mt, 1H : CH en 5γ) ; 5,87 (q dédoublé, J = 7 et 1 Hz, 1H : CH en 1β) ; 6,45 (d, J = 8 Hz, 2H : H aromatiques en 4ε) ; 6,60 (d, J = 10 Hz, 1H : CONH en 2) ; 6,86 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,72 (dd, J = 4 et 1 Hz, 1H : 1' H₆) ; 8,42 (d, J = 10 Hz, 1H : CONH en 1) ; 8,50 (d, J = 8 Hz, 1H : CONH en 6) ; 11,68 (s, 1H : OH).

### Exemple 28

On opère comme à l'exemple 1 mais à partir d'un mélange brut contenant la 5δ-chlorométhyl-4ζ-méthylamino-4ζ-désdiméthylamino-4ε-chloro-5δ;5γ-déhydro pristinamycine I_{E}. On obtient 0,88 g de 5δ-morpholinométhyl-4ζ-méthylamino-4ζ-désdiméthylamino-5δ,5γ-déhydro-4ε-chloro pristinamycine I_{E}, sous forme d'un solide jaune fondant à 170°C.

Spectre de R.M.N. ¹H (400 MHz, CDCl₃) : 0,93 (t, J = 7,5 Hz, 3H : CH₃ en 2γ) ; de 1,15 à 1,40 (mt, 3H : 1H du CH₂ en 3β - 1H du CH₂ en 3γ et 1H du CH₂ en 5β) ; 1,30 (d, J = 7 Hz, 3H : CH₃ en 1γ) ; 1,57 (mt, 1H : l'autre H du CH₂ en 3γ) ; de 1,60 à 1,85 (mt : les 2H correspondant au CH₂ en 2β) ; 1,98 (mt, 1H : l'autre H du CH₂ en 3β) ; 2,35 (mf, 4H : NCH₂ de la morpholine) ; 2,59 (dd large, J = 16,5 et 5,5 Hz, 1H : l'autre H du CH₂ en 5β) ; de 2,85 à 3,05 (mt, 3H : CH₂N et 1H du CH₂ en 4β) ; 2,88 (d, J = 5 Hz, 3H : ArNCH₃) ; de 3,05 à 3,25 (mt, 1H : l'autre H du CH₂ en 4β) ; 3,14(s, 3H : NCH₃) ; 3,28 (mt, 1H : 1H du CH₂ en 3δ) ; 3,36 (d large, J = 18 Hz, 1H : 1H du CH, en 5ε) ; 3,47 (mt, 1H : l'autre H du CH₂ en 3δ) ; 3,68 (mt, 4H : CH₂O de la morpholine) ; 4,30 (q, J = 5 Hz, 1H : ArNH) ; 4,56 (dd, J = 8 et 5,5 Hz, 1H : CH en 3α) ; de 4,70 à 4,85 (mt, 2H : CH en 2α et l'autre H du CH₂ en 5ε) ; 4,87 (dd, J = 10 et 1Hz, 1H : CH en 1α) ; 5,18 (d, J = 5 Hz, 1H : CH en 5α) ; de 5,20 à 5,35 (mt, 1H : CH en 4α) ; 5,52 (d, J = 8 Hz, 1H : CH en 6α) ; 5,57 (mt, 1H : CH en 5γ) ; 5,88 (q dédoublé, J = 7 et 1 Hz, 1H : CH en 1β) ; 6,43 (d, J = 8 Hz, 1H : H aromatique en 4ε) ; 6,57 (d, J= 10 Hz, 1H : CONH en 2); 6,83 (dd, J= 8 et 1,5 Hz, 1H : H aromatique en 4δ en para du Cl) ; 6,95 (d, J = 1,5 Hz, 1H : H aromatique en 4δ en ortho du CI) ; de 7,20 à 7,40 (mt : les 7H correspondant aux H aromatiques en 6α - au 1' H₄ et au 1' H₅) ; 7,64 (dd, J = 4 et 1 Hz, 1H : 1' H₆) ; 8,35 (d, J = 10 Hz, 1H : CONH en 1) ; 8,51 (d, J = 8 Hz, 1H : CONH en 6) ; 11,68 (s, 1H : OH).

### Exemple 29

On opère comme à l'exemple 1, mais à partir d'un mélange brut contenant la 5δ-chlorométhyl-4ζ-(N-méthyl-N-allyloxycarbonyl)amino-4ζ-désdiméthylamino-4ε-chloro-5δ,5γ-déhydro pristinamycine I_{E}. On obtient 0,85 g de 5δ-morpholinométhyl-4ζ-(N-méthyl-N-allyloxycarbonyl)amino-4ζ-désdiméthylamino-5δ,5γ-déhydro pristinamycine I_{E}, sous forme d'un solide crème fondant à 154°C.

Spectre de R.M.N. ¹H (400 MHz, CDCl₃) : 0,92 (t, J = 7,5 Hz, 3H : CH₃ en 2γ) ; 1,13 (d très large, J = 16 Hz, 1H : 1H du CH₂ en 5β); de 1,15 à 1,40 (mt, 2H : 1H du CH₂ en 3β et 1H du CH₂ en 3γ) ; 1,31 (d, J = 7 Hz, 3H : CH₃ en 1γ) ; 1,55 (mt, 1H : l'autre H du CH₂ en 3γ) ; de 1,55 à 1,80 (mt : les 2H correspondant au CH₂ en 2β) ; 2,00 (mt, 1H : l'autre H du CH₂ en 3β) ; 2,35 (mf, 4H : NCH₂ de la morpholine) ; 2,55 (dd large, J = 16 et 5,5 Hz, 1H : l'autre H du CH₂ en 5β) ; 2,85 (s, 2H : CH₂N) ; 3,08 (dd, J = 14,5 et 7 Hz, 1H : 1H du CH₂ en 4β) ; 3,15 (s, 3H : NCH₃) ; de 3,20 à 3,35 (mt, 2H : l'autre H du CH₂ en 4β et 1H du CH₂ en 3δ) ; 3,29 (s, 3H : ArNCH₃) ; 3,35 (d large, J = 18 Hz, 1H : 1H du CH₂ en 5ε) ; 3,48 (mt, 1H : l'autre H du CH₂ en 3δ) ; 3,68 (mt, 4H : CH₂O de la morpholine) ; 4,56 (dd, J = 8,5 et 6 Hz, 1H : CH en 3α) ; 4,64 (d, J = 5,5 Hz, 2H : ArNCOOCH₂) ; 4,78 (mt, 1H : CH en 2α) ; 4,82 (d large, J = 18 Hz, 1H : l'autre H du CH, en 5ε) ; 4,85 (dd, J = 10 et 1Hz, 1H : CH en 1α) ; 5,12 (d, J = 5,5 Hz, 1H : CH en 5α) ; 5,19 et 5,27 (2 d larges, respectivement J = 11 Hz et J = 18 Hz, 1H chacun ; =CH₂) ; 5,31 (dd, J = 9 et 7 Hz, 1H : CH en 4α) ; de 5,50 à 5,60 (mt, 1H : CH en 5γ) ; 5,53 (d, J = 8 Hz, 1H : CH en 6α) ; de 5,80 à 6,00 (mt, 1H : CH=) ; 5,87 (q dédoublé, J = 7 et 1 Hz, 1H : CH en 1β) ; 6,56 (d, J = 9,5 Hz, 1H : CONH en 2) ; 7,03 (d, J = 8 Hz, 2H : H aromatiques en 4ε) ; 7,12 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,68 (dd, J = 4 et 1 Hz, 1H : 1' H₆) ; 8,37 (d, J = 10 Hz, 1H : CONH en 1) ; 8,44 (d, J = 8 Hz, 1H : CONH en 6) ; 11,67 (s, 1H : OH).

En opérant comme à l'exemple 1 on prépare la 5δ-chlorométhyl-4ζ-(N-méthyl-N-allyloxycarbonyl)amino-4ζ-désdiméthylamino-4ε-chloro-5δ,5γ-déhydro pristinamycine I_{E}.

### Exemple 30

On opère comme à l'exemple 1 mais à partir de 150 cm³ d'acétonitrile, de 15 g d'un mélange brut contenant 33 % molaire de 5δ-chlorométhyl-5δ,5γ-déhydro pristinamycine I_{E} et de 2,5 cm³ de tétrahydroisoquinoléine. Le mélange réactionnel est chauffé à reflux 1 heure puis est concentré à sec. Le solide obtenu est repris par 3 fois un mélange dichlorométhane/eau saturée en bicarbonate de sodium. Après décantation, la phase organique est concentrée à sec (45°C-2,7 kPa) pour donner 15,4 g de solide. Celui-ci est purifié par chromatographie sur silice (éluant : gradiant dichlorométhane/méthanol 99,5/0,5 à 98,5/1,5 en volumes) puis par CLHP préparative sur 450 g de silice Kromasil® C8 10 µm 100Å, avec comme éluant un mélange eau-acétonitrile (60/40 en volumes contenant 0,1 % d'acide trifluoroacétique). Après concentration des fractions pour éliminer l'acétonitrile, la phase aqueuse est amenée à pH 7-8 par addition d'une solution saturée de bicarbonate de sodium. Le précipité apparu est filtré, lavé par 25 cm³ d'eau et agité une nuit dans une solution saturée en bicarbonate de sodium. Le solide ainsi obtenu est filtré, lavé par 20 cm³ d'eau puis séché à 40°C sous pression réduite (90 Pa) pour donner 0,73 g de 5δ-(tétrahydroisoquinolyl)méthyl-5δ,5γ-déhydro pristinamycine I_{E}, sous forme de cristaux blancs, fondant à 212°-214°C.

Spectre de R.M.N. ¹H (400 MHz, CDCl₃) : 0,92 (t, J = 7,5 Hz, 3H : CH, en 2γ) ; 1,12 (d très large, J = 16,5 Hz, 1H : 1H du CH₂ en 5β) ; 1,23 (mt, 2H : 1H du CH₂ en 3β et 1H du CH₂ en 3γ) ; 1,33 (d, J = 7 Hz, 3H : CH₃ en 1γ) ; 1,54 (mt, 1H : l'autre H du CH₂ en 3γ) ; de 1,60 à 1,85 (mt : les 2H correspondant au CH₂ en 2β) ; 1,96 (mt, 1H : l'autre H du CH₂ en 3β) ; 2,51 (d large, J = 16,5 et 5,5 Hz, 1H : l'autre H du CH₂ en 5β) ; 2,66 (mt, 2H : ArCH₂) ; de 2,80 à 3,05 (mt, 3H : 1H du CH₂ en 4β et NCH₂) ; 2,94 (s, 6H : ArN(CH₃)₂) ; 3,03 (AB, J = 13 Hz, 2H : CH₂N) ; de 3,10 à 3,35 (mt, 2H : l'autre H du CH₂ en 4β et 1H du CH₂ en 3δ) ; 3,16 (s, 3H : NCH₃) ; 3,39 (d large, J = 18 Hz, 1H : 1H du CH₂ en 5ε) ; 3,46 (mt, 1H : l'autre H du CH₂ en 3δ) ; 3,57 (s, 2H : ArCH₂N) ; 4,57 (dd, J = 8 et 6 Hz, 1H : CH en 3α) ; 4,77 (mt, 1H : CH en 2α) ; 4,84 (d large, J = 18 Hz, 1H : l'autre H du CH₂ en 5ε) ; 4,87 (dd, J = 10 et 1 Hz, 1H : CH en 1α) ; 5,13 (d large, J = 5 Hz, 1H : CH en 5α) ; 5,23 (dd, J = 9 et 7 Hz, 1H : CH en 4α) ; 5,51 (d, J = 8 Hz, 1H : CH en 6α) ; 5,58 (mt, 1H : CH en 5γ) ; 5,88 (q dédoublé, J = 7 et 1 Hz, 1H : CH en 1β) ; 6,58 (d, J = 9,5 Hz, 1H : CONH en 2) ; 6,62 (d, J = 8 Hz, 2H : H aromatiques en 4ε) ; 6,93 (d, J = 8 Hz, 2H : H aromatiques en 4δ) ; de 7,00 à 7,40 (mt : les 11H correspondant aux H aromatiques en 6α - aux 4H aromatiques de la 3,4-dihydro-1H-isoquinoline- au 1' H₄ et au 1' H₅) ; 7,73 (dd, J = 4 et 1 Hz, 1H : 1' H₆) ; de 8,35 à 8,45 (mt, 2H : CONH en 1 et CONH en 6) ; 11,68 (s, 1H : OH).

### Exemple 31

On opère comme à l'exemple 1 mais à partir de 150 cm³ d'acétonitrile, de 15 g d'un mélange brut contenant 33 % molaire de 5δ-chlorométhyl-5δ,5γ-déhydro pristinamycine I_{E} et de 3,6 g de 4-fluorophénylpipérazine. Le milieu réactionnel est chauffé 4 heures à reflux puis concentré à sec. Le solide obtenu est repris par 250 cm³ d'acétate d'éthyle. La solution résultante est lavée par 3 fois 150 cm³ d'eau et 3 fois 150 cm³ d'acide chlorhydrique. Les phases aqueuses sont rassemblées puis amenées à pH 7-8 par addition d'une solution saturée en bicarbonate de sodium. Le précipité apparu est filtré, lavé par 50 cm³ d'eau puis séché pour donner 11,2 g de solide. Le solide obtenu est purifié par chromatographie sur silice (éluant : dichlorométhane-méthanol 99/1 en volumes) puis par deux CLHP préparatives sur 450 g de silice Kromasil C8 10 µm 100Å, avec comme éluant un mélange eau-acétonitrile (60/40 en volumes contenant 0,1 % d'acide trifluoroacétique). Après concentration des fractions pour éliminer l'acétonitrile, la phase aqueuse résiduelle est amenée à pH 7-8 par addition d'une solution saturée de bicarbonate de sodium. Le précipité apparu est filtré pour donner 0,7 g de solide qui est agité dans 20 cm³ d'éther, filtré puis séché à 40°C sous pression réduite (90 Pa). On obtient ainsi 285 mg de 5δ-[4-(4-fluorophény) pipérazin-1-yl]méthyl-5δ,5γ-déhydro pristinamycine I_{E}, sous forme de cristaux blanc cassé fondant à 165-167°C.

Spectre de R.M.N. ¹H (400 MHz, CDCl₃): 0,91 (t, J = 7,5 Hz, 3H : CH₃ en 2γ) ; 1,03 (d très large, J = 16 Hz, 1H : 1H du CH₂ en 5β) ; 1,21 (mt, 2H : 1H du CH₂ en 3β et 1H du CH₂ en 3γ); 1,32 (d, J = 7 Hz, 3H : CH₃ en 1γ) ; 1,53 (mt, 1H : l'autre H du CH₂ en 3γ) ; de 1,60 à 1,80 (mt : les 2H correspondant au CH₂ en 2β) ; 1,98 (mt, 1H : l'autre H du CH₂ en 3β) ; 2,46 (mt, 1H : l'autre H du CH₂ en 5β) ; de 2,55 à 3,35 (mt, 10H : CH₂N de la pipérazine et CH₂N) ; 2,95 (s, 6H : ArN(CH₃)₂) ; 2,98 (mt, 1H : 1H du CH₂ en 4β) ; de 3,10 à 3,35 (mt, 2H : 1H du CH₂ en 3δ et l'autre H du CH₂ en 4β) ; 3,18 (s, 3H : NCH₃) ; de 3,35 à 3,50 (mt, 2H : 1H du CH₂ en 5ε et l'autre H du CH₂ en 3δ) ; 4,56 (dd, J = 8 et 6 Hz, 1H : CH en 3α) ; 4,77 (mt, 1H : CH en 2α) ; 4,83 (mt, 1H : l'autre H du CH₂ en 5ε) ; 4,88 (d large, J = 10 Hz, 1H : CH en 1α) ; 5,14 (d large, J = 5 Hz, 1H : CH en 5α) ; 5,20 (dd, J = 9 et 6 Hz, 1H : CH en 4α) ; 5,54 (d, J = 8 Hz, 1H : CH en 6α) ; 5,65 (mf, 1H : CH en 5γ) ; 5,88 (q large, J = 7 Hz, 1H : CH en 1β) ; 6,56 (d, J = 9,5 Hz, 1H : CONH en 2) ; 6,60 (d, J = 8 Hz, 2H : H aromatiques en 4ε) ; de 6,80 à 7,05 (mt, 6H : H aromatiques en 4δ et H aromatiques du fluoro phényle) ; de 7,20 à 7,40 (mt : les 7H correspondant aux H aromatiques en 6α - au 1' H₄ et au 1' H₅) ; 7,73 (d large, J = 4 Hz, 1H : 1' H₆) ; 8,38 (d, J = 10 Hz, 1H : CONH en 1) ; 8,57 (mf, 1H : CONH en 6) ; 11,66 (s, 1H : OH).

### Exemple 32

On opère comme à l'exemple 1, mais à partir d'un mélange brut contenant la 5δ-chlorométhyl-4ζ-méthylamino-4ζ-désdiméthylamino-5δ,5γ-déhydro pristinamycine I_{E} pour donner 0,61 g de 5δ-pipéridinométhyl-4ζ-méthylamino-4ζ-désdiméthylamino-5δ,5γ-déhydro pristinamycine I_{E}, sous forme de cristaux blancs fondant à 234°C.

Spectre de R.M.N. ¹H (600 MHz, CDCl₃, δ en ppm). Nous observons la présence de traces infimes d'autres pristinamycines non identifiées et de conformères.

0,91 (t, J = 7,5 Hz, 3H : CH₃ en 2γ) ; 1,16 (d très large, J = 17,5 Hz, 1H : 1H du CH₂ en 5β) ; 1,23 (mt, 2H : 1H du CH₂ en 3β et 1H du CH₂ en 3γ) ; 1.31 (d, J = 7 Hz, 3H : CH₃ en 1γ) ; de 1,35 à 1,65 (mt, 7H : l'autre H du CH₂ en 3γ et CH₂ de la pipéridine) ; de 1,60 à 1,80 (mt : les 2H correspondant au CH₂ en 2β) ; 1,97 (mt, 1H : l'autre H du CH₂ en 3β) ; de 2,20 à 2,40 (mf, 4H : NCH₂ de la pipéridine) ; 2,49 (dd large, J = 17,5 et 5,5 Hz, 1H : l'autre H du CH₂ en 5β) ; 2,78 (s, 2H : CH₂N) ; 2,82 (s, 3H : ArNCH₃) ; 2,95 (dd, J = 14 et 7 Hz, 1H : 1H du CH₂ en 4β) ; de 3,10 à 3,30 (mt, 2H : l'autre H du CH₂ en 4β et 1H du CH₂ en 3δ) ; 3,15 (s, 3H : NCH₃) ; 3,35 (d large, J = 18 Hz, 1H : 1H du CH₂ en 5ε) ; 3,45 (mt, 1H : l'autre H du CH₂ en 3δ) ; de 3,60 à 3,80 (mf étalé, 1H : ArNH) ; 4,55 (dd, J = 8 et 5,5 Hz, 1H : CH en 3α) ; de 4,70 à 4,90 (mt, 2H : CH en 2α et l'autre H du CH₂ en 5ε) ; 4,86 (d large, J = 10 Hz, 1H : CH en 1α) ; 5,10 (d, J = 5,5 Hz, 1H : CH en 5α) ; 5,22 (dd, J = 10 et 7 Hz, 1H : CH en 4α) ; 5,48 (mt, 1H : CH en 5γ) ; 5,52 (d, J = 8 Hz, 1H : CH en 6α) ; 5,85 (q large, J = 7 Hz, 1H : CH en 1β) ; 6,45 (d, J = 8 Hz, 2H : H aromatiques en 4ε) ; 6,58 (d, J = 9,5 Hz, 1H : CONH en 2) ; 6,85 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,71 (d large, J = 4 Hz, 1H : 1' H₆) ; 8,41 (mt, 2H : CONH en 1 et CONH en 6) ; 11,67 (s, 1H : OH).

### Exemple 33

On opère comme à l'exemple 1, mais à partir de 100 cm³ d'acétonitrile, de 30 g d'un mélange brut contenant 33% molaire de 5δ-chlorométhyl-5δ,5γ-déhydro pristinamycine I_{E} et de 2,9 cm³ de 2-méthoxyéthylamine. Le mélange réactionnel est chauffé à reflux durant 2 heures puis est concentré à sec sous pression réduite, à 45°C (2,7 kPa). Le solide obtenu est repris par 50 cm³ de dichlorométhane et 3 fois 50 cm³ d'acide chlorhydrique 0,1 N. La solution aqueuse est ajustée à pH 7-8 par addition d'une solution aqueuse saturée de bicarbonate de sodium. et extraite par 2 fois 50 cm³ de dichlorométhane. La phase organique est décantée, séchée sur sulfate de sodium, filtrée et concentrée à sec à 45°C sous pression réduite (2,7 kPa). On obtient ainsi 8,5 g de solide qui est chromatographié sur silice (éluant : gradient dichloroinéthane/méthanol 98/3 puis 97/3 en volumes). On obtient un solide qui est agité dans 25 cm³ d'éther diéthylique, filtré, lavé par 10 cm³ d'éther diéthylique, puis séché sous pression réduite à 45°C (90 Pa), pour donner 0,77 g de 5δ-(2-méthoxyéthylaminométhyl)-5δ,5γ-déhydro pristinamycine I_{E}, sous forme d'un solide crème fondant à 200°C.

Spectre de R.M.N. ¹H (400 MHz, CDCl₃, δ en ppm). Nous observons la présence de traces infimes d'autres pristinamycines non identifiées et de conformères.

0,92 (t, J = 7,5 Hz, 3H : CH₃ en 2γ) ; 1,20 (d très large, J = 17 Hz, 1H : 1H du CH₂ en 5β) ; 1,25 (mt, 2H : 1H du CH₂ en 3β et 1H du CH₂ en 3γ) ; 1,31 (d, J = 7 Hz, 3H : CH₃ en 1γ) ; 1,55 (mt, 1H : l'autre H du CH₂ en 3γ) ; de 1,60 à 1,80 (mt : les 2H correspondant au CH₂ en 2β) ; 1,98 (mt, 1H : l'autre H du CH₂ en 3β) ; 2,51 (dd large, J = 17 et 5,5 Hz, 1H : l'autre H du CH₂ en 5β) ; 2,71 (mt, 2H : NCH₂) ; de 2,85 à 2,95 (mt, 1H : NH) ; 2,93 (s, 6H : ArN(CH₃)₂) ; 2,99 (dd, J = 14 et 7 Hz, 1H : 1H du CH₂ en 4β) ; de 3,10 à 3,30 (mt, 4H : l'autre H du CH₂ en 4β - 1H du CH₂ en 3δ et CH₂N) ; 3,13 (s, 3H : NCH₃) ; de 3,30 à 3,40 (mt, 1H : 1H du CH₂ en 5ε) ; 3,33 (s, 3H : OCH₃) ; de 3,40 à 3,55 (mt, 1H : l'autre H du CH₂ en 3δ) ; 3,48 (t, J = 5,5 Hz, 2H : OCH₂) ; 4,57 (dd, J = 8,5 et 5 Hz, 1H : CH en 3α) ; de 4,70 à 4,85 (mt, 2H : CH en 2α et l'autre H du CH₂ en 5ε) ; 4,87 (d large, J = 10 Hz, 1H : CH en 1α) ; 5,12 (d, J = 5,5 Hz, 1H : CH en 5α) ; 5,25 (dd, J = 8 et 7 Hz, 1H : CH en 4α) ; 5,53 (mt, 1H : CH en 5γ) ; 5,56 (d, J = 8 Hz, 1H : CH en 6α) ; 5,86 (q large, J = 7 Hz, 1H : CH en 1β) ; de 6,50 à 6,65 (mt, 1H : CONH en 2) ; 6,57 (d, J = 8 Hz, 2H : H aromatiques en 4ε) ; 6,89 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,69 (d large, J = 4 Hz, 1H : 1' H₆) ; 8,39 (d, J = 10 Hz, 1H : CONH en 1) ; 8,54 (d, J = 8 Hz, 1H : CONH en 6); 11,65 (mf étalé, 1H : OH).

### Exemple 34

On opère comme à l'exemple 1, mais à partir de 100 cm³ d'acétonitrile, de 10 g d'un mélange brut contenant 33 % molaire de 5δ-chlorométhyl-5δ,5γ-déhydro pristinamycine I_{E} et de 2,27 g de bis-(2-méthoxyéthyl)amine. Le mélange réactionnel est chauffé à reflux durant 18 heures puis concentré à sec sous pression réduite à 45°C (2,7 kPa) pour donner un solide qui est repris par 200 cm³ de dichlorométhane et 100 cm³ d'une solution aqueuse. Le pH de la phase aqueuse est ajustée à pH 7-8 par addition de bicarbonate de sodium. La phase organique est décantée, séchée sur sulfate de sodium, filtrée et concentrée à sec à 45°C sous pression réduite (2,7 kPa) pour donner 9,4 g de meringue jaune qui est chromatographiée sur silice (éluant : dichlorométhane/méthanol 97/3 en volumes). Les fractions contenant l'attendu sont concentrées à sec. Le solide ainsi obtenu est repris par 200 cm³ de chlorure de méthylène. La solution obtenue est extraite par 3 fois 150 cm³ d'une solution d'acide chlorhydrique 0,1N. La phase aqueuse est reprise par une solution aqueuse saturée en bicarbonate de sodium puis extraite par 3 fois 150 cm³ de chlorure de méthylène. Les phases organiques sont rassemblées puis concentrées à sec pour donner 2 g de produit qui est recristallisé dans 30 cm³ de méthanol. Le solide ainsi obtenu est filtré, lavé par 2 fois 5 cm³ de méthanol puis séché sous pression réduite à 45°C (90 Pa) pour donner 1,38 g de 5δ-[bis-(2-méthoxyéthyl)aminométhyl)-5δ,5γ-déhydro pristinamycine I_{E}, sous forme de cristaux blancs fondant à 180-182°C.

Spectre de R.M.N. ¹H (400 MHz, CDCl₃, δ en ppm). Nous observons la présence de traces infimes d'autres pristinamycines non identifiées et de conformères.

0,91 (t, J = 7,5 Hz, 3H : CH₃ en 2γ) ; 1,05 (d très large, J = 17 Hz, 1H : 1H du CH₂ en 5β) ; 1,24 (mt, 2H : 1H du CH₂ en 3β et 1H du CH₂ en 3γ) ; 1,31 (d, J = 7 Hz, 3H : CH₃ en 1γ) ; de 1,45 à 1,65 (mt : 1H correspondant à l'autre H du CH₂ en 3γ) ; 1,66 et 1,73 (2 mts, 1H chacun : CH₂ en 2β) ; 1,99 (mt, 1H : l'autre H du CH₂ en 3β); 2,46 (d large, J = 17 Hz, 1H : l'autre H du CH₂ en 5β) ; 2,68 (mt, 4H : NCH₂) ; de 2,90 à 3,05 (mt, 1H : 1H du CH₂ en 4β) ; 2,94 (s, 6H : ArN(CH₃)₂) ; 3,05 (s large, 2H : CH₂N) ; de 3,10 à 3,30 (mt, 2H : l'autre H du CH₂ en 4β et 1H du CH₂ en 3δ) ; 3,18 (s, 3H : NCH₃) ; de 3,25 à 3,40 (mt, 1H : 1H du CH₂ en 5ε) ; 3,33 (s, 6H : OCH₃) ; de 3,40 à 3,55 (mt, 1H : l'autre H du CH₂ en 3δ) ; 3,48 (t, J = 6 Hz, 4H : OCH₂) ; 4,58 (mt, 1H : CH en 3α) ; 4,78 (mt, 1H : CH en 2α) ; de 4,80 à 4,95 (mt, 1H : l'autre H du CH₂ en 5ε) ; 4,87 (d large, J = 10 Hz, 1H : CH en 1α) ; 5,12 (d, J = 5 Hz, 1H : CH en 5α) ; 5,22 (mt, 1H : CH en 4α) ; 5,48 (mt, 1H : CH en 5γ) ; 5,57 (d, J = 8 Hz, 1H : CH en 6α) ; 5,86 (q large, J = 7 Hz, 1H : CH en 1β) ; 6,55 (d, J = 9,5 Hz, 1H : CONH en 2) ; 6,62 (d, J = 8 Hz, 2H : H aromatiques en 4ε) ; 6,92 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,70 (d large, J = 4 Hz, 1H : 1' H₆) ; 8,37 (d, J = 10 Hz, 1H : CONH en 1) ; 8,45 (d, J = 8 Hz, 1H : CONH en 6) ; 11,66 (s, 1H : OH).

### Exemple 35

Dans un tricol contenant 50 cm³ d'acétonitrile, on introduit 0,14 g de 2-mercapto-1-méthyl imidazole puis 62 mg d'hydrure de sodium, 0,33 cm³ de triéthylamine et 2,5 g d'un mélange brut contenant 33 % molaire de 5δ-chlorométhyl-5δ,5γ-déhydro pristinamycine I_{E}. Le mélange réactionnel est chauffé à 40°C pendant 48 heures. Après addition de 2 cm³ d'eau, le mélange réactionnel est concentré à sec sous pression réduite (45°C-2,7 kPa). Le résidu est repris par 25 cm³ de dichlorométhane et le mélange obtenu lavé par 2 fois 20 cm³ d'eau. La phase organique est décantée puis extraite par 3 fois 20 cm³ d'acide chlorhydrique 0,1N. Après décantation, la phase aqueuse est amené à pH 7 par addition d'une solution aqueuse saturée en bicarbonate de sodium. La phase organique est extraite par 2 fois 25 cm³ de dichlorométhane, séchée sur sulfate de sodium, filtrée puis concentrée à sec. Le résidu est repris par 20 cm³ d'éther diisopropylique, filtré et séché à 40°C sous 90 Pa pour donner 0,6 g de 5δ-(1-méthyl-2-imidazolyl thiométhyl)-5δ,5γ-déhydro pristinamycine I_{E}, sous forme d'un solide crème fondant à 147°C.

Spectre de R.M.N. ¹H (400 MHz, CDCl₃, δ en ppm). Nous observons la présence de traces infimes d'autres pristinamycines non identifiées et de conformères.

0,91 (t, J = 7,5 Hz, 3H : CH₃ en 2γ) : de 1,15 à 1,35 (mt, 3H : 1H du CH₂ en 5β -1H du CH₂ en 3β et 1H du CH₂ en 3γ) ; 1,29 (d, J = 7 Hz, 3H : CH₃ en 1γ) ; 1,56 (mt, 1H : l'autre H du CH₂ en 3γ) ; 1,64 et 1,72 (2 mts, 1H chacun : CH₂ en 2β); 1,98 (mt : 1H correspondant à l'autre H du CH₂ en 3β) ; 2,38 (dd large, J = 17 et 5 Hz, 1H : l'autre H du CH₂ en 5β) ; de 2,90 à 3,00 (mt, 1H : 1H du CH₂ en 4β) ; 2,92 (s, 6H : ArN(CH₃)₂) ; de 3,10 à 3,30 (mt, 2H : l'autre H du CH₂ en 4β - 1H du CH₂ en 3δ) ; 3,18 (s, 3H : NCH₃) ; 3,30 (d large, J = 18 Hz, 1H : 1H du CH₂ en 5ε) ; 3,47 (mt, 1H : l'autre H du CH₂ en 3δ); 3,55 (d, J = 14 Hz, 1H : 1H du SCH₂) ; 3,65 (s, 3H : NCH₃) ; 3,81 (d, J = 14 Hz, 1H : l'autre H du SCH₂) ; 4,56 (dd, J = 8 et 7 Hz, 1H : CH en 3α) ; 4,76 (mt, 1H : CH en 2α) ; 4,87 (d large, J = 10 Hz, 1H : CH en 1α) ; 5,00 (d large, J = 18 Hz, 1H : l'autre H du CH₂ en 5ε) ; 5,05 (d, J = 5,5 Hz, 1H : CH en 5α) ; 5,17 (dd, J = 10 et 6 Hz, 1H : CH en 4α) ; 5,50 (mt, 1H : CH en 5γ) ; 5,58 (d, J = 8 Hz, 1H : CH en 6α) ; 5,87 (q large, J = 7 Hz, 1H : CH en 1β) ; 6,53 (d, J = 8 Hz, 2H : H aromatiques en 4ε) ; 6,57 (d, J = 9 Hz, 1H : CONH en 2) ; 6,87 (d, J = 8 Hz, 2H : H aromatiques en 4δ) ; 6,93 et 7,10 (2 s larges, 1H chacun : CH=CH de l'imidazole) ; de 7,20 à 7,40 (mt : les 7H correspondant aux H aromatiques en 6α - au 1' H₄ et au 1' H₅) ; 7,68 (d large, J = 4 Hz, 1H : 1' H₆) ; 8,39 (d, J = 10 Hz, 1H : CONH en 1) ; 8,45 (d, J = 8 Hz, 1H : CONH en 6) ; 11,65 (mf étalé, 1H : OH).

### Exemple 36

Dans un tricol contenant 25 cm³ d'acétonitrile, on introduit successivement 0,57 cm³ de 2-(diéthylamino)éthanethiol, 18 mg d'hydrure de sodium et 0,54 cm³ de triéthylamine. A la solution résultante, on ajoute une solution de 50 cm³ d'acétonitrile et de 7,5 g d'un mélange brut contenant 40 % molaire de 5δ-chlorométhyl-5δ,5γ-déhydro pristinamycine I_{E} (obtenu comme à l'exemple 1), prélablement ajustée à pH 7 par addition de triéthylamine. Le mélange réactionnel est chauffé à 45°C pendant 48 heures. On ajoute alors 2 cm³ d'eau et le mélange résultant est concentré à sec sous pression réduite (45°C-2,7 kPa). Le résidu est repris par 50 cm³ de dichlorométhane et la solution obtenue est lavée par 2 fois 25 cm³ d'eau. La phase organique est décantée, séchée sur sulfate de sodium, filtrée et concentrée à sec pour donner 6 g d'un produit brut qui est chromatographié sur silice (éluant : gradient dichlorométhane/méthanol 98/2 à 95/5 en volumes). Les fractions contenant le produit attendu sont concentrées à sec et le résidu repris par 20 cm³ de dichlorométhane. La solution obtenue est filtrée puis concentrée à sec (45°C-2,7 kPa). Le résidu est repris par 25 cm³ d'éther diisopropylique, filtré, séché à 40°C sous 90 Pa pour donner 1 g de 5δ-diéthylaminoéthylthiométhyl-5δ,5γ-déhydro pristinamycine I_{E}, sous forme d'hémichlorhydrate jaune pâle, fondant à 135°C.

Spectre de R.M.N. ¹H (600 MHz, CDCl₃, δ en ppm).
0,92 (t, J = 7,5 Hz, 3H : CH₃ en 2γ) ; 1,10 (mt, 1H : 1H du CH₂ en 5β) ; 1,17 (mf, 6H : CH₃ éthyle) ; de 1,15 à 1,35 (mt, 2H : 1H du CH₂ en 3β et 1H du CH₂ en 3γ) ; 1,31 (d, J = 7 Hz, 3H : CH₃ en 1γ) ; 1,57 (mt, 1H : l'autre H du CH₂ en 3γ) ; 1,66 et 1,73 (2 mts, 1H chacun : CH₂ en 2β) ; 2,01 (mt, 1H : l'autre H du CH₂ en 3β) ; 2,47 (dd large, J = 17 et 5,5 Hz, 1H : l'autre H du CH₂ en 5β) ; de 2,55 à 2,95 (mt, 6H : NCH₂) ; 2,94 (s, 6H : ArN(CH₃)₂) ; 3,00 (dd, J = 14 et 6,5 Hz, 1H : 1H du CH₂ en 4β) ; 3,09 (d, J = 15 Hz, 1H : 1H du CH₂S) ; de 3,10 à 3,30 (mt, 3H : l'autre H du CH₂ en 4β - 1H du CH₂ en 3δ et l'autre H du CH₂S) ; 3,18 (s, 3H : NCH₃) ; 3,46 (mt, 1H : 1H du CH₂ en 3δ) ; 3,53 (d large, J = 18 Hz, 1H : l'autre H du CH₂ en 5ε) ; 4,58 (dd, J = 8 et 6 Hz, 1H : CH en 3α) ; de 4,70 à 4,85 (mt, 2H : CH en 2α et l'autre H du CH₂ en 5ε) ; 4,87 (d large, J = 10 Hz, 1H : CH en 1α) ; 5,11 (d, J = 5 Hz, 1H : CH en 5α) ; 5,22 (dd, J = 10 et 6,5 Hz, 1H : CH en 4α) ; 5,47 (d, J = 8 Hz, 1H : CH en 6α) ; 5,56 (mt, 1H : CH en 5γ) ; 5,86 (q large, J = 7 Hz, 1H: CH en 1β) ; de 6,55 à 6,65 (mt, 1H : CONH en 2) ; 6,60 (d, J = 8 Hz, 2H : H aromatiques en 4ε) ; 6,93 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,72 (d large, J = 4 Hz, 1H : 1' H₆) ; 8,40 (d, J = 10 Hz, 1H : CONH en 1) ; 8,54 (d, J = 8 Hz, 1H : CONH en 6) ; 11,67 (mf, 1H : OH).

### Exemple 37

On opère comme à l'exemple 36 mais à partir de 25 cm³ d'acétonitrile, 0,32 cm³ de (4-pyridyl)méthanethiol, 120 mg d'hydrure de sodium, d'une part et 5 g d'un mélange brut contenant 40 % molaire de 5δ-chlorométhyl-5δ,5γ-déhydro pristinamycine I_{E} et de la triéthylamine en solution dans 40 cm³ d'acétonitrile, d'autre part. Le mélange réactionnel est chauffé à 45°C pendant 1,5 heure puis traité comme à l'exemple 36 pour donner 5 g de produit brut qui est chromatographié par chromatographie flash sur silice 0,04-0,063 mm (éluant : gradient dichlorométhane/méthanol 99/1 à 98/2 en volumes). Les fractions contenant le produit attendu sont concentrées à sec pour donner 1,2 g d'une meringue jaune qui est délitée dans 25 cm³ d'éther diisopropylique pendant 1 heure sous agitation. Le solide obtenu est filtré, lavé par 10 cm³ d'éther diisopropylique puis séché à 45°C sous 90 Pa. On obtient ainsi 0,85 g de 5δ-(4-pyridylméthyl)thiométhyl-5δ,5γ-déhydro pristinamycine I_{E}, sous forme de solide jaune pâle, fondant à 138°C.

Le 4-pyridylméthanethiol peut être préparé selon J. BARNES et Coll., Eur. J. Med. Chem., 23, 211-16, (1988)

Spectre de R.M.N. ¹H (400 MHz, CDCl₃, δ en ppm).
0,92 (t, J = 7,5 Hz, 3H : CH₃ en 2γ) ; 1,08 (d très large, J = 17 Hz, 1H : 1H du CH₂ en 5β) ; de 1,20 à 1,35 (mt, 2H : 1H du CH₂ en 3β et 1H du CH₂ en 3γ) ; 1,33 (d, J = 7 Hz, 3H : CH₃ en 1γ) ; 1,58 (mt, 1H : l'autre H du CH₂ en 3γ) ; 1,67 et 1,74 (2 mts, 1H chacun : CH₂ en 2β) ; 2,01 (mt, 1H : l'autre H du CH₂ en 3β); 2,53 (dd large, J = 17 et 5 Hz, 1H : l'autre H du CH₂ en 5β) ; de 2,85 à 3,00 (mt, 2H : CH₂S) ; 2,95 (s, 6H : ArN(CH₃)₂) ; 3,00 (dd, J = 14 et 6,5 Hz, 1H : 1H du CH₂ en 4β) ; de 3,10 à 3,25 (mt, 1H : l'autre H du CH₂ en 4β) ; 3,19 (s, 3H : NCH₃) ; 3,32 (mt, 1H : 1H du CH₂ en 3δ) ; de 3,45 à 3,60 (mt, 1H : 1H du CH₂ en 5ε) ; 3,50 (mt, 1H : l'autre H du CH₂ en 3δ) ; 3,54 et 3,62 (2 d, J = 14 Hz, 1H chacun : SCH₂Ar) ; 4,60 (dd, J = 8 et 6 Hz, 1H : CH en 3α) ; de 4,70 à 4,85 (mt, 2H : CH en 2α et l'autre H du CH₂ en 5ε) ; 4,90 (d large, J = 10 Hz, 1H : CH en 1α) ; 5,13 (d large, J = 5 Hz, 1H : CH en 5α) ; 5,26 (dd, J = 9,5 et 6,5 Hz, 1H : CH en 4α) ; 5,39 (d large, J = 4 Hz, 1H : CH en 5γ) ; 5,60 (d, J = 8 Hz, 1H : CH en 6α) ; 5,89 (q large, J = 7 Hz, 1H : CH en 1β) ; de 6,50 à 6,65 (mt, 1H : CONH en 2) ; 6,60 (d, J = 8 Hz, 2H : H aromatiques en 4ε) ; 6,93 (d, J = 8 Hz, 2H : H aromatiques en 4δ) ; de 7,20 à 7,40 (mt : les 9H correspondant aux H aromatiques en 6α - au 1' H₄ - au 1' H₅ et aux H aromatiques en β de la pyridine) ; 7,74 (d large, J = 4 Hz, 1H : 1' H₆) ; 8,40 (d, J = 10 Hz, 1H : CONH en 1) ; de 8,45 à 8,60 (mt, 3H : CONH en 6 et H aromatiques en α de la pyridine) ; 11,68 (s, 1H : OH).

### Exemple 38

On opère comme à l'exemple 36, mais à partir de 25 cm³ d'acétonitrile, 0,84 g de (3-pyridyl)méthanethiol, 0,24 g d'hydrure de sodium, d'une part et 10 g d'un mélange brut contenant 40 % molaire de 5δ-chlorométhyl-5δ,5γ-déhydro pristinamycine I_{E} et de la triéthylamine dans 75 cm³ d'acétonitrile, d'autre part. Le mélange réactionnel est chauffé à 60°C pendant 2 heures puis traité comme à l'exemple 36 pour donner 8 g de produit brut qui est chromatographié sur silice (éluant: gradient dichlorométhane/méthanol 99/1 à 98/2 en volumes). On obtient ainsi 2,1 g de produit qui est repurifié par chromatographie flash sur silice 0,040-0,063 mm (éluant : gradient dichlorométhane/méthanol 99/1 à 97/3). Les fractions contenant le produit attendu sont concentrées et le résidu obtenu est séché à 45°C sous 90 Pa pour donner 0,19 g de 5δ-3-pyridylméthylthiométhyl-5δ,5γ-déhydro pristinamycine I_{E}, sous forme de solide jaune fondant à 128°C.

Le (3-pyridyl)méthanethiol peut être préparé selon T. BROWN et Coll., J. Med. Chem., 35, 3613-24, (1992).

Spectre de R.M.N. ¹H (400 MHz, CDCl₃, δ en ppm).
0,92 (t, J = 7,5 Hz, 3H : CH₃ en 2γ) ; 1,08 (d très large, J = 17 Hz, 1H : 1H du CH₂ en 5β) ; 1,27 (mt, 2H : 1H du CH₂ en 3β et 1H du CH₂ en 3γ) ; 1,33 (d, J = 7 Hz, 3H : CH₃ en 1γ) ; 1,58 (mt, 1H : l'autre H du CH₂ en 3γ) ; de 1,60 à 1,80 (mt : les 2H correspondant au CH₂ en 2β) ; 2,01 (mt, 1H : l'autre H du CH₂ en 3β) ; 2,55 (dd, J = 17 et 5,5 Hz, 1H : l'autre H du CH₂ en 5β) ; 2,93 (s, 6H : ArN(CH₃)₂) ; de 2,85 à 3,05 (mt, 3H : 1H du CH₂ en 4β et SCH₂) ; de 3,15 à 3,25 (mt, 1H : l'autre H du CH₂ en 4β) ; 3,18 (s, 3H : NCH₃) ; 3,31 (mt, 1H : 1H du CH₂ en 3δ) ; de 3,45 à 3,60 (mt, 2H : 1H du CH₂ en 5ε et l'autre H du CH₂ en 3δ) ; 3,57 et 3,66 (2d, J = 14 Hz, 1H chacun : SCH₂Ar) ; 4,60 (dd, J = 8 et 5,5 Hz, 1H : CH en 3α) ; de 4,75 à 4,85 (mt, 2H : CH en 2α et l'autre H du CH₂ en 5ε) ; 4,88 (dd, J = 10 et 1,5 Hz, 1H : CH en 1α) ; 5,12 (d, J = 5,5 Hz, 1H : CH en 5α) ; 5,24 (dd, J = 9 et 6,5 Hz, 1H : CH en 4α) ; 5,54 (d très large, J = 5 Hz, 1H : 5γ) ; 5,60 (d, J = 8 Hz, 1H : CH en 6α) ; 5,88 (q dédoublé, J = 7 et 1 Hz, 1H : CH en 1β) ; 6,57 (d, J = 9 Hz, 1H : CONH en 2) ; 6,59 (d, J = 8 Hz, 2H : H aromatiques en 4ε) ; 6,93 (d, J = 8 Hz, 2H : H aromatiques en 4δ) ; de 7,25 à 7,45 (mt : les 8H correspondant aux H aromatiques en 6α - à l'H en 5 de la pyridine - au 1' H₄ et au 1' H₅) ; de 7,70 à 7,80 (mt, 2H : 1' H₆ et H en 4 de la pyridine) ; 8,41 (d, J = 10 Hz, 1H : CONH en 1) ; 8,48 (dd, J = 5 et 1 Hz, 1H : H en 6 de la pyridine) ; 8,51(d, J = 8 Hz, 1H : CONH en 6) ; 8,58 d, J = 1 Hz, 1H : H en 2 de la pyridine) ; 11,68 (s, 1H : OH).

### Example 39

On opère comme à l'exemple 36, mais à partir de 2 litres d'acétonitrile, 12,2 g de 2-pipéridinoéthanethiol, 4,7 g d'hydrure de sodium, d'une part et 190 g d'un mélange brut contenant 40 % molaire de 5δ-chlorométhyl-5δ,5γ-déhydro pristinamycine I_{E} et de la triéthylamine en solution dans 1 litre d'acétonitrile, d'autre part. Le mélange réactionnel est chauffé à 55°C pendant 4 heures puis traité comme à l'exemple 36 pour donner 215 g de produit brut qui est purifié par chromatographie flash sur silice 0,04-0,063 mm (éluant : gradient dichlorométhane/méthanol 100/0 à 95/5 en volumes). Les fractions contenant le produit attendu sont concentrées puis à nouveau purifiées par chromatographie liquide haute performance (CLHP) préparative sur 500 g de silice Kromasil C8 10µm 100Å, avec comme éluant un mélange eau-acétonitrile (70/30 en volumes contenant 0,1 % d'acide trifluoroacétique). Après concentration des fractions contenant le produit attendu, la phase aqueuse résiduelle est amenée à pH 7-8 par addition d'une solution saturée de bicarbonate de sodium puis extraite par 100 cm³ de dichlorométhane. La phase organique est décantée, séchée sur sulfate de magnésium, filtrée puis concentrée à sec. Le résidu est séché à 45°C sous 90 Pa pour donner 5,2 g de 5δ-pipéridinoéthylthiométhyl-5δ,5γ-déhydro pristinamycine I_{E}, sous forme de meringue jaune fondant à 128°C.

Le 2-pipéridinoéthanethiol peut être préparé selon CLINTON et Coll., J. Am. Chem. Soc., 70, 950-51, (1948).

Spectre de R.M.N. ¹H (400 MHz, CDCl₃, δ en ppm).
0,92 (t, J = 7,5 Hz, 3H : CH₃ en 2γ) ; 1,10 (d très large, J = 16,5 Hz, 1H : 1H du CH₂ en 5β) ; 1,26 (mt, 2H : 1H du CH₂ en 3β et 1H du CH₂ en 3γ) ; 1,31 (d, J = 7 Hz, 3H : CH₃ en 1γ) ; 1,43 (mt, 2H : CH₂); 1,59 (mt, 5H : 2 CH₂ et l'autre H du CH₂ en 3γ) ; de 1,60 à 1,80 (mt, 2H : CH₂ en 2β) ; 1,99 (mt, 1H : l'autre H du CH₂ en 3β) ; de 2,35 à 2,60 (mt, 5H : SCH₂CH₂N et l'autre H du CH₂ en 5β) ; 2,58 (mf, 4H : NCH₂) ; 2,95 (s, 6H : ArN(CH₃)₂) ; 2,99 (dd, J = 14 et 7 Hz, 1H : 1H du CH₂ en 4β) ; 3,09 (s large, 2H : SCH₂) ; de 3,10 à 3,20 (mt, 1H : l'autre H du CH₂ en 4β) ; 3,17 (s, 3H : NCH₃) ; 3,28 (mt, 1H : 1H du CH₂ en 3δ) ; de 3,40 à 3,55 (mt, 2H : 1H du CH₂ en 5ε et l'autre H du CH₂ en 3δ) ; 4,57 (dd, J = 8 et 5 Hz, 1H : CH en 3α) ; 4,78 (mt, 1H : CH en 2α) ; 4,84 (d large, J = 18 Hz, 1H : l'autre H du CH₂ en 5ε) ; 4,87 (dd, J = 10 et 1,5 Hz, 1H : CH en 1α) ; 5,09 (d large, J = 5,5 Hz, 1H : CH en 5α) ; 5,22 (dd, J = 9 et 7 Hz, 1H : CH en 4α) ; 5,50 (d très large, J = 4,5 Hz, 1H : CH en 5γ) ; 5,52 (d, J = 8 Hz, 1H : CH en 6α) ; 5,87 (q dédoublé, J = 7 et 1,5 Hz, 1H : CH en 1β) ; 6,58 (d, J = 9 Hz, 1H : CONH en 2) ; 6,61 (d, J = 8 Hz, 2H : H aromatiques en 4ε) ; 6,91 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,70 (dd, J = 4 et 1 Hz, 1H : 1' H₆) ; 8,38 (d, J = 10 Hz, 1H : CONH en 1) ; 8,45 (d, J = 8 Hz, 1H : CONH en 6) ; 11,67 (mf, 1H : OH).

### Exemple 40

On opère comme à l'exemple 36, mais à partir de 4 cm³ d'acétonitrile, 67,3, mg de 2-mercaptobenzimidazole, 21,5 mg d'hydrure de sodium, d'une part et 750 mg d'un mélangé brut contenant 40% molaire de 5δ-chlorométhyl-5δ,5γ-déhydro pristinamycine I_{E} et de la triéthylamine dans 2 cm³ d'acétonitrile, d'autre part. Le mélange réactionnel est chauffé à 45°C pendant 4 heures puis traité comme à l'exemple 36 pour donner un produit brut qui est chromatographiée par chromatographie flash sur silice 32-63 µm (éluant : gradient dichlorométhane/méthanol 99/1 à 95/5 en volumes). Les fractions contenant le produit attendu sont concentrées et le résidu obtenu séché à 45°C sous 90 Pa pour donner 115 mg de 5δ-2-benzimidazolylthiométhyl-5δ,5γ-déhydro pristinamycine I_{E}, sous forme d'un solide blanc.

### Exemples 41 à 49

En opérant par analogie avec l'exemple 29 ou avec la méthode décrite dans la demande internationale WO 99/43699, on prépare les produits suivants :

### Exemple 41

### 4ζ-désdiméthylamino-4ζ-(N-méthyl-N-4-pyridylméthyl)amino-5δ-morpholinométhyl-5δ,5γ-déhydro pristinamycine I_{E}, sous forme d'un solide saumon.

Spectre de R.M.N. ¹H (400 MHz, CDCl₃, δ en ppm).
0,91 (t, J = 7,5 Hz, 3H : CH₃ en 2γ) ; 1,03 (mf, 1H : 1H du CH₂ en 5β) ; 1,25 (mt, 2H : 1H du CH₂ en 3β et 1H du CH₂ en 3γ) ; 1,33 (d, J = 7 Hz, 3H : CH₃ en 1γ) ; de 1,40 à 1,80 (mt : les 3H correspondant à l'autre H du CH₂ en 3γ et au CH₂ en 2β) ; 1,99 (mt, 1H : l'autre H du CH₂ en 3β) ; 2,39 (mf, 4H : NCH₂) ; 2,48 (d très large, J = 17 Hz, 1H : l'autre H du CH₂ en 5β) ; de 2,85 à 3,05 (mt, 3H : 1H du CH₂ en 4β et NCH₂) ; 3,09 (s, 3H : ArNCH₃) ; de 3,10 à 3,45 (mt, 3H : l'autre H du CH₂ en 4β - 1H du CH₂ en 3δ et 1H du CH₂ en 5ε ) ; 3,16 (s, 3H : NCH₃) ; 3,46 (mt, 1H : l'autre H du CH₂ en 3δ) ; 3,73 (mf, 4H : CH₂O) ; 4,53 (AB, J = 17 Hz, 2H : ArNCH₂Ar) ; 4,57 (dd, J = 8,5 et 5,5 Hz, 1H : CH en 3α) ; de 4,70 à 4,85 (mt, 2H : CH en 2α et l'autre H du CH₂ en 5ε) ; 4,87 (dd, J = 10 et 1,5 Hz, 1H : CH en 1α) ; 5,10 (d large, J = 5,5 Hz, 1H : CH en 5α) ; 5,20 (dd, J = 10 et 7 Hz, 1H : CH en 4α) ; de 5,50 à 5,65 (mf, 1H : CH en 5γ) ; 5,53 (d, J = 8 Hz, 1H : CH en 6α) ; 5,86 (q dédoublé, J = 7 et 1,5 Hz, 1H : CH en 1β) ; 6,52 (d, J = 8 Hz, 2H : H aromatiques en 4ε) ; 6,90 (d, J = 8 Hz, 2H : H aromatiques en 4δ) ; 7,00 (mf, 1H : CONH en 2) ; de 7,20 à 7,40 (mt : les 9H correspondant aux H aromatiques en 6α - aux H aromatiques en β de la pyridine - au 1' H₄ et au 1' H₅) ; 7,62 (d large, J = 4 et 1 Hz, 1H : 1' H₆) ; 8,37 (d, J = 10 Hz, 1H : CONH en 1) ; 8,48 (d large, J = 8 Hz, 1H : CONH en 6) ; 8,60 (d, J = 6 Hz, 2H : H aromatiques en α de la pyridine) ; 11,65 (s, 1H : OH).

### Exemple 42

### 4ζ-désdiméthylamino-4ζ-(N-méthyl-N-phényloxycarbonyl)amino-5δ-morpholinométhyl-5δ,5γ-déhydro pristinamycine I_{E}, sous forme d'une poudre amorphe blanche.

Spectre de masse - Ionisation chimique DCI (Desorption Chemical Ionization - ammoniac)
m/z 1056 correspondant à M+H⁺
Pureté 85 % (CLHP éluant eau-CH₃CN 50/50 en volumes + 0,1 % d'acide trifluoroacétique)

### Exemple 43

### 4ζ-désdiméthylamino-4ζ-(N-méthyl-N-propyloxycarbonyl)amino-5δ-morpholinométhyl-5δ,5γ-déhydro pristinamycine I_{E}, sous forme de poudre amorphe blanche.

Spectre de masse - Ionisation chimique DCI (Desorption Chemical Ionization - ammoniac)
m/z 1022 correspondant à M+H⁺
Pureté 86 % (CLHP éluant eau-CH₃CN 50/50 en volumes + 0,1 % d'acide trifluoroacétique)

### Exemple 44

### 4ζ-désdiméthylamino-4ζ-(N-méthyl-N-isobutyloxycarbonyl)amino-5δ-morpholino-méthyl-5δ,5γ-déhydro pristinamycine I_{E}, sous forme de poudre amorphe blanche fondant vers 148°C (déc.).

Spectre de R.M.N. ¹H (400 MHz, CDCl₃, δ en ppm).
De 0,80 à 1,00 (mt, 9H : CH₃ en 2γ et les CH₃ de l'isobutyle) ; de 1,15 à 1,35 (mt, 3H : 1H du CH₂ en 5β - 1H du CH₂ en 3β et 1H du CH₂ en 3γ) ; 1,32 (d, J = 7 Hz, 3H : CH₃ en 1γ) ; de 1,55 à 1,80 (mt : les 3H correspondant à l'autre H du CH₂ en 3γ et au CH₂ en 2β) ; de 1,85 à 2,05 (mt, 2H : l'autre H du CH₂ en 3β et le CH l'isobutyle) ; 2,36 (mf, 4H : NCH₂) ; 2,59 (dd large, J = 18 et 5 Hz, 1H : l'autre H du CH₂ en 5β) ; 2,86 (s, 2H : NCH₂) ; 3,09 (dd, J = 14 et 6,5 Hz, 1H : 1H du CH₂ en 4β) ; 3,14 (s, 3H : NCH₃) ; de 3,20 à 3,35 (mt, 2H : l'autre H du CH₂ en 4β et 1H du CH₂ en 3δ) ; 3,30 (s, 3H : ArNCH₃) ; 3,36 (d large, J = 17 Hz, 1H : 1H du CH₂ en 5ε) ; 3,49 (mt, 1H : l'autre H du CH₂ en 3δ) ; 3,70 (mt, 4H : CH₂O) ; 3,93 (d, J = 7 Hz, 2H : ArNCOOCH₂) ; 4,56 (dd, J = 7 et 5,5 Hz, 1H : CH en 3α) ; de 4,75 à 4,90 (mt, 3H : CH en 2α - l'autre H du CH₂ en 5ε et CH en 1α) ; 5,15 (d large, J = 5 Hz, 1H : CH en 5α) ; 5,35 (mt, 1H : CH en 4α) ; 5,54 (d, J = 8 Hz, 1H : CH en 6α) ; 5,56 (mt, 1H : CH en 5γ) ; 5,88 (q dédoublé, J = 7 et 1,5 Hz, 1H : CH en 1β) ; 6,57 (d, J = 9 Hz, 1H : CONH en 2) ; 7,03 (d, J = 8 Hz, 2H : H aromatiques en 4ε) ; 7,13 (d, J = 8 Hz, 2H : H aromatiques en 4δ) ; de 7,15 à 7,40 (mt : les 7H correspondant aux H aromatiques en 6α - au 1' H₄ et au 1' H₅) ; 7,70 (d large, J = 4,5 Hz, 1H : 1' H₆) ; 8,37 (d, J = 10 Hz, 1H : CONH en 1) ; 8,46 (d, J = 8 Hz, 1H : CONH en 6) ; 11,68 (s, 1H : OH).

### Exemple 45

### 4ζ-désdiméthylamino-4ζ-(N-méthyl-N-éthyloxycarbonyl)amino-5δ-morpholinométhyl-5δ,5γ-déhydro pristinamycine I_{E}, sous forme d'une poudre amorphe blanche fondant vers 158°C (déc.).

Spectre de R.M.N. ¹H (400 MHz, CDCl₃, δ en ppm).
0,93 (t, J = 7,5 Hz, 3H : CH₃ en 2γ) ; de 1,05 à 1,20 (mt, 1H : 1H du CH₂ en 5β) ; de 1,20 à 1,40 (mt, 2H : 1H du CH₂ en 3β et 1H du CH₂ en 3γ) ; 1,26 (t, J = 7 Hz, 3H : CH₃) ; 1,30 (d, J = 7 Hz, 3H : CH₃ en 1γ) ; de 1,50 à 1,80 (mt : les 3H correspondant à l'autre H du CH₂ en 3γ et au CH₂ en 2β) ; 2,01 (mt, 1H : l'autre H du CH₂ en 3β) ; 2,37 (mf, 4H : NCH₂) ; 2,54 (mt, 1H : l'autre H du CH₂ en 5β) ; 2,86 (s, 2H : NCH₂) ; 3,08 (dd, J = 13 et 7 Hz, 1H : 1H du CH₂ en 4β) ; 3,17 (s, 3H : NCH₃) ; de 3,20 à 3,40 (mt, 2H : l'autre H du CH₂ en 4β et 1H du CH₂ en 3δ) ; 3,28 (s, 3H : ArNCH₃) ; 3,35 (d large, J = 17 Hz, 1H : 1H du CH₂ en 5ε) ; 3,50 (mt, 1H : l'autre H du CH₂ en 3δ) ; 3,70 (mt, 4H : CH₂O) ; 4,21 (q, J = 7 Hz, 2H : ArNCOOCH₂) ; 4,58 (dd, J = 8 et 5,5 Hz, 1H : CH en 3α) ; de 4,70 à 4,90 (mt, 2H : CH en 2α et l'autre H du CH₂ en 5ε) ; 4,88 (d large, J = 10 Hz, 1H : CH en 1α) ; 5,13 (d large, J = 5 Hz, 1H : CH en 5α) ; 5,31 (dd, J = 10 et 7 Hz, 1H : CH en 4α) ; de 5,50 à 5,60 (mt, 2H : CH en 5γ et CH en 6α) ; 5,88 (q large, J = 7 Hz, 1H : CH en 1β) ; 6,56 (d, J = 9 Hz, 1H : CONH en 2) ; 7,05 (d, J = 8 Hz, 2H : H aromatiques en 4ε); 7,13 (d, J = 8 Hz, 2H : H aromatiques en 4δ); de 7,20 à 7,40 (mt : les 7H correspondant aux H aromatiques en 6α - au 1' H₄ et au 1' H₅) ; 7,68 (d large, J = 4,5 Hz, 1H : 1' H₆) ; 8,37 (d, J = 10 Hz, 1H : CONH en 1) ; 8,45 (d, J = 8 Hz, 1H : CONH en 6) ; 11,67 (s, 1H : OH).

### Exemple 46

### 4ζ-désdiméthylamino-4ζ-(N-méthyl-N-para-tolyloxycarbonyl)amino-5δ-morpholinométhyl-5δ,5γ-déhydro pristinamycine I_{E}, sous forme de poudre amorphe blanche fondant vers vers 147°C (déc.).

Spectre de R.M.N. ¹H (400 MHz, CDCl₃, δ en ppm).
0,93 (t, J = 7,5 Hz, 3H : CH₃ en 2γ) ; de 1,15 à 1,35 (mt, 3H : 1H du CH₂ en 5β - 1H du CH₂ en 3β et 1H du CH₂ en 3γ) ; 1,31 (d, J = 7 Hz, 3H : CH₃ en 1γ) ; de 1,50 à 1,65 (mt : 1H correspondant à l'autre H du CH₂ en 3γ) ; 1,67 et 1,77 (2 mts, 1H chacun : CH₂ en 2β) ; 2,00 (mt, 1H : l'autre H du CH₂ en 3β) ; de 2,30 à 2,40 (mf, 4H : NCH₂) ; 2,35 (s, 3H : ArCH₃) ; 2,52 (mt, 1H : l'autre H du CH₂ en 5β) ; 2,82 (s, 2H : NCH₂) ; 3,11 (dd, J = 14 et 7 Hz, 1H : 1H du CH₂ en 4β) ; 3,16 (s, 3H : NCH₃) ; de 3,20 à 3,50 (mt, 3H : l'autre H du CH₂ en 4β - 1H du CH₂ en 3δ et 1H du CH₂ en 5ε) ; 3,40 (s large, 3H : ArNCH₃) ; 3,50 (mt, 1H : l'autre H du CH₂ en 3δ) ; 3,70 (mt, 4H : CH₂O) ; 4,58 (dd, J = 8 et 6,5 Hz, 1H : CH en 3α) ; de 4,75 à 4,90 (mt, 3H : CH en 2a - l'autre H du CH₂ en 5ε et CH en 1α) ; 5,13 (d large, J = 5,5 Hz, 1H : CH en 5α) ; 5,34 (dd, J = 7,5 et 7 Hz, 1H : CH en 4α) ; 5,45 (mf, 1H : CH en 5γ) ; 5,53 (d, J = 8 Hz, 1H : CH en 6α) ; 5,87 (q dédoublé, J = 7 et 1,5 Hz, 1H: CH en 1β) ; 6,56 (d, J = 9 Hz, 1H : CONH en 2) ; de 6,95 à 7,40 (mt : les 15H correspondant aux H aromatiques en 4ε - aux H aromatiques en 4δ - aux H aromatiques du tolyle - aux H aromatiques en 6α - au 1' H₄ et au 1' H₅) ; 7,64 (d large, J = 4,5 Hz, 1H : 1' H₆) ; 8,35 (d, J = 10 Hz, 1H : CONH en 1) ; 8,44 (d, J = 8 Hz, 1H : CONH en 6) ; 11,67 (s, 1H : OH).

### Exemple 47

### 4ζ-désdiméthylamino-4ζ-(N-méthyl-N-3-butènyloxycarbonyl)amino-5δ-morpholinométhyl-5δ,5γ-déhydro pristinamycine I_{E}, sous forme d'un solide blanc fondant à 138-140°C.

Spectre de R.M.N. ¹H (400 MHz, CDCl₃, δ en ppm).
0,93 (t, J = 7,5 Hz, 3H : CH₃ en 2γ) ; de 1,15 à 1,35 (mt, 3H : 1H du CH₂ en 5β - 1H du CH₂ en 3β et 1H du CH₂ en 3γ) ; 1,31 (d, J = 7 Hz, 3H : CH₃ en 1γ) ; de 1,50 à 1,65 (mt : 1H correspondant à l'autre H du CH₂ en 3γ) ; de 1,65 à 1,80 (mt, 2H : CH₂ en 2β) ; 2,00 (mt, 1H : l'autre H du CH₂ en 3β) ; de 2,30 à 2,45 (mt, 2H : CH₂) ; 2,37 (mf, 4H : NCH₂) ; 2,57 (dd large, J = 17 et 5,5 Hz, 1H : l'autre H du CH₂ en 5β) ; 2,86 (s, 2H : NCH₂) ; 3,09 (dd, J = 14 et 7 Hz, 1H : 1H du CH₂ en 4β) ; 3,14 (s, 3H : NCH₃) ; de 3,15 à 3,35 (mt, 2H : l'autre H du CH₂ en 4β et 1H du CH₂ en 3δ) ; 3,27 (s, 3H : ArNCH₃) ; 3,36 (d large, J = 17 Hz, 1H : 1H du CH₂ en 5ε) ; 3,50 (mt, 1H : l'autre H du CH₂ en 3δ) ; 3,69 (mt, 4H : CH₂O) ; 4,19 (t, J = 7 Hz, 2H : ArNCOOCH₂) ; 4,57 (dd, J = 8 et 6,5 Hz, 1H : CH en 3α) ; de 4,70 à 4,85 (mt, 2H : CH en 2α et l'autre H du CH₂ en 5ε) ; 4,87 (dd, J = 10 et 1,5 Hz, 1H : CH en 1α) ; de 5,05 à 5,15 (mt, 2H : =CH₂) ; 5,14 (d large, J = 5,5 Hz, 1H : CH en 5α) ; 5,34 (dd, J = 7,5 et 7 Hz, 1H : CH en 4α) ; 5,54 (d, J = 8 Hz, 1H : CH en 6α) ; 5,56 (mt, 1H : CH en 5γ) ; de 5,65 à 5,85 (mt, 1H : CH=) ; 5,88 (q dédoublé, J = 7 et 1,5 Hz, 1H : CH en 1β) ; 6,57 (d, J = 9 Hz, 1H : CONH en 2) ; 7,04 (d, J = 8 Hz, 2H : H aromatiques en 4ε) ; 7,12 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,69 (dd, J = 4,5 et 1 Hz, 1H : 1' H₆) ; 8,37 (d, J = 10 Hz, 1H : CONH en 1) ; 8,46 (d, J = 8 Hz, 1H : CONH en 6) ; 11,68 (s, 1H : OH).

### Exemple 48

### 4ζ-désdiméthylamino-4ζ-(N-méthyl-N-néopentyloxycarbonyl)amino-5δ-morpholinométhyl-5δ,5γ-déhydro pristinamycine I_{E}, sous forme de solide blanc fondant à 140-146°C.

Spectre de R.M.N. ¹H (400 MHz, CDCl₃, δ en ppm).
De 0,75 à 1,00 (mt, 12H : CH₃ en 2γ et C(CH₃)₃) ; de 1,15 à 1,35 (mt, 3H : 1H du CH₂ en 5β - 1H du CH₂ en 3β et 1H du CH₂ en 3γ) ; 1,30 (d, J = 7 Hz, 3H : CH₃ en 1γ) ; de 1,50 à 1,80 (mt : les 3H correspondant à l'autre H du CH₂ en 3γ et au CH₂ en 2β) ; 1,99 (mt, 1H : l'autre H du CH₂ en 3β) ; 2,35 (mf, 4H : NCH₂) ; 2,59 (dd large, J = 17 et 5 Hz, 1H : l'autre H du CH₂ en 5β) ; 2,85 (s, 2H : NCH₂) ; de 3,10 à 3,40 (mt, 4H : CH₂ en 4β - 1H du CH₂ en 3δ et 1H du CH₂ en 5ε) ; 3,12 (s, 3H : NCH₃) ; 3,30 (s, 3H : ArNCH₃) ; 3,49 (mt, 1H : l'autre H du CH₂ en 3δ) ; 3,68 (mt, 4H : CH₂O) ; 3,84 (s, 2H : ArNCOOCH₂) ; 4,55 (dd, J = 7 et 5 Hz, 1H : CH en 3α) ; de 4,70 à 4,85 (mt, 2H : CH en 2α et l'autre H du CH₂ en 5ε) ; 4,86 (dd, J = 10 et 1,5 Hz, 1H : CH en 1α) ; 5,15 (d large, J = 5 Hz, 1H : CH en 5α) ; 5,35 (t, J = 8 Hz, 1H : CH en 4α) ; de 5,50 à 5,60 (mt, 2H : CH en 6α et CH en 5γ) ; 5,87 (q dédoublé, J = 7 et 1,5 Hz, 1H : CH en 1β) ; 6,57 (d, J = 9 Hz, 1H : CONH en 2) ; 7,03 (d, J = 8 Hz, 2H : H aromatiques en 4ε) ; 7,12 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,69 (dd, J = 4,5 et 1 Hz, 1H : 1' H₆) ; 8,37 (d, J = 10 Hz, 1H : CONH en 1) ; 8,46 (d, J = 8 Hz, 1H : CONH en 6) ; 11,67 (s, 1H : OH).

### Exemple 49

Dans un tricol contenant 30 cm³ de dioxane, on introduit 1,3 g de 4ζ-désdiméthylamino-4ζ-(N-méthyl-N-allyloxycarbonyl)amino-5δ-morpholinométhyl-5δ,5γ-déhydro pristinamycine I_{E}, préparé comme dans l'exemple 29 puis 10 mg de trisphénylphosphine et 20 mg de palladium trisbenzylidène acétone. Le mélange réactionnel est porté au reflux 39 heures en ajoutant 3 fois 20 mg de palladuim trisbenzylidène acétone au cours des dernières 21 heures. Le mélange réactionnel est concentré sous pression réduite pour donner 1,1 g de meringue verte qui est chromatographiée sur silice (éluant : dichlorométhane/méthanol 95/5 en volumes). On obtient 0,45 g d'un produit qui est repurifié par CLHP préparative sur 450 g de silice Kromasil C8 10µm 100Å, ( éluant : eau-acétonitrile 65/35 en volumes contenant 0,1 % d'acide trifluoroacétique). Après concentration des fractions contenant le produit attendu, la phase aqueuse résiduelle est amenée à pH 7-8 par addition d'une solution saturée de bicarbonate de sodium. Le mélange obtenu est extrait par 2 fois 20 cm³ de dichlorométhane. Les phases organiques sont rassemblées, séchées sur sulfate de magnésium, filtrée puis concentrées à sec. Le résidu est séché à 45°C sous 90Pa pour donner 130 mg de 4ζ-désdiméthylamino-4ζ-(N-méthyl-N-allyl)amino-5δ-morpholinométhyl-5δ,5γ-déhydro pristinamycine I_{E}, sous forme de solide jaune pâle fondant à 156°C.

Spectre de R.M.N. ¹H (400 MHz, CDCl₃, δ en ppm).
0,92 (t, J = 7,5 Hz, 3H : CH₃ en 2γ) ; de 0,90 à 1,00 (mt, 1H : 1H du CH₂ en 5β) ;1,23 (mt, 2H : 1H du CH₂ en 3β et 1H du CH₂ en 3γ) ; 1,31 (d, J = 7 Hz, 3H : CH₃ en 1γ) ; 1,55 (mt, 1H : l'autre H du CH₂ en 3γ) ; de 1,60 à 1,80 (mt : les 2H correspondant au CH₂ en 2β) ; 2,00 (mt, 1H : l'autre H du CH₂ en 3β) ; 2,37 (mf, 4H : NCH₂) ; 2,43 (dd large, J = 17 et 5 Hz, 1H : l'autre H du CH₂ en 5β) ; 2,85 (mf, 2H : NCH₂) ; de 2,90 à 3,00 (mt, 1H : 1H du CH₂ en 4β) ; 2,96 (s, 3H : ArNCH₃) ; de 3,15 à 3,25 (mt, 1H : l'autre H du CH₂ en 4β) ; 3,20 (s, 3H : NCH₃) ; 3,33 (d large, J = 18 Hz, 1H : 1H du CH₂ en 5ε) ; 3,47 (mt, 1H : 1H du CH₂ en 3δ) ; de 3,65 à 3,80 (mt, 5H : l'autre H du CH₂ en 3δ et CH₂O) ; 3,91 et 3,99 (2 dd, J = 16 et 5 Hz, 1H chacun : ArNCH₂) ; 4,60 (dd, J = 8 et 5,5 Hz, 1H : CH en 3α) ; 4,78 (mt, 1H : CH en 2α) ; 4,85 (d large, J = 18 Hz, 1H : l'autre H du CH₂ en 5ε) ; 4,88 (dd, J = 10 et 1,5 Hz, 1H : CH en 1α) ; 5,10 (d large, J = 5 Hz, 1H : CH en 5α) ; de 5,10 à 5,25 (mt, 3H : =CH₂ et CH en 4α) ; 5,49 (d très large, J = 5 Hz, 1H : CH en 5γ) ; 5,52 (d, J = 8 Hz, 1H : CH en 6α) ; de 5,80 à 5,95 (mt, 1H : CH=) ; 5,86 (q dédoublé, J = 7 et 1,5 Hz, 1H : CH en 1β) ; 6,56 (d, J = 9 Hz, 1H : CONH en 2) ; 6,57 (d, J = 8 Hz, 2H : H aromatiques en 4ε) ; 6,93 (d, J = 8 Hz, 2H : H aromatiques en 4δ) ; de 7,20 à 7,40 (mt : les 7H correspondant aux H aromatiques en 6α - au 1' H₄ et au 1' H₅) ; 7,75 (dd, J = 4,5 et 1 Hz, 1H : 1' H₆) ; 8,39 (d, J = 10 Hz, 1H : CONH en 1) ; 8,47 (d, J = 8 Hz, 1H : CONH en 6) ; 11,66 (s, 1H : OH).

A titre d'exemple des dérivés de streptogramines de formule générale (β) peuvent être préparés selon ou par analogie avec la méthode ci-après :

### Exemple de référence

### (16R)-16-Désoxo-16-fluoro pristinamycine II_{B}

A 1,12 g de (16*R*)-16-désoxo-16-fluoro-14-O-(tert-butyldiphénylsilyl) pristinamycine II_{B} en solution dans 10 cm³ de tétrahydrofurane, on ajoute à 20°C, sous atmosphère d'argon, 0,2 cm³ d'acide acétique et 0,6 g de fluorure de tétra n-butylammonium trihydraté. Après 168 heures d'agitation, le mélange réactionnel est concentré à sec sous pression réduite (2,7 kPa) pour donner 1 g d'une huile marron qui est purifiée par chromatographie-flash [éluant : dichlorométhane / méthanol / acétonitrile (90 / 5 / 5 en volumes)]. On obtient 0,3 g de (16*R*)-16-désoxo-16-fluoro pristinamycine II_{B}, sous forme d'un solide beige clair fondant vers 125°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H) ; de 1,55 à 2,05 (mt : 5H) ; 1,83 (s : 3H) ; de 2,10 à 2,30 (mt : 2H) ; 2,76 (mt : 1H) ; 2,98 (mt : 1H) ; 3,21 (mt : 1H) ; 3,48 (mt : 1H) ; 3,87 (mt : 1H) ; 4,07 (mt : 1H) ; 4,55 (mt : 1H) ; de 4,75 à 4,90 (mt : 3H) ; 5,14 (doublet démultiplié, J _{HF} = 48 Hz : 1H) ; 5,39 (d, J = 9 Hz : 1H) ; 5,71 (mt : 1H) ; 5,82 (dd, J = 17 et 2 Hz : 1H) ; 6,00 (mt : 1H) ; 6,21 (d, J = 16 Hz : 1H) ; 6,52 (dd, J = 17 et 5 Hz : 1H) ; 8,12 (s : 1H).

La (16*R*)-16-désoxo-16-fluoro-14-O-(tert-butyldiphénylsilyl) pristinamycine II_{B} peut être préparée de la manière suivante :

A 2 g de (16*S*)-16-hydroxy-14-O-(tert-butyldiphénylsilyl) pristinamycine II_{B} en solution dans 50 cm³ de dichlorométhane, on ajoute lentement à 20°C, sous atmosphère d'argon, 0,464 cm³ de trifluorure de diéthylaminosulfure. Après 2 heures d'agitation, le mélange réactionnel est versé sur 100 cm³ d'une solution aqueuse saturée de bicarbonate de sodium. La phase organique est décantée, lavée par 2 fois 100 cm³ d'eau, séchée sur sulfate de magnésium, filtrée puis concentrée à sec sous pression réduite (2,7 kPa) pour donner 2,1 g d'un solide ocre qui est purifié par chromatographie-flash [éluant : gradient dichlorométhane / acétonitrile / méthanol (100/0/0; 99/ 0,5 / 0,5 puis 98 / 1 / 1 en volumes)]. On obtient 1,35 g de (16*R*)-16-désoxo-16-fluoro-14-O-(tert-butyldiphénylsilyl) pristinamycine II_{B}, sous forme d'un solide blanc fondant vers 116°C (déc.).

Spectre de R.M.N. ¹H (400 MHz, CDCl₃, δ en ppm) : 0,96 (d, J = 6,5 Hz : 3H) ; 0,99 (d, J = 6,5 Hz : 3H) ; de 1,00 à 1,15 (mt : 12H) ; 1,29 (s : 3H) ; de 1,55 à 1,95 (mt : 4H) ; 1,96 (mt : 1H) ; 2,13 (mt : 1H) ; 2,24 (mt : 1H) ; 2,76 (mt : 1H) ; 2,85 (mt : 1H) ; 3,03 (mt : 1H) ; 3,39 (mt : 1H) ; 3,80 (mt : 1H) ; 4,01 (mt : 1H) ; 4,57 (mt : 1H) ; 4,72 (mt : 1H) ; de 4,75 à 4,85 (mt : 2H) ; 5,01 (doublet démultiplié, J _{HF} = 48 Hz : 1H) ; 5,38 (d, J = 9 Hz : 1H) ; 5,50 (mt : 1H) ; 5,81 (dd, J = 17 et 1,5 Hz : 1H) ; 5,97 (mt : 1H) ; 6,10 (d, J = 15,5 Hz : 1H) ; 6,49 (dd, J = 17 et 5 Hz : 1H) ; de 7,30 à 7,50 (mt : 6H) ; 7,63 (d large, J = 7 Hz : 2H) ; 7,68 (d large, J = 7 Hz : 2H) ; 8,08 (s : 1H).

La (16*S*)-16-hydroxy-14-O-(tert-butyldiphénylsilyl) pristinamycine Ils peut, être préparée de la manière suivante :

A 22 g de (16*S*)-16-hydroxy pristinamycine II_{B} en solution dans 200 cm³ de dichlorométhane, on ajoute à 20°C, sous atmosphère d'argon, 29 cm³ de diisopropyléthylamine, 43,2 cm³ de tert-butyldiphénylchlorosilane goutte à goutte et 1,01 g de 4-diméthylaminopyridine. Après 22 heures d'agitation, le mélange réactionnel est versé sur 600 cm³ d'une solution aqueuse saturée de bicarbonate de sodium. La phase aqueuse est décantée puis extraite par 2 fois 100 cm³ de dichlorométhane. Les phases organiques sont rassemblées, lavées par 400 cm³ d'une solution aqueuse saturée de chlorure de sodium, séchées sur sulfate de sodium, filtrées puis concentrées à sec sous pression réduite (2,7 kPa) pour donner 70,6 g d'une huile visqueuse orange qui est agité dans 600 cm³ d'éther diisopropylique pendant 16 heures. Après filtration et séchage sous pression réduite (2,7 kPa) à 20°C, on obtient 28 g de (16*S*)-16-hydroxy-14-O-(tert-butyldiphénylsilyl) pristinamycine II_{B}, sous forme d'un solide rosé, fondant vers 133°C (déc.).

Spectre de R.M.N. ¹H (400 MHz, CDCl₃, δ en ppm) : 0,95 (d, J = 6,5 Hz : 3H) ; de 1,00 à 1,05 (mt : 9H) ; 1,08 (s : 9H) ; de 1,40 à 1,80 (mt : 3H) ; de 1,90 à 2,15 (mt : 3H) ; 2,23 (d large, J = 14 Hz : 1H) ; 2,75 (mt : 1H) ; 2,83 (dd, J = 17 et 11 Hz : 1H) ; 3,10 (dd, J = 17 et 2,5 Hz : 1H); 3,25 (mt : 1H) ; de 3,60 à 3,75 (mt : 2H) ; 4,49 (mt : 1H) ; 4,56 (mt : 1H) ; de 4,60 à 4,70 (mt : 2H) ; 4,87 (mt : 1H) ; 5,49 (mt : 1H) ; 5,74 (dd, J = 17 et 2 Hz : 1H) ; 5,78 (d, J = 9 Hz : 1H) ; 5,95 (mt : 1H) ; 6,04 (d, J = 16 Hz : 1H) ; 6,41 (dd, J = 17 et 4 Hz : 1H) ; de 7,30 à 7,50 (mt : 6H) ; 7,64 (dd, J = 7 et 1,5 Hz : 2H) ; 7,69 (dd, J = 7 et 1,5 Hz : 2H) ; 8,11 (s : 1H).

La (16*S*)-16-hydroxy pristinamycine II_{B} peut être préparée de la manière suivante : Une suspension de 11,35 g de borohydrure de sodium dans 550 cm³ de dichlorométhane est chauffée au reflux pendant 20 minutes. On ajoute alors, goutte à goutte, en environ 30 minutes, 68,6 cm³ d'acide acétique puis une solution (préalablement séchée sur sulfate de sodium) de 52,75 g de pristinamycine II_{B} dans 230 cm³ de dichlorométhane, en environ 45 minutes. Le mélange réactionnel est agité 4,5 heures au reflux puis 16 heures à 20°C. On ajoute alors au mélange réactionnel 500 cm³ de dichlorométhane et 1500 cm³ d'eau. La phase organique est décantée et la phase aqueuse est extraite par 500 cm³ de dichlorométhane. Les phases organiques sont réunies et le pH est ajusté à 8 par une addition lente de 1000 cm³ d'une solution aqueuse saturée de bicarbonate de sodium. La phase organique résultante est lavée successivement par 1000 cm³ d'eau et 1000 cm³ d'une solution aqueuse saturée de chlorure de sodium puis traitée au noir végétal 3S, séchée sur sulfate de sodium, filtrée et concentrée à sec sous pression réduite (2,7 kPa) pour donner 50 g d'un solide jaune clair. A une solution du solide précédent dans 900 cm³ de dichlorométhane, on ajoute à 20°C, 378 cm³ d'une solution aqueuse d'hydroxyde d'ammonium 0,5 M. Après 16 heures d'agitation à 20°C, la phase organique est décantée, lavée par 1000 cm³ d'eau puis 1000 cm³ d'une solution aqueuse saturée de chlorure de sodium, séchée sur sulfate de sodium, filtrée et concentrée à sec sous pression réduite (2,7 kPa) pour donner 46 g d'un solide jaune pâle qui est purifié par chromatographie-flash [éluant : gradient dichlorométhane / méthanol (98 / 2 et 97 / 3 en volumes)]. On obtient 31,68 g de (16*S*)-16-hydroxy pristinamycine II_{B}, sous forme d'un solide blanc cassé fondant vers 131°C (déc.).

Spectre de R.M.N. ¹H (400 MHz, CDCl₃, δ en ppm) : 0,96 (d, J = 6,5 Hz : 3H) ; 1,02 (d, J = 6,5 Hz : 3H) ; 1,07 (d, J = 6,5 Hz : 3H) ; de 1,70 à 1,90 (mt : 3H) ; 1,76 (s : 3H) ; 1,97 (mt : 2H) ; 2,12 (mt : 1H) ; 2,26 (d large : 14,5 Hz : 1H) ; 2,56 (d, J = 3 Hz : 1H) ; 2,76 (mt : 1H) ; 2,90 (dd, J = 16 et 10 Hz : 1H) ; 3,08 (dd, J = 16 et 3 Hz : 1H) ; 3,35 (mt : 1H) ; 3,82 (mt : 2H) ; 3,99 (d, J = 2,5 Hz : 1H) ; de 4,40 à 4,55 (mt : 2H) ; de 4,65 à 4,75 (mt : 2H) ; 5,03 (mt : 1H) ; de 5,65 à 5,85 (mt : 3H) ; 6,01 (mt : 1H) ; 6,21 (d, J = 16 Hz : 1H) ; 6,46 (dd, J = 17 et 5 Hz : 1H) ; 8,13 (s : 1H).

La présente invention concerne également les compositions pharmaceutiques contenant au moins un dérivé de streptogramine selon l'invention, le cas échéant sous forme de sel, à l'état pur ou sous forme d'une association avec un ou plusieurs diluants ou adjuvants compatibles et pharmaceutiquement acceptables. L'invention concerne également les compositions pharmaceutiques ci-dessus lorsqu'elles contiennent en outre, au moins un dérivé de streptogramine du groupe A, ou le cas échéant un de ses sels, associé à la/les streptogramine(s) de formule générale (I).

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention, généralement sous forme d'association est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate "d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de streptogramine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 1 et 3 g de produit actif en 2 ou 3 prises par jour, par voie orale pour un adulte.

L'exemple suivant illustre une composition selon l'invention.

### EXEMPLE

On prépare selon la technique habituelle des comprimés dosés à 250 mg de produit actif, ayant la composition suivante :
- de 5δ-(1-morpholino)méthyl 5δ,5γ-déhydro pristinamycine I_{E} 75 mg
- pristinamycine II_{B} 175 mg
- excipient : amidon, silice hydratée, dextrine, gélatine, stéarate de magnésium : qsp 500 mg

## Revendications

1. Un dérivé du groupe B des streptogramines de formule générale : 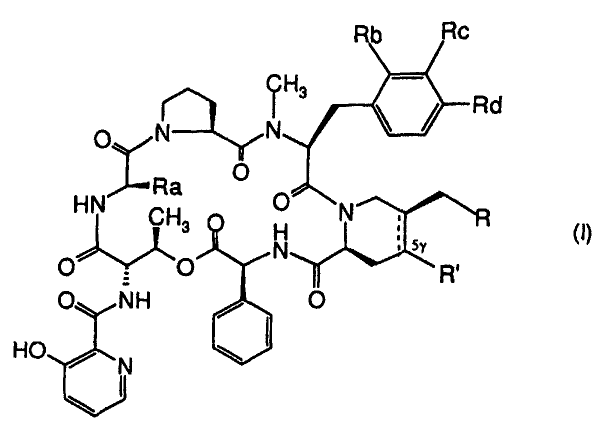 dans laquelle :
R représente un radical -NR₁R₂ ou -SR₃ pour lequel :
R₁ et R₂ identiques ou différents représentent un atome d'hydrogène, un radical alcoyle (1 à 8 carbones) éventuellement substitué par hydroxy, alcényle (3 à 8 carbones), cycloalcoyle (3 à 8 carbones), alcoyloxy (1 à 8 carbones), dialcoylamino, phénylalcoyle éventuellement substitué [par un ou plusieurs atomes d'halogène ou radicaux alcoyle, hydroxyalcoyle, alcoyloxy ou dialcoylamino], hétérocyclylalcoyle saturé ou insaturé (3 à 8 chaînons) contenant 1 ou plusieurs hétéroatomes choisis parmi l'azote, le soufre ou l'oxygène, ou dialcoylaminoalcoyle, ou bien
R₁ et R₂ forment ensemble avec l'atome d'azote auquel ils sont attachés, un hétérocycle mono ou polycyclique, saturé, partiellement saturé ou insaturé, de 3 à 12 chaînons, contenant éventuellement un autre hétéroatome choisi parmi l'oxygène le soufre ou l'azote et éventuellement substitué [par un ou plusieurs radicaux hydroxy, alcoyle, phényle éventuellement substitué par un atome d'halogène, phénylalcoyle, phéhylalcényle (alcényle contenant 2 à 4 carbones), hydroxyalcoyle, acyle, alcoyloxycarbonyle, ou hétérocyclyle ou hétérocyclyl-carbonyle dont la partie hétérocyclyle est saturée ou insaturée (4 à 6 chaînons) et contient 1 ou plusieurs hétéroatomes choisis parmi l'oxygène le soufre ou l'azote],
R₃ est un radical alcoyle (contenant 1 à 8 atomes de carbone) ou cycloalcoyle (contenant 3 à 8 atomes de carbone) substitués par un radical -NR₁R₂ pour lequel R₁ et R₂ identiques ou différents représentent un atome d'hydrogène ou un radical alcoyle ou forment ensemble avec l'atome d'azote auquel ils sont attachés, un hétérocycle tel que défini ci-dessus, ou bien R₃ représente un radical hétérocyclyle mono ou polycyclique ou hétérocyclylméthyle saturé ou insaturé contenant 3 à 7 chaînons et éventuellement un autre hétéroatome choisi parmi l'oxygène le soufre ou l'azote et éventuellement substitué par un radical alcoyle. 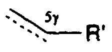
représente le reste d'un cycle insaturé non substitué en 5γ : 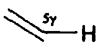 ou
le reste d'un cycle saturé substitué en 5γ par un radical fluoro : 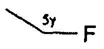 Ra est un radical méthyle ou éthyle, et
Rb, Rc et Rd ont les définitions ci-après :
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle (3 à 5C), et Rd est un radical -NMe-R''' pour lequel R''' représente un radical alcoyle, hydroxyalcoyle (2 à 4C), ou alcényle (2 à 8C) éventuellement substitué par phényle, cycloalcoyl (3 à 6C) méthyle, benzyle, benzyle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R''' représente un radical cyanométhyle ou carboxyméthyle, ou représente -CORe ou -CH₂CORe pour lesquels soit Re est -OR'e, R'e étant alcoyle (1 à 6 carbones), alcényle (2 à 6 carbones), benzyle, phényle, tolyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
7) Rc est un atome d'hydrogène et Rb et Rd représentent un radical méthyle,
étant entendu que sauf mention spéciale, les radicaux alcoyle ou acyle sont droits ou ramifiés contiennent 1 à 4 atomes de carbone et que les radicaux alcényle sont également en chaîne droite ou ramifiée et contiennent 2 à 4 atomes de carbone, ainsi que ses sels lorsqu'ils existent.

2. Un dérivé du groupe B des streptogramines selon la revendication 1, **caractérisé en ce que**
R représente un radical -NR₁R₂ ou -SR₃ pour lequel :
R₁ et R₂ identiques ou différents représentent un atome d'hydrogène, un radical alcoyle (1 à 8 carbones) éventuellement substitué par hydroxy, alcényle (3 à 8 carbones), cycloalcoyle (3 à 8 carbones), alcoyloxy (1 à 8 carbones), dialcoylamino, phénylalcoyle éventuellement substitué [par un ou plusieurs atomes d'halogène ou radicaux alcoyle, hydroxyalcoyle, alcoyloxy ou dialcoylamino], hétérocyclylalcoyle saturé ou insaturé (3 à 8 chaînons) contenant 1 ou plusieurs hétéroatomes choisis parmi l'azote, le soufre ou l'oxygène, ou dialcoylaminoalcoyle, ou bien
R₁ et R₂ forment ensemble avec l'atome d'azote auquel ils sont attachés, un hétérocycle mono ou polycyclique, saturé, partiellement saturé ou insaturé, de 3 à 12 chaînons, contenant éventuellement un autre hétéroatome choisi parmi l'oxygène le soufre ou l'azote et éventuellement substitué [par un ou plusieurs radicaux hydroxy, alcoyle, phényle éventuellement substitué par un atome d'halogène, phénylalcoyle, hydroxyalcoyle, acyle, alcoyloxycarbonyle, ou hétérocyclyle ou hétérocyclylcarbonyle dont la partie hétérocyclyle est saturée ou insaturée (4 à 6 chaînons) et contient 1 ou plusieurs hétéroatomes choisis parmi l'oxygène le soufre ou l'azote],
R₃ est un radical alcoyle (contenant 1 à 8 atomes de carbone) substitué par un radical -NR₁R₂ pour lequel R₁ et R₂ identiques ou différents représentent un radical alcoyle ou forment ensemble avec l'atome d'azote auquel ils sont attachés, un hétérocycle tel que défini ci-dessus, ou bien R₃ représente un radical hétérocyclyle mono ou polycyclique ou hétérocyclylméthyle saturé ou insaturé contenant 3 à 7 chaînons et éventuellement un autre hétéroatome choisi parmi l'oxygène le soufre ou l'azote et éventuellement substitué par un radical alcoyle. 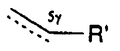
représente le reste d'un cycle insaturé non substitué en 5γ : 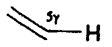 ou
le reste d'un cycle saturé substitué en 5γ par un radical fluoro : 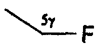 Ra est un radical éthyle, et
Rb, Rc et Rd ont les définitions ci-après :
1) Rb et Rc sont des atomes d'hydrogène et Rd est radical méthylamino ou diméthylamino,
2) Rb est un atome d'hydrogène, Rc est un atome d'hydrogène ou de chlore et Rd est un radical -NMe-R"' pour lequel R''' représente un radical alcényle (2 à 8C), hétérocyclylméthyle ou représente -COOR'e ou R'e étant alcoyle (1 à 6 carbones), alcényle (2 à 6 carbones), phényle ou tolyle
3) Rb est un atome d'hydrogène, Rd est un radical -NHCH₃ ou -N(CH₃)₂ et Rc est un atome de chlore, ainsi que ses sels lorsqu'ils existent.

3. Un dérivé du groupe B des streptogramines selon la revendication 1, **caractérisé en ce qu'**il s'agit du 5δ-(1-morpholino)méthyl 5δ,5γ-déhydro pristinamycine I_{E}.

4. Un dérivé du groupe B des streptogramines selon la revendication 1, **caractérisé en ce qu'**il s'agit du 5δ-[N-méthyl N-2-(1,3-dioxolanyl)méthyl]aminométhyl-5δ,5γ-déhydro pristinamycine I_{E}.

5. Un dérivé du groupe B des streptogramines selon la revendication 1, **caractérisé en ce qu'**il s'agit du 5δ-morpholinométhyl-4ζ-méthylamino-4ζ-désdiméthylamino-5δ,5γ-déhydro pristinamycine I_{E}.

6. Un dérivé du groupe B des streptogramines selon la revendication 1, **caractérisé en ce qu'**il s'agit du 5δ-morpholinométhyl-4ζ-méthylamino-4ζ-désdiméthylamino-5δ,5γ-déhydro-4ε-chloro pristinamycine I_{E}.

7. Un dérivé du groupe B des streptogramines selon la revendication 1, **caractérisé en ce qu'**il s'agit du 5δ-[bis-(2-méthoxyéthyl)aminométhyl]-5δ,5γ-déhydro pristinamycine I_{E}.

8. Un procédé de préparation d'un dérivé de streptogramine selon la revendication 1, **caractérisé en ce que** l'on fait agir un agent de fluoration sur le dérivé de synergistine du groupe B de formule générale : 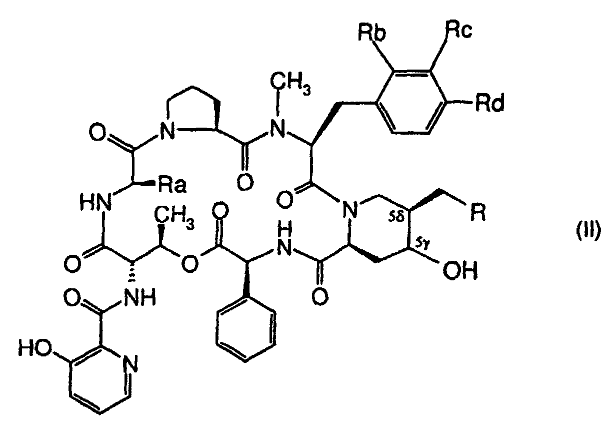 dans laquelle R, Ra, Rb, Rc et Rd sont définis comme dans la revendication 1, puis sépare le dérivé fluoré ou le dérivé insaturé en position 5γ,5δ, et le cas échéant transforme le dérivé de streptogramine obtenu en un sel.

9. Un procédé selon la revendication 8, **caractérisé en ce que** la séparation du dérivé fluoré et du dérivé insaturé en position 5γ,5δ, s'effectue par chromatographie ou par cristallisation.

10. Un procédé de préparation d'un dérivé de streptogramine selon la revendication 1, pour lequel le symbole 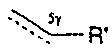 représente 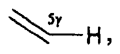 **caractérisé en ce que** l'on fait agir un halogénure de thionyle, en présence d'une base azotée, sur un dérivé de synergistine du groupe B de formule générale : 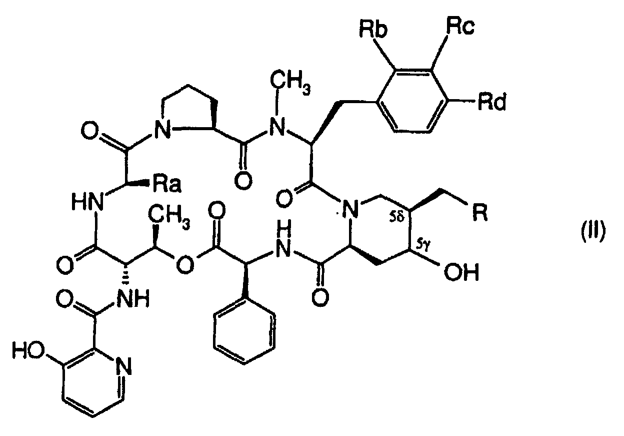 dans laquelle R, Ra, Rb, Rc et Rd sont définis comme dans la revendication 1, puis le cas échéant transforme le dérivé de streptogramine obtenu en un sel.

11. Un procédé de préparation d'un dérivé de streptogramine selon la revendication 1, pour lequel le symbole 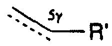 représente 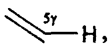 **caractérisé en ce que** l'on fait agir une amine HNR₁R₂ ou un thiol HS-R₃ sur un dérivé halogéné de streptogramine de formule générale : 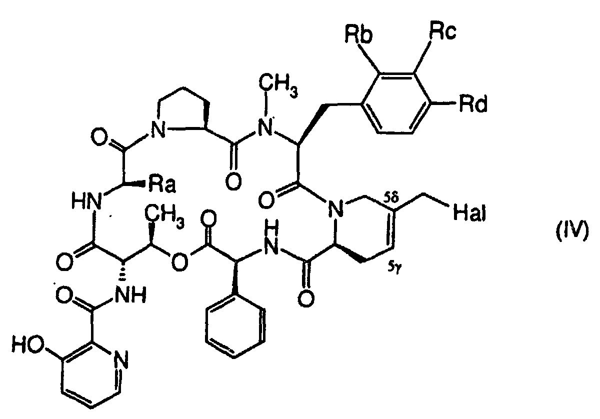 dans laquelle Ra, Rb, Rc et Rd sont définis comme dans la revendication 1, et Hal représente un atome d'halogène, puis le cas échéant transforme le dérivé de streptogramine obtenu en un sel.

12. Un dérivé de streptogramine formule générale : 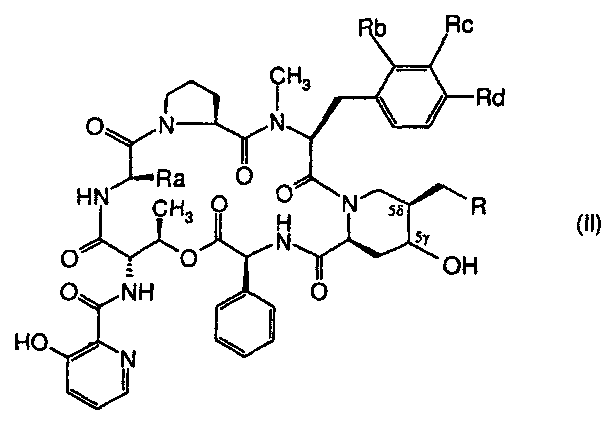 dans laquelle R, Ra, Rb, Rc et Rd sont définis comme dans la revendication 1.

13. Compositions pharmaceutiques comprenant au moins un dérivé de streptogramine du groupe B selon la revendication 1, à l'état pur ou sous forme d'association avec au moins un dérivé de streptogramine du groupe A, le cas échéant sous forme de sel, et/ou sous forme d'association avec un ou plusieurs diluants ou adjuvants compatibles et pharmaceutiquement acceptables.

14. Compositions pharmaceutiques selon la revendication 13, **caractérisées en ce que** le dérivé de la streptogramine du groupe A est choisi parmi la pristinamycine IIA, la pristinamycine IIB, la pristinamycine IIC, la pristinamycine IID, la pristinamycine IIE, la pristinamycine IIF, la pristinamycine IIG ou parmi des dérivés d'hémisynthèse connus ou parmi les dérivés de formule générale : 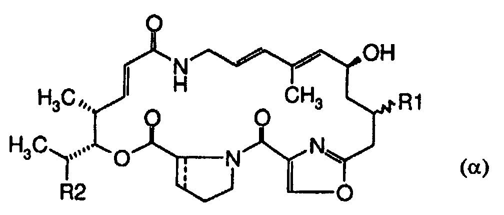 dans laquelle R₁ est un radical -NR'R" pour lequel R' est un atome d'hydrogène ou un radical méthyle, et R" est un atome d'hydrogène, un radical alcoyle, cycloalcoyle, allyle, propargyle, benzyle, ou -OR''', R''' étant un atome d'hydrogène, un radical alcoyle, cycloalcoyle, allyle, propargyle ou benzyle, ou -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, R₂ est un atome d'hydrogène ou un radical méthyle ou éthyle, et la liaison --- représente une liaison simple ou une liaison double,
ou encore parmi des dérivés d'hémisynthèse de formule générale : 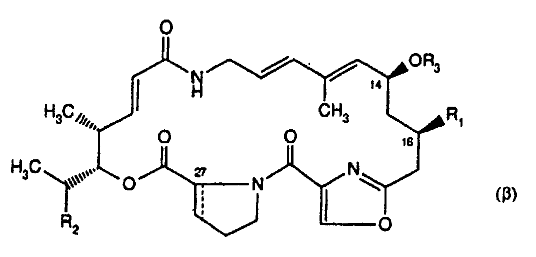 dans laquelle R₁ représente un atome d'halogène ou un radical azido ou thiocyanato, R₂ représente un atome d'hydrogène ou un radical méthyle ou éthyle, R₃ représente un atome d'hydrogène, ou le reste d'un ester aliphatique, cycloaliphatique, aromatique, araliphatique, hétérocyclique ou hétérocyclylaliphatique pouvant être substitué, et la liaison --- représente une liaison simple (stéréochimie 27R) ou une liaison double, et leurs sels lorsqu'ils existent. Et notamment les produits de formule générale (β) pour lesquels le reste d'ester R₃ peut être choisi parmi : des radicaux R'₃-CO- pour lesquels R'₃ est phényle ou phénylalcoyle non substitués ou substitués sur le radical phényle [par un ou plusieurs radicaux choisis parmi alcoyle, portant éventuellement un radical NR"R''' dont les radicaux R" et R''' identiques ou différents peuvent être des atomes d'hydrogène ou des radicaux alcoyle pouvant former ensemble avec l'atome d'azote auquel ils sont attachés un radical hétérocyclyle saturé ou insaturé de 3 à 8 chaînons, comprenant éventuellement un autre hétéroatome choisi parmi l'oxygène, le soufre ou l'azote, ledit hétérocycle pouvant être lui même substitué par un ou plusieurs radicaux (alcoyle, hydroxyalcoyle, alcoyloxyalcoyle, alcoyloxycarbonylalcoyle, aryle, hétérocyclyle, hétérocyclylalcoyle saturés ou insaturés de 3 à 8 chaînons ou -CH₂-CO-NR''R'''), ou bien R" et/ou R''' peuvent être un radical hydroxyalcoyle, phényle, hétérocyclylalcoyle saturé ou insaturé de 3 à 8 chaînons, -CO-NR''R''' pour lequel NR''R''' est défini comme précédemment, ou alcoyle où acyle substitués par NR''R''' défini tel que ci-dessus], ou bien R'₃ peut être choisi parmi des radicaux phényle ou phénylalcoyle substitués sur le radical phényle par un ou plusieurs radicaux [choisis parmi alcoyle, pouvant être substitués par un radical alcoyloxy ou alcoylthio éventuellement portant eux-même un radical carboxy ou un radical NR"R''' tel que défini ci-dessus, ou choisis parmi acyloxy pouvant être substitué par NR"R''' défini tel que précédemment], ou bien R'₃ peut être choisi parmi des radicaux alcoyle ou cycloalcoyle éventuellement substitués [par un radical carboxy, carboxyalcoyldisulfanyle ou par un radical NR"R''', -CH₂-NR"R''', -CO-NR"R''', ou par un radical alcoyloxycarbonyle, alcoyloxy, ou alcoyldisulfanyle éventuellement substitués par NR"R''' ou -CO-NR"R''' pour lesquels NR"R''' est défini tel que précédemment], ou bien R'₃ peut être choisi parmi des radicaux hétérocyclyle saturés ou insaturés de 3 à 8 chaînons éventuellement substitués [par alcoyle ou acyle eux-même éventuellement substitués par NR''R'''].

15. Associations d'un dérivé de la streptogramine du groupe B, selon la revendication 1, avec au moins un dérivé de la streptogramine du groupe A tel que défini dans la revendication 14.

16. Association selon la revendication 15 avec la (16*R*)-16-Désoxo-16-fluoro pristinamycine II_{B}.

## Patentansprüche

1. Ein Derivat der Gruppe B von Streptograminen der allgemeinen Formel (I) 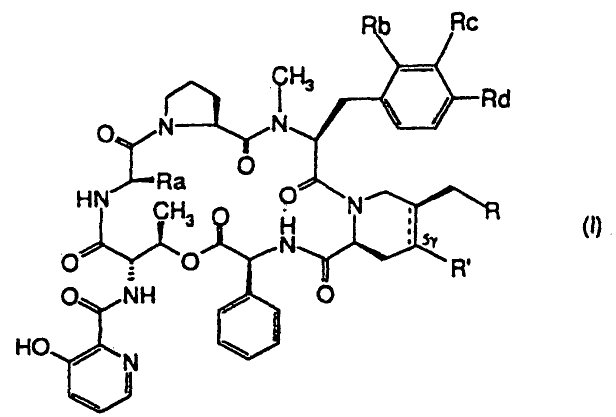 in der
R einen Rest -NR₁R₂ oder -SR₃ darstellt, worin:
R₁ und R₂, gleich oder verschieden, ein Wasserstoffatom, einen Rest Alkyl (1 bis 8 Kohlenstoffe), gegebenenfalls substituiert durch Hydroxy, Alkenyl (3 bis 8 Kohlenstoffe), Cycloalkyl (3 bis 8 Kohlenstoffe), Alkyloxy (1 bis 8 Kohlenstoffe), Dialkylamino, Phenylalkyl, gegebenenfalls substituiert [durch ein oder mehrere Halogenatome oder Reste Alkyl, Hydroxyalkyl, Alkyloxy oder Dialkylamino], Heterocyclylalkyl (3 bis 8 Ringglieder), gesättigt oder ungesättigt und enthaltend ein oder mehrere Heteroatome, ausgewählt unter Stickstoff, Schwefel oder Sauerstoff, oder Dialkylaminoalkyl bedeuten, oder
R₁ und R₂ zusammen mit dem Stickstoffatom, an das sie gebunden sind, einen gesättigten, teilweise gesättigten oder ungesättigten, mono- oder polycylischen Heterocyclus mit 3 bis 12 Ringgliedern bilden, der gegebenenfalls ein weiteres Heteroatom enthält, ausgewählt unter Sauerstoff, Schwefel oder Stickstoff, und gegebenenfalls substituiert ist [durch einen oder mehrere Reste Hydroxy, Alkyl, Phenyl, gegebenenfalls substituiert durch ein Halogenatom, Phenylalkyl, Phenylalkenyl (Alkenyl mit 2 bis 4 Kohlenstoffen), Hydroxyalkyl, Acyl, Alkyloxycarbonyl, oder Heterocyclyl oder Heterocyclyl-carbonyl, dessen Teil Heterocyclyl (4 bis 6 Ringglieder) gesättigt oder ungesättigt ist und ein oder mehrere Heteroatome enthält, ausgewählt unter Sauerstoff, Schwefel oder Stickstoff],
R₃ einen Rest Alkyl (enthaltend 1 bis 8 Kohlenstoffatome) oder Cycloalkyl (enthaltend 3 bis 8 Kohlenstoffatome) bedeutet, substituiert durch einen Rest -NR₁R₂, worin R₁ und R₂, gleich oder verschieden, ein Wasserstoffatom oder einen Rest Alkyl darstellen oder zusammen mit dem Stickstoffatom, an das sie gebunden sind, einen wie oben definierten Heterocyclus bilden, oder R₃ auch einen mono- oder polycyclischen Rest Heterocyclyl oder Heterocyclylmethyl darstellt, gesättigt oder ungesättigt und enthaltend 3 bis 7 Ringglieder und gegebenenfalls ein weiteres Heteroatom, ausgewählt unter Sauerstoff, Schwefel oder Stickstoff und gegebenenfalls substituiert durch einen Rest Alkyl, 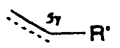
den Rest eines ungesättigten Ringes, der in 5γ nicht substituiert ist: 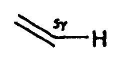 oder den Rest eines gesättigten Ringes darstellt,
der in 5γ durch einen Fluor-Rest substituiert ist: 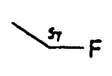 Ra ein Rest Methyl oder Ethyl ist, und
Rb, Rc und Rd die nachstehenden Bedeutungen besitzen:
1) Rb und Rc sind Wasserstoffatome und Rd ist ein Wasserstoffatom oder ein Rest Methylamino oder Dimethylamino,
2) Rb ist ein Wasserstoffatom, Rc ist ein Atom von Wasserstoff, Chlor oder Brom oder stellt einen Rest Alkenyl (3 bis 5 Kohlenstoffe) dar, und Rd ist ein Rest -NMe-R"', worin R"' einen Rest Alkyl, Hydroxyalkyl (2 bis 4 Kohlenstoffe) oder Alkenyl (2 bis 8 Kohlenstoffe), gegebenenfalls substituiert durch Phenyl, Cycloalkyl (3 bis 6 Kohlenstoffe), Methyl, Benzyl, Benzyl substituiert [durch ein oder mehrere Halogenatome oder Reste Hydroxy, Alkyl, Alkyloxy, Alkylthio, Alkylsulfinyl, Alkylsulfonyl, Amino, Alkylamino oder Dialkylamino], Heterocyclylmethyl oder Heterocyclylethyl bedeutet, dessen Teil Heterocyclyl gesättigt oder ungesättigt ist und 5 bis 6 Ringglieder und 1 oder 2 Heteroatome enthält, ausgewählt unter Schwefel, Sauerstoff oder Stickstoff, gegebenenfalls substituiert [durch einen Rest Alkyl, Alkenyl (2 bis 8 Kohlenstoffe), Cycloalkyl (3 bis 8 Kohlenstoffe), Heterocyclyl (4 bis 6 Kohlenstoffe), gesättigt oder ungesättigt, Phenyl, Phenyl substituiert, wie oben bei der Definition von R₁ oder Benzyl angegeben], oder R"' auch einen Rest Cyanomethyl oder Carboxymethyl darstellt oder -CORe oder -CH₂CORe bedeutet, worin Re entweder -OR'e ist, R'e Alkyl (1 bis 6 Kohlenstoffe), Alkenyl (2 bis 6 Kohlenstoffe), Benzyl, Phenyl, Tolyl oder Heterocyclylmethyl darstellt, dessen Teil Heterocyclyl 5 bis 6 Ringglieder und 1 oder 2 Heteroatome enthält, ausgewählt unter Schwefel, Sauerstoff oder Stickstoff, oder Re ist ein Rest Alkylamino, Alkylmethylamino, Heterocyclylamino oder Heterocyclylmethylamino, dessen Teil Heterocyclyl gesättigt ist und 5 bis 6 Ringglieder und 1 oder 2 Heteroatome enthält, ausgewählt unter Schwefel, Sauerstoff oder Stickstoff, gegebenenfalls substituiert durch einen Rest Alkyl, Benzyl oder Alkyloxycarbonyl,
3) Rb ist ein Wasserstoffatom, Rd ist ein Rest -NHCH₃ oder -N(CH₃)₂ und Rc ist ein Atom von Chlor oder Brom oder stellt einen Rest Alkenyl (3 bis 5 Kohlenstoffe) dar [wenn Rd -N(CH₃)₂ ist],
4) Rb und Rd sind Wasserstoffatome und Rc ist ein Halogenatom oder ein Rest Alkylamino oder Dialkylamino, Alkyloxy, Trifluormethoxy, Thioalkyl, Alkyl (1 bis 6 Kohlenstoffe) oder Trihalogenmethyl,
5) Rb und Rc sind Wasserstoffatome und Rd ist ein Halogenatom oder ein Rest Ethylamino, Diethylamino oder Methylethylamino, Alkyloxy oder Trifluormethoxy, Alkylthio, Alkylsulfinyl, Alkylsulfonyl, Alkyl (1 bis 6 Kohlenstoffe), Phenyl oder Trihalogenmethyl,
6) Rb ist ein Wasserstoffatom und Rc ist ein Halogenatom oder ein Rest Alkylamino oder Dialkylamino, Alkyloxy oder Trifluormethoxy, Thioalkyl, Alkyl (1 bis 3 Kohlenstoffe), und Rd ist ein Halogenatom oder ein Rest Amino, Alkylamino oder Dialkylamino, Alkyloxy oder Trifluormethoxy, Thioalkyl, Alkyl (1 bis 6 Kohlenstoffe), oder Trihalogenmethyl,
7) Rc ist ein Wasserstoffatom und Rb und Rd bedeuten einen Rest Methyl,
mit der Maßgabe, daß außer bei spezieller Erwähnung die Reste Alkyl oder Acyl gerade oder verzweigt sind und 1 bis 4 Kohlenstoffatome enthalten, und daß die Reste Alkenyl ebenfalls in gerader oder verzweigter Kette vorliegen und 2 bis 4 Kohlenstoffatome enthalten,
sowie ihre Salze, wenn sie existieren.

2. Ein Derivat der Gruppe B von Streptograminen nach Anspruch 1, **dadurch gekennzeichnet, daß**
R einen Rest -NR₁R₂ oder -SR₃ darstellt, worin:
R₁ und R₂, gleich oder verschieden, ein Wasserstoffatom, einen Rest Alkyl (1 bis 8 Kohlenstoffe), gegebenenfalls substituiert durch Hydroxy, Alkenyl (3 bis 8 Kohlenstoffe), Cycloalkyl (3 bis 8 Kohlenstoffe), Alkyloxy (1 bis 8 Kohlenstoffe), Dialkylamino, Phenylalkyl, gegebenenfalls substituiert [durch ein oder mehrere Halogenatome oder Reste Alkyl, Hydroxyalkyl, Alkyloxy oder Dialkylamino], Heterocyclylalkyl (3 bis 8 Ringglieder), gesättigt oder ungesättigt und enthaltend ein oder mehrere Heteroatome, ausgewählt unter Stickstoff, Schwefel oder Sauerstoff, oder Dialkylaminoalkyl bedeuten, oder
R₁ und R₂ zusammen mit dem Stickstoffatom, an das sie gebunden sind, einen gesättigten, teilweise gesättigten oder ungesättigten, mono- oder polycylischen Heterocyclus mit 3 bis 12 Ringgliedern bilden, der gegebenenfalls ein weiteres Heteroatom enthält, ausgewählt unter Sauerstoff, Schwefel oder Stickstoff, und gegebenenfalls substituiert ist [durch einen oder mehrere Reste Hydroxy, Alkyl, Phenyl, gegebenenfalls substituiert durch ein Halogenatom, Phenylalkyl, Hydroxyalkyl, Acyl, Alkyloxycarbonyl, oder Heterocyclyl oder Heterocyclyl-carbonyl, dessen Teil Heterocyclyl (4 bis 6 Ringglieder) gesättigt oder ungesättigt ist und ein oder mehrere Heteroatome enthält, ausgewählt unter Sauerstoff, Schwefel oder Stickstoff],
R₃ einen Rest Alkyl (enthaltend 1 bis 8 Kohlenstoffatome) bedeutet, substituiert durch einen Rest -NR₁R₂, worin R₁ und R₂, gleich oder verschieden, einen Rest Alkyl darstellen oder zusammen mit dem Stickstoffatom, an das sie gebunden sind, einen wie oben definierten Heterocyclus bilden, oder R₃ auch einen mono- oder polycyclischen Rest Heterocyclyl oder Heterocyclylmethyl darstellt, gesättigt oder ungesättigt und enthaltend 3 bis 7 Ringglieder und gegebenenfalls ein weiteres Heteroatom, ausgewählt unter Sauerstoff, Schwefel oder Stickstoff und gegebenenfalls substituiert durch einen Rest Alkyl, 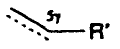
den Rest eines ungesättigten Ringes, der in 5γ nicht substituiert ist: 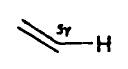 oder den Rest eines gesättigten Ringes darstellt,
der in 5γ durch einen Fluor-Rest substituiert ist: 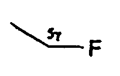 Ra ein Rest Ethyl ist, und
Rb, Rc und Rd die nachstehenden Bedeutungen besitzen:
1) Rb und Rc sind Wasserstoffatome und Rd ist ein Rest Methylamino oder Dimethylamino,
2) Rb ist ein Wasserstoffatom, Rc ist ein Atom von Wasserstoff oder Chlor und Rd ist ein Rest -NMe-R"', worin R"' einen Rest Alkenyl (2 bis 8 Kohlenstoffe), Heterocyclylmethyl bedeutet oder -COOR'e darstellt, worin R'e Alkyl (1 bis 6 Kohlenstoffe), Alkenyl (2 bis 6 Kohlenstoffe), Phenyl oder Tolyl ist,
3) Rb ist ein Wasserstoffatom, Rd ist ein Rest -NHCH₃ oder -N(CH₃)₂ und Rc ist ein Chloratom,
sowie ihre Salze, wenn sie existieren.

3. Ein Derivat der Gruppe B von Streptograminen nach Anspruch 1, **dadurch gekennzeichnet, daß** es sich um 5δ-(1-Morpholino)-methyl-5δ,5γ-dehydro-pristinamycin I_{E} handelt.

4. Ein Derivat der Gruppe B von Streptograminen nach Anspruch 1, **dadurch gekennzeichnet, daß** es sich um 5δ-[N-Methyl-N-2-(1,3-dioxolanyl) -methyl] -aminomethyl-5δ,5γ-dehydro-pristinamycin I_{E} handelt.

5. Ein Derivat der Gruppe B von Streptograminen nach Anspruch 1, **dadurch gekennzeichnet, daß** es sich um 5δ-Morpholinomethyl-4ζ-methylamino-4ζ-desdimethylamino-5δ,5γ-dehydro-pristinamycin I_{E} handelt.

6. Ein Derivat der Gruppe B von Streptograminen nach Anspruch 1, **dadurch gekennzeichnet, daß** es sich um 5δ-Morpholinomethyl-4ζ-methylamino-4ζ-desdimethylamino-5δ,5γ-dehydro-4ε-chlor-pristinamycin I_{E} handelt.

7. Ein Derivat der Gruppe B von Streptograminen nach Anspruch 1, **dadurch gekennzeichnet, daß** es sich um 5δ-[Bis-(2-Methoxyethyl)-aminomethyl]-5δ,5γ-dehydro-pristinamycin I_{E} handelt.

8. Verfahren zur Herstellung eines Streptogramin-Derivates nach Anspruch 1, **dadurch gekennzeichnet, daß** man ein Fluorierungsmittel mit dem Derivat von Synergistin der Gruppe B der allgemeinen Formel (II) 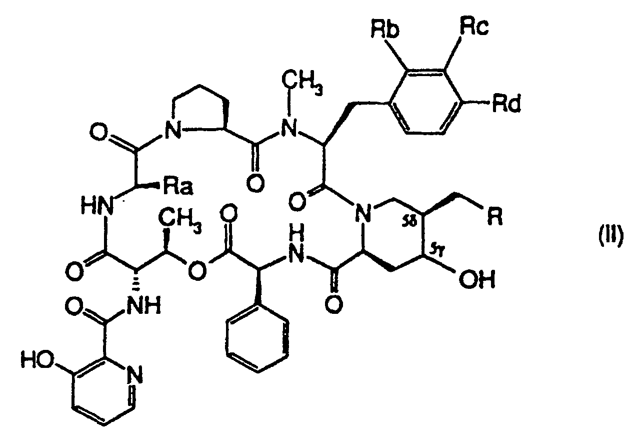 in der R, Ra, Rb, Rc und Rd wie in Anspruch 1 definiert sind, zur Reaktion bringt, anschließend das fluorierte Derivat oder das in Position 5γ,5δ ungesättigte Derivat abtrennt und gegebenenfalls das erhaltene Streptogramin-Derivat in ein Salz überführt.

9. Verfahren nach Anspruch 8, **dadurch gekennzeichnet, daß** die Abtrennung des fluorierten Derivates und des in Position 5γ,5δ ungesättigten Derivates durch Chromatographie oder durch Kristallisation erfolgt.

10. Verfahren zur Herstellung eines Streptogramin-Derivates nach Anspruch 1, worin das Symbol 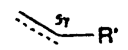 das Symbol 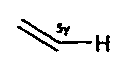 darstellt,
**dadurch gekennzeichnet, daß** man ein Thionylhalogenid in Anwesenheit einer Stickstoffbase mit einem Derivat von Synergistin der Gruppe B der allgemeinen Formel (II) 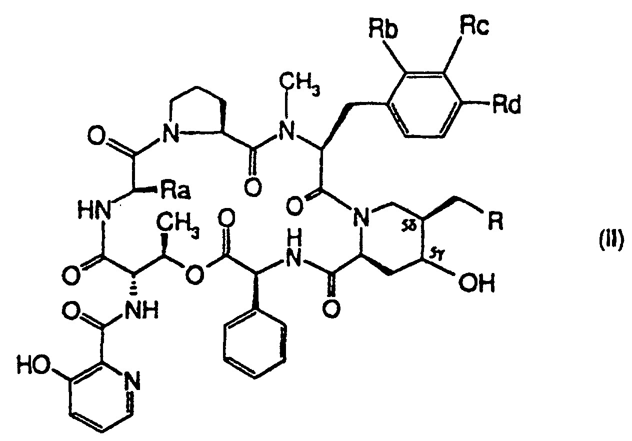 in der R, Ra, Rb, Rc und Rd wie in Anspruch 1 definiert sind, zur Reaktion bringt und anschließend gegebenenfalls das erhaltene Streptogramin-Derivat in ein Salz überführt.

11. Verfahren zur Herstellung eines Streptogramin-Derivates nach Anspruch 1, worin das Symbol 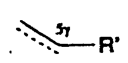 das Symbol 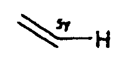 darstellt,
**dadurch gekennzeichnet, daß** man ein Amin HNR₁R₂ oder ein Thiol HS-R₃ mit einem halogenierten Streptogramin-Derivat der allgemeinen Formel (IV) 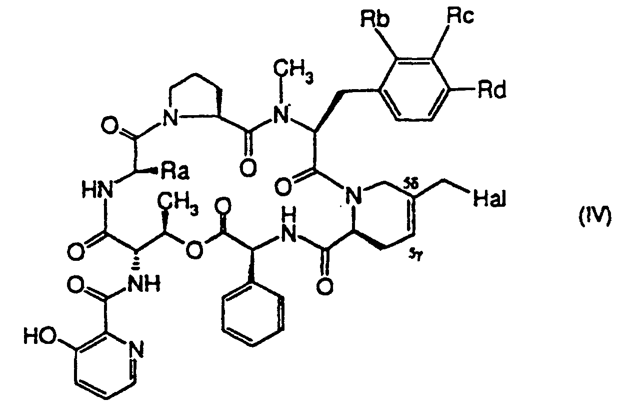 in der R, Ra, Rb, Rc und Rd wie in Anspruch 1 definiert sind und Hal ein Halogenatom darstellt, zur Reaktion bringt und anschließend gegebenenfalls das erhaltene Streptogramin-Derivat in ein Salz überführt.

12. Streptogramin-Derivat der allgemeinen Formel (II) 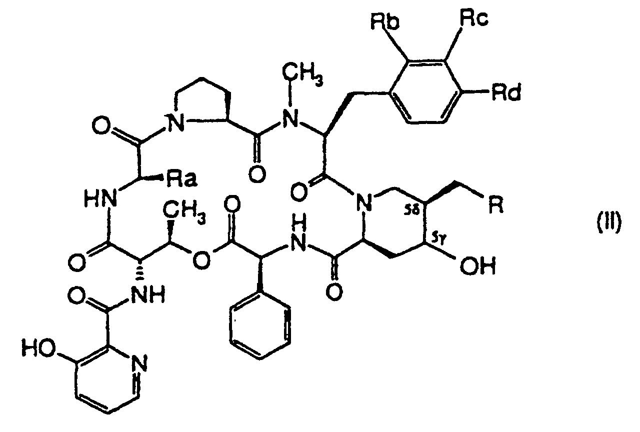 in der R, Ra, Rb, Rc und Rd wie in Anspruch 1 definiert sind.

13. Pharmazeutische Zusammensetzungen, umfassend mindestens ein Streptogramin-Derivat der Gruppe B nach Anspruch 1 in reinem Zustand oder in Form einer Assoziation mit mindestens einem Streptogramin-Derivat der Gruppe A, gegebenenfalls in Form von Salz und/oder in Form einer Assoziation mit einem oder mehreren kompatiblen und pharmazeutisch akzeptablen Verdünnungsmitteln oder Zusatzstoffen.

14. Pharmazeutische Zusammensetzungen nach Anspruch 13, **dadurch gekennzeichnet, daß** das Streptogramin-Derivat der Gruppe A ausgewählt wird unter Pristinamycin IIA, Pristinamycin IIB, Pristinamycin IIC, Pristinamycin IID, Pristinamycin IIE, Pristinamycin IIF, Pristinamycin IIG oder unter den bekannten Derivaten der Hemisynthese oder unter den Derivaten der allgemeinen Formel (α) 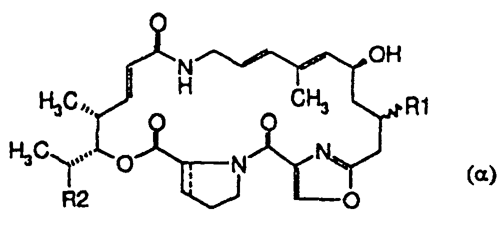 in der R₁ ein Rest -NR'R" ist, worin R' ein Wasserstoffatom oder einen Rest Methyl darstellt und R" ein Wasserstoffatom, einen Rest Alkyl, Cycloalkyl, Allyl, Propargyl, Benzyl oder -OR"', worin R"' ein Wasserstoffatom, ein Rest Alkyl, Cycloalkyl, Allyl, Propargyl oder Benzyl ist, oder -NR₃R₄ bedeutet, R₃ und R₄ einen Rest Methyl darstellen können oder zusammen mit dem Stickstoffatom, an das sie gebunden sind, einen gesättigten oder ungesättigten Heterocyclus mit 4 oder 5 Ringgliedern bilden, der außerdem ein weiteres Heteroatom enthalten kann, ausgewählt unter Stickstoff, Sauerstoff oder Schwefel, R₂ ein Wasserstoffatom oder ein Rest Methyl oder Ethyl ist und die Bindung ---- eine einfache Bindung oder eine Doppelbindung darstellt,
oder auch unter den Derivaten der Hemisynthese der allgemeinen Formel (β) 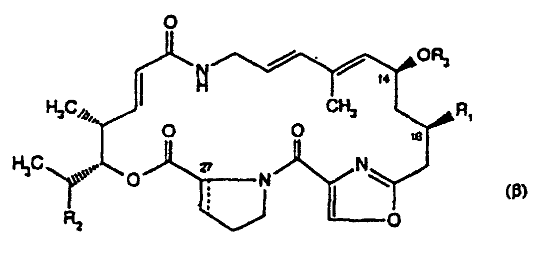 in der
R₁ ein Halogenatom oder einen Rest Azido oder Thiocyanato darstellt, R₂ ein Wasserstoffatom oder ein Rest Methyl oder Ethyl ist, R₃ ein Wasserstoffatom oder den Rest eines aliphatischen, cycloaliphatischen, aromatischen, araliphatischen, heterocyclischen oder heterocyclylaliphatischen Esters bedeutet, der substituiert sein kann, und die Bindung ---- eine einfache Bindung (Stereochemie 27R) oder eine Doppelbindung darstellt, und ihre Salze, wenn sie existieren; und insbesondere die Produkte der allgemeinen Formel (β), in denen der Ester-Rest R₃ ausgewählt wird unter: den Resten R'₃-CO-, worin R'₃ Phenyl oder Phenylalkyl ist, nicht substituiert oder substituiert an dem Rest Phenyl [durch einen oder mehrere Reste, ausgewählt unter Alkyl, das gegebenenfalls einen Rest NR"R"' trägt, dessen Reste R" und R"', gleich oder verschieden, Wasserstoffatome oder Reste Alkyl sein können, die zusammen mit dem Stickstoffatom, an das sie gebunden sind, einen gesättigten oder ungesättigten Heterocyclus mit 3 bis 8 Ringgliedern bilden können, der gegebenenfalls ein weiteres Heteroatom umfaßt, ausgewählt unter Sauerstoff, Schwefel oder Stickstoff, wobei der genannte Heterocyclus selbst substituiert sein kann durch einen oder mehrere Reste (Alkyl, Hydroxyalkyl, Alkyloxyalkyl, Alkyloxycarbonylalkyl, Aryl, Heterocyclyl, Heterocyclylalkyl, gesättigt oder ungesättigt und mit 3 bis 8 Ringgliedern, oder -CH₂-CO-NR"R"') oder R" und/oder R"' können sein ein Rest Hydroxyalkyl, Phenyl, Heterocyclylalkyl, gesättigt oder ungesättigt und mit 3 bis 8 Ringgliedern, -CO-NR"R"', worin NR"R"' wie oben definiert ist, oder Alkyl oder Acyl, substituiert durch NR"R"' wie oben definiert], oder R'₃ kann ausgewählt werden unter den Resten Phenyl oder Phenylalkyl, substituiert an dem Rest Phenyl durch einen oder mehrere Reste [ausgewählt unter Alkyl, das durch einen Rest Alkyloxy oder Alkylthio substituiert sein kann und gegebenenfalls selbst einen Rest Carboxy oder einen wie oben definierten Rest NR"R"' trägt, oder ausgewählt unter Acyloxy, das durch NR"R"', wie oben definiert, substituiert sein kann], oder R'₃ kann auch ausgewählt werden unter den Resten Alkyl oder Cycloalkyl, gegebenenfalls substituiert [durch einen Rest Carboxy, Carboxyalkyldisulfanyl oder durch einen Rest NR"R"', -CH₂-NR"R"', -CO-NR"R"', oder durch einen Rest Alkyloxycarbonyl, Alkyloxy oder Alkyldisulfanyl, gegebenenfalls substituiert durch NR"R"' oder -CO-NR"R"', worin NR"R"' wie oben definiert ist], oder R'₃ kann auch ausgewählt werden unter den gesättigten oder ungesättigten Resten Heterocyclyl mit 3 bis 8 Ringgliedern, gegebenenfalls substituiert [durch Alkyl oder Acyl, gegebenenfalls selbst substituiert durch NR"R'''].

15. Assoziationen von einem Streptogramin-Derivat der Gruppe B nach Anspruch 1 mit mindestens einem Streptogramin-Derivat der Gruppe A wie in Anspruch 14 definiert.

16. Assoziation nach Anspruch 15 mit (16*R*)-16-Desoxy-16-fluorpristinamycin II_{B}.

## Claims

1. B-group streptogramin derivative of general formula: 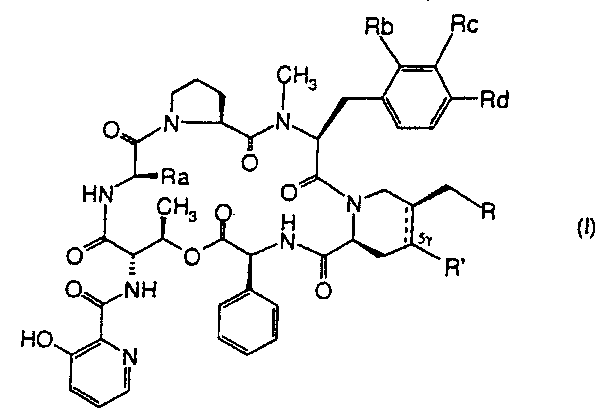 in which:
R represents a radical -NR₁R₂ or -SR₃ for which:
R₁ and R₂, which may be identical or different, represent a hydrogen atom, an alkyl radical (1 to 8 carbons) optionally substituted with hydroxyl, alkenyl (3 to 8 carbons), cycloalkyl (3 to 8 carbons), alkyloxy (1 to 8 carbons), dialkylamino, phenylalkyl which is optionally substituted [with one or more halogen atoms or alkyl, hydroxyalkyl, alkyloxy or dialkylamino radicals], saturated or unsaturated heterocyclylalkyl (3- to 8-membered) containing one or more hetero atoms chosen from nitrogen, sulphur and oxygen, or dialkylaminoalkyl, or alternatively
R₁ and R₂ form, together with the nitrogen atom to which they are attached, a 3- to 12-membered, saturated, partially saturated or unsaturated monocyclic or polycyclic heterocycle optionally containing another hetero atom chosen from oxygen, sulphur and nitrogen and optionally substituted [with one or more hydroxyl, alkyl, phenyl optionally substituted with a halogen atom, phenylalkyl, phenylalkenyl (alkenyl containing 2 to 4 carbons), hydroxyalkyl, acyl or alkyloxycarbonyl radicals, or heterocyclyl or heterocyclylcarbonyl radicals in which the heterocyclyl portion is saturated or unsaturated (4- to 6-membered) and contains one or more hetero atoms chosen from oxygen, sulphur and nitrogen],
R₃ is an alkyl (containing 1 to 8 carbon atoms) or cycloalkyl (containing 3 to 8 carbon atoms) radical substituted with a radical -NR₁R₂ for which R₁ and R₂, which may be identical or different, represent a hydrogen atom or an alkyl radical or form, together with the nitrogen atom to which they are attached, a heterocycle as defined above, or alternatively R₃ represents a 3- to 7-membered, saturated or unsaturated monocyclic or polycyclic heterocyclyl or heterocyclylmethyl radical optionally containing another hetero atom chosen from oxygen, sulphur and nitrogen and optionally substituted with an alkyl radical, 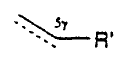
represents an unsaturated ring residue which is unsubstituted at 5γ: 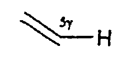 or a saturated ring residue which is substituted at 5γ with a fluoro radical: 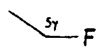 Ra is a methyl or ethyl radical, and
Rb, Rc and Rd have the definitions below:
1) Rb and Rc are hydrogen atoms and Rd is a hydrogen atom or a methylamino or dimethylamino radical,
2) Rb is a hydrogen atom, Rc is a hydrogen, chlorine or bromine atom or represents an alkenyl radical (3 to 5C) and Rd is a radical -NMe-R''' for which R"' represents an alkyl, hydroxyalkyl (2 to 4C) or alkenyl (2 to 8C) optionally substituted with phenyl, cycloalkyl (3 to 6C) methyl, benzyl, substituted benzyl [which is substituted with one or more halogen atoms or hydroxyl, alkyl, alkyloxy, alkylthio, alkylsulphinyl, alkylsulphonyl, amino, alkylamino or dialkylamino radicals], heterocyclylmethyl or heterocyclylethyl radical in which the heterocyclyl portion is saturated or unsaturated and 5- or 6-membered and contains 1 or 2 hetero atoms chosen from sulphur, oxygen and nitrogen which is optionally substituted [with an alkyl, alkenyl (2 to 8 carbons), cycloalkyl (3 to 6 carbons), saturated or unsaturated heterocyclyl (4- to 6-membered), phenyl, substituted phenyl as defined above for the definition of R₁ or benzyl radical], or alternatively R''' represents a cyanomethyl or carboxymethyl radical, or represents -CORe or -CH₂CORe for which either Re is -OR'e, R'e being alkyl (1 to 6 carbons), alkenyl (2 to 6 carbons), benzyl, phenyl, tolyl or heterocyclylmethyl in which the heterocyclyl portion is 5- or 6-membered and contains 1 or 2 hetero atoms chosen from sulphur, oxygen and nitrogen or Re is an alkylamino, alkylmethylamino, heterocyclylamino or heterocyclylmethylamino radical in which the heterocyclyl portion is saturated and 5- or 6-membered and contains 1 or 2 hetero atoms chosen from sulphur, oxygen and nitrogen which is optionally substituted with an alkyl, benzyl or alkyloxycarbonyl radical,
3) Rb is a hydrogen atom, Rd is an -NHCH₃ or -N(CH₃)₂ radical and Rc is a chlorine or bromine atom or represents an alkenyl radical (3 to 5C) [if Rd is -N(CH₃)₂],
4) Rb and Rd are hydrogen atoms and Rc is a halogen atom or an alkylamino or dialkylamino, alkyloxy, trifluoromethoxy, thioalkyl, alkyl (1 to 6C) or trihalomethyl radical,
5) Rb and Rc are hydrogen atoms and Rd is a halogen atom or an ethylamino, diethylamino or methylethylamino, alkyloxy or trifluoromethoxy, alkylthio, alkylsulphinyl, alkylsulphonyl, alkyl (1 to 6C), phenyl or trihalomethyl radical,
6) Rb is a hydrogen atom and Rc is a halogen atom or an alkylamino or dialkylamino, alkyloxy or trifluoromethoxy, thioalkyl or alkyl (1 to 3C) radical and Rd is a halogen atom or an amino, alkylamino or dialkylamino, alkyloxy or trifluoromethoxy, thioalkyl, alkyl (1 to 6C) or trihalomethyl radical,
7) Rc is a hydrogen atom and Rb and Rd represent a methyl radical,
it being understood that, unless especially mentioned, the alkyl and acyl radicals are straight or branched and contain 1 to 4 carbon atoms and that the alkenyl radicals also have a straight or branched chain and contain 2 to 4 carbon atoms, as well as the salts thereof, when they exist.

2. B-group streptogramin derivative according to Claim 1, **characterized in that**
R represents a radical -NR₁R₂ or -SR₃ for which:
R₁ and R₂, which may be identical or different, represent a hydrogen atom, an alkyl radical (1 to 8 carbons) optionally substituted with hydroxyl, alkenyl (3 to 8 carbons), cycloalkyl (3 to 8 carbons), alkyloxy (1 to 8 carbons), dialkylamino, phenylalkyl which is optionally substituted [with one or more halogen atoms or alkyl, hydroxyalkyl, alkyloxy or dialkylamino radicals], saturated or unsaturated heterocyclylalkyl (3- to 8-membered) containing one or more hetero atoms chosen from nitrogen, sulphur and oxygen, or dialkylaminoalkyl, or alternatively
R₁ and R₂ form, together with the nitrogen atom to which they are attached, a 3- to 12-membered, saturated, partially saturated or unsaturated monocyclic or polycyclic heterocycle optionally containing another hetero atom chosen from oxygen, sulphur and nitrogen and optionally substituted [with one or more hydroxyl, alkyl, phenyl optionally substituted with a halogen atom, phenylalkyl, hydroxyalkyl, acyl or alkyloxycarbonyl radicals, or heterocyclyl or heterocyclylcarbonyl radicals in which the heterocyclyl portion is saturated or unsaturated (4- to 6-membered) and contains one or more hetero atoms chosen from oxygen, sulphur and nitrogen],
R₃ is an alkyl (containing 1 to 8 carbon atoms) radical substituted with a radical -NR₁R₂ for which R₁ and R₂, which may be identical or different, represent an alkyl radical or form, together with the nitrogen atom to which they are attached, a heterocycle as defined above, or alternatively R₃ represents a 3- to 7-membered, saturated or unsaturated monocyclic or polycyclic heterocyclyl or heterocyclylmethyl radical optionally containing another hetero atom chosen from oxygen, sulphur and nitrogen and optionally substituted with an alkyl radical, 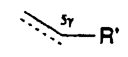
represents an unsaturated ring residue which is unsubstituted at 5γ: 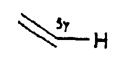 or a saturated ring residue which is substituted at 5γ with a fluoro radical: 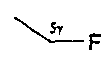 Ra is an ethyl radical, and
Rb, Rc and Rd have the definitions below:
1) Rb and Rc are hydrogen atoms and Rd is a methylamino or dimethylamino radical,
2) Rb is a hydrogen atom, Rc is a hydrogen or chlorine atom and Rd is a radical -NMe-R''' for which R''' represents an alkenyl (2 to 8C) or heterocyclylmethyl radical or represents -COOR'e in which R'e is alkyl (1 to 6 carbons), alkenyl (2 to 6 carbons), phenyl or tolyl,
3) Rb is a hydrogen atom, Rd is an -NHCH₃ or -N(CH₃)₂ radical and Rc is a chlorine atom, as well as the salts thereof, when they exist.

3. B-group streptogramin derivative according to Claim 1, **characterized in that** it is 5δ-(1-morpholino)methyl-5δ,5γ-dehydropristinamycin I_{E}.

4. B-group streptogramin derivative according to Claim 1, **characterized in that** it is 5δ-[N-methyl-N-2-(1,3-dioxolanyl)methyl]aminomethyl-5δ, 5γ-dehydropristinamycin I_{E}.

5. B-group streptogramin derivative according to Claim 1, **characterized in that** it is 5δ-morpholinomethyl-4ζ-methylamino-4ζ-dedimethylamino-5δ,5γ-dehydropristinamycin I_{E}.

6. B-group streptogramin derivative according to Claim 1, **characterized in that** it is 5δ-morpholinomethyl-4ζ-methylamino-4ζ-dedimethylamino-5δ,5γ-dehydro-4ε-chloropristinamycin I_{E}.

7. B-group streptogramin derivative according to Claim 1, **characterized in that** it is 5δ-[bis(2-methoxyethyl)aminomethyl]-5δ,5γ-dehydropristinamycin I_{E}.

8. Process for preparing a streptogramin derivative according to Claim 1, **characterized in that** a fluorinating agent is reacted with the B-group synergistin derivative of general formula: 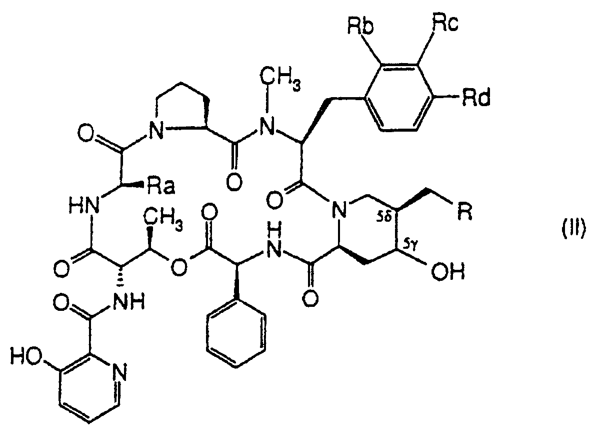 in which R, Ra, Rb, Rc and Rd are defined as in Claim 1, after which the fluoro derivative or the derivative which is unsaturated in position 5γ,5δ is separated out and, where appropriate, the streptogramin derivative obtained is converted into a salt.

9. Process according to Claim 8, **characterized in that** the separation of the fluoro derivative and of the derivative which is unsaturated in position 5γ,5δ is carried out by chromatography or by crystallization.

10. Process for preparing a streptogramin derivative according to Claim 1, for which the symbol 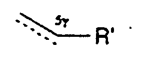 represents 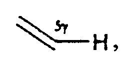 **characterized in that** a thionyl halide is reacted, in the presence of a nitrogenous base, with a B-group synergistin derivative of general formula: 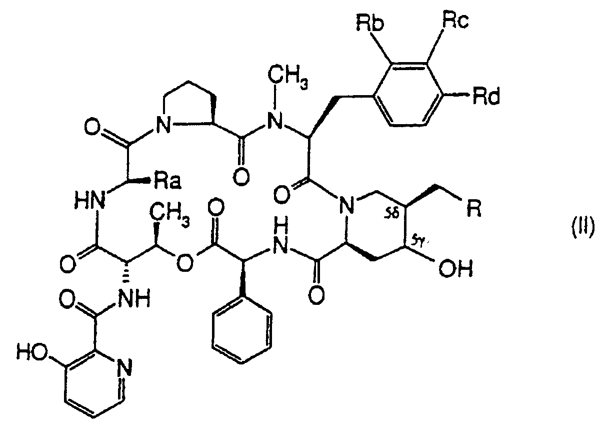 in which R, Ra, Rb, Rc and Rd are defined as in Claim 1, and, where appropriate, the streptogramin derivative obtained is then converted into a salt.

11. Process for preparing a streptogramin derivative according to Claim 1, for which the symbol 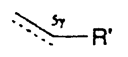 represents 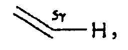 **characterized in that** an amine HNR₁R₂ or a thiol HS-R₃ is reacted with a halogenated streptogramin derivative of general formula: 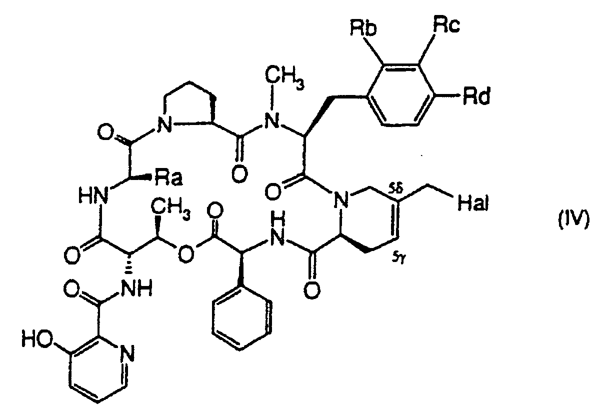 in which Ra, Rb, Rc and Rd are defined as in Claim 1 and Hal represents a halogen atom, and, where appropriate, the streptogramin derivative obtained is then converted into a salt.

12. Streptogramin derivative of general formula: 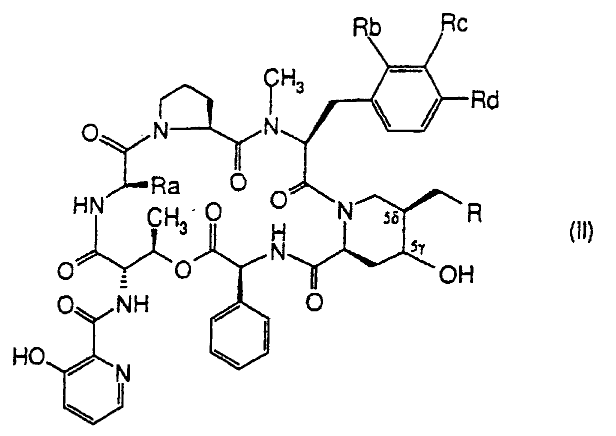 in which R, Ra, Rb, Rc and Rd are defined as in Claim 1.

13. Pharmaceutical compositions comprising at least one B-group streptogramin derivative according to Claim 1, in pure form or in the form of a combination with at least one A-group streptogramin derivative, where appropriate in the form of a salt, and/or in the form of a combination with one or more compatible and pharmaceutically acceptable diluents or adjuvants.

14. Pharmaceutical compositions according to Claim 13, **characterized in that** the A-group streptogramin derivative is chosen from pristinamycin IIA, pristinamycin IIB, pristinamycin IIC, pristinamycin IID, pristinamycin IIE, pristinamycin IIF and pristinamycin IIG or from known semisynthetic derivatives or from the derivatives of general formula: 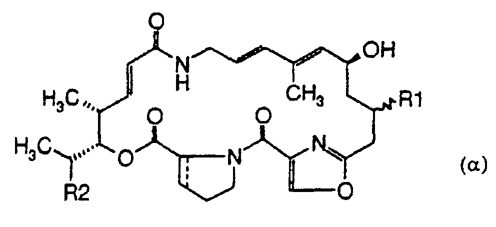 in which R₁ is a radical -NR'R" for which R' is a hydrogen atom or a methyl radical and R" is a hydrogen atom or an alkyl, cycloalkyl, allyl, propargyl, benzyl radical or a radical -OR''', R"' being a hydrogen atom or an alkyl, cycloalkyl, allyl, propargyl or benzyl radical or a radical -NR₃R₄, R₃ and R₄ possibly representing a methyl radical or forming, together with the nitrogen atom to which they are attached, a saturated or unsaturated 4- or 5-membered heterocycle which can also contain another hetero atom chosen from nitrogen, oxygen and sulphur, R₂ is a hydrogen atom or a methyl or ethyl radical and the bond --- represents a single bond or a double bond,
or alternatively from semisynthetic derivatives of general formula: 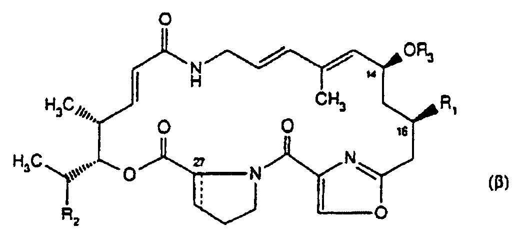 in which R₁ represents a halogen atom or an azido or thiocyanato radical, R₂ represents a hydrogen atom or a methyl or ethyl radical, R₃ represents a hydrogen atom or an aliphatic, cycloaliphatic, aromatic, araliphatic, heterocyclic or heterocyclylaliphatic ester residue which may be substituted, and the bond --- represents a single bond (27R stereochemistry) or a double bond, and the salts thereof, when they exist, and in particular the products of general formula (β) for which the ester residue R₃ can be chosen from: radicals R'₃-CO- for which R'₃ is phenyl or phenylalkyl which are unsubstituted or substituted on the phenyl radical [with one or more radicals chosen from alkyl, optionally bearing a radical NR"R''' in which the radicals R" and R"', which may be identical or different, may be hydrogen atoms or alkyl radicals which can form, together with the nitrogen atom to which they are attached, a saturated or unsaturated 3-to 8-membered heterocyclyl radical, optionally comprising another hetero atom chosen from oxygen, sulphur and nitrogen, it being possible for the said heterocycle itself to be substituted with one or more radicals (saturated or unsaturated 3- to 8-membered alkyl, hydroxyalkyl, alkyloxyalkyl, alkyloxycarbonylalkyl, aryl, heterocyclyl or heterocyclylalkyl or -CH₂-CO-NR"R'''), or alternatively R" and/or R''' can be a saturated or unsaturated 3- to 8-membered hydroxyalkyl, phenyl or heterocyclylalkyl radical, a radical -CO-NR"R''' for which NR"R''' is defined as above, or an alkyl or acyl radical substituted with NR"R''' defined as above], or alternatively R'₃ can be chosen from phenyl and phenylalkyl radicals which are substituted on the phenyl radical with one or more radicals [chosen from alkyl, which may be substituted with an alkyloxy or alkylthio radical, themselves optionally bearing a carboxyl radical or a radical NR"R''' as defined above, or chosen from acyloxy which can be substituted with NR"R''' defined as above], or alternatively R'₃ can be chosen from alkyl and cycloalkyl radicals which are optionally substituted [with a carboxyl or carboxyalkyldisulphanyl radical or with a radical NR"R''', -CH₂-NR"R''', -CO-NR"R''', or with an alkyloxycarbonyl, alkyloxy or alkyldisulphanyl radical, which are optionally substituted with NR"R''' or -CO-NR"R''' for which NR"R''' is defined as above], or alternatively R'₃ can be chosen from saturated or unsaturated 3- to 8-membered heterocyclyl radicals which are optionally substituted [with alkyl or acyl which are themselves optionally substituted with NR"R'''].

15. Combinations of a B-group streptogramin derivative, according to Claim 1, with at least one A-group streptogramin derivative as defined in Claim 14.

16. Combination, according to Claim 15, with (16R)-16-deoxo-16-fluoropristinamycin II_{B}.
